# FASCICULE DE BREVET EUROPEEN

(11) **EP 1 183 256 B1**
(45) Date de publication et mention de la délivrance du brevet: **22.08.2007**
(21) Numéro de dépôt: 00929625.2
(22) Date de dépôt: 18.05.2000
(51) Int. Cl.: C07D 473/16, C07D 473/40, A61K 31/52, A61P 25/28

(54) **DERIVES DE LA PURINE, LEUR PROCEDE DE PREPARATION, ET COMPOSITIONS PHARMACEUTIQUES LES CONTENANT**
PURINDERIVATE, VERFAHREN ZU DEREN HERSTELLUNG UND SIE ENTHALTENDE PHARMAZEUTISCHE ZUSAMMENSETZUNGEN
PURINE DERIVATIVES, PREPARATION METHOD AND PHARMACEUTICAL COMPOSITIONS CONTAINING SAME

(30) Priorité: 21.05.1999 FR 9906456
(43) Date de publication de la demande: 06.03.2002
(73) Titulaire: Aventis Pharma S.A., 92160 Antony (FR)
(72) Inventeur: HAESSLEIN, Jean-Luc, F-77181 Courtry (FR)
(74) Mandataire: Vieillefosse, Jean-Claude
(86) Numéro de dépôt international: PCT/FR2000/001335
(87) Numéro de publication internationale: WO 2000/071543

(56) Documents cités:
- EP-A- 0 452 680
- EP-A- 0 545 413
- WO-A-97/16452
- WO-A-97/20842
- WO-A-98/05335
- WO-A-98/16528
- WO-A-99/07705

## Description

La présente invention concerne de nouveaux dérivés de la purine, leur procédé de préparation, les nouveaux intermédiaires obtenus, leur application à titre de médicaments, les compositions pharmaceutiques les renfermant et la nouvelle utilisation de tels dérivés de la purine.

L'invention a ainsi pour objet de nouveaux dérivés de la purine possédant des propriétés anti-prolifératives et notamment des dérivés de la purine dotés d'un effet inhibiteur vis-à-vis des protéines kinases cycline-dépendantes soit 'cdk' en abrégé que nous utiliserons dans la suite du texte.

L'étude des mécanismes moléculaires qui contrôlent le cycle cellulaire a permis de mettre en évidence le rôle régulateur des cdk ainsi définies. Les cdk sont des protéines constituées d'au moins deux sous-unités, une sous-unité catalytique (dont cdc2 est le prototype) et une sous-unité régulatrice (cycline). On connaît ainsi un certain nombre de cdk. Les cdk forment donc des complexes protéiques dont chacun est impliqué dans une phase du cycle cellulaire.

De nombreux documents de la littérature décrivent l'existence et le rôle des cdk et à titre d'exemple, on peut citer notamment le document WO 97/20842.

Plusieurs inhibiteurs de kinases ont été décrits comme la butyrolactone, le flavopiridol et la 2(2-hydroxyéthyl-amino)-6-benzylamino-9-méthylpurine appelée olomoucine.

La présente invention a ainsi pour objet les produits de formule (I): 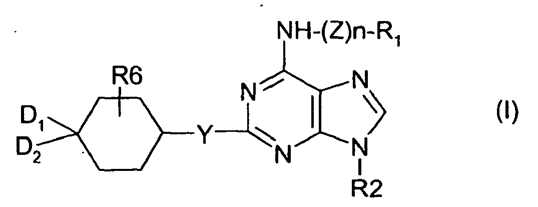 dans laquelle:
Z représente le radical divalent -CH₂-, -SO₂-, -CO-, -(CH₂)₂-NH-, -(CH₂)₂-Nalkyle, -(CH₂)₂-N-CH₂-phényle dans lesquels les radicaux phényle sont éventuellement substitués par un atome d'halogène, un radical hydroxyle, trifluoro-méthyle, alcoxy renfermant au plus 4 atomes de carbone ou carboxy libre, salifié ou estérifié,
n représente l'entier 0 ou 1,
R₁ est choisi parmi l'atome d'hydrogène et les radicaux phényle, -CH₂-phényle, -SO₂-phényle, -CO-phényle, pyridyle, alkyle et -SO₂-alkyle, dans lesquels les radicaux alkyle renferment au plus 4 atomes de carbone et sont éventuellement substitués par un radical carboxy libre, salifié ou estérifié, et tous les radicaux phényle sont éventuellement substitués par un ou plusieurs radicaux choisis parmi les atomes d'halogène et les radicaux hydroxyle, cyano, nitro, trifluorométhyle, trifluorométhoxy, thioalkyle et alcoxy renfermant au plus 4 atomes de carbone, alkyle renfermant au plus 4 atomes de carbone éventuellement substitué par un radical cyano, -COOH ou COOalk, les radicaux phényle, tétrazolyle, cycloalkyle interrompu par un ou plusieurs atomes d'oxygène ou d'azote, les radicaux -SO₂NH₂ et SO₂-NH-thiazolyle, les radicaux dioxol, carboxy libre, estérifié ou salifié et les radicaux -NHR₄ et -CONHR₄ dans lesquels R₄ représente un atome d'hydrogène, un radical alkyle renfermant au plus 4 atomes de carbone ou un radical cyclohexyle éventuellement substitué par un radical NH₂,
R₂ représente les radicaux cyclopentyle, tétrahydrofuryle ou le radical tétrahydrothiényle,
Y représente l'atome d'oxygène ou le radical -NH ou -Nalkyle dans lequel le radical alkyle linéaire ou ramifié renferme au plus 4 atomes de carbone,
D₁ et D₂ soit, identiques ou différents, sont choisis parmi l'atome d'hydrogène, le radical hydroxyle, les radicaux alkyle et alcoxy linéaires ou ramifiés renfermant au plus 4 atomes de carbone et les radicaux -NH₂, -NH-COOtBu ou -NHalkyle dans lequel le radical alkyle linéaire ou ramifié renferme au plus 4 atomes de carbone, soit forment ensemble le radical =O ou =N-Oalkyle, dans lequel le radical alkyle linéaire ou ramifié renferme au plus 4 atomes de carbone,
R₆ représente l'atome d'hydrogène, un atome d'halogène ou le radical hydroxyle,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

Dans les produits de formule (I) et dans ce qui suit :
- le terme radical alkyle linéaire ou ramifié désigne les radicaux méthyle, éthyle, propyle, isopropyle, butyle, isobutyle, sec-butyle, tert-butyle,
- le terme radical alcoxy linéaire ou ramifié désigne les radicaux méthoxy, éthoxy, propoxy, isopropoxy, butoxy linéaire, secondaire ou tertiaire,
- le terme atome d'halogène désigne de préférence l'atome de chlore, mais peut aussi représenter un atome de fluor, de brome ou d'iode,
- le terme radical cycloalkyle désigne les radicaux cyclo-propyle, cyclobutyle et tout particulièrement les radicaux cyclopentyle et cyclohexyle.

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxy-carbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxy-méthyle, propionyloxyméthyle, méthylthiométhyle, diméthyl-aminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

La présente invention a plus particulièrement pour objet les produits de formule (I) telle que définie ci-dessus répondant à la formule (Id): 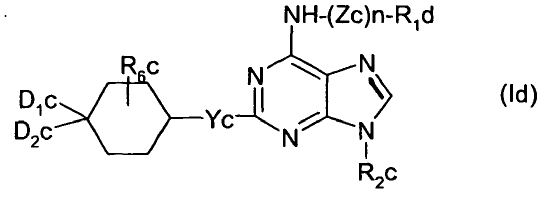 dans laquelle:
Zc représente le radical divalent -CH₂-, -SO₂- -CO-, -(CH₂)₂-NH-, -(CH₂)₂-Nalkyle, -(CH₂)₂-N-CH₂-phényle dans lesquels les radicaux phényle sont éventuellement substitués par un atome d'halogène, un radical hydroxyle, trifluoro-méthyle, alcoxy renfermant au plus 4 atomes de carbone ou carboxy libre, salifié ou estérifié,
n représente l'entier 0 ou 1,
R₁d est choisi parmi l'atome d'hydrogène et les radicaux phényle, -CH₂-phényle, -SO₂-phényle, -CO-phényle, alkyle et
   -SO₂-alkyle dans lesquels les radicaux alkyle renferment au plus 4 atomes de carbone et sont éventuellement substitués par un radical carboxy libre, salifié ou estérifié, et tous les radicaux phényle sont éventuellement substitués par un ou plusieurs radicaux choisis parmi les atomes d'halogène et les radicaux hydroxyle, cyano, nitro, trifluorométhyle, trifluorométhoxy, thioalkyle et alcoxy renfermant au plus 4 atomes de carbone, alkyle renfermant au plus 4 atomes de carbone éventuellement substitué par un radical cyano ou carboxy libre ou estérifié, les radicaux morpholinyle, phényle, tétrazolyle, -SO₂NH₂, SO₂-NH-thiazolyle, dioxol, carboxy libre, estérifié ou salifié, -NHR₄c et -CONHR₄c dans lesquels R₄c représente un atome d'hydrogène, un radical alkyle renfermant au plus 4 atomes de carbone ou un radical cyclohexyle éventuellement substitué par un radical NH₂,
R₂c représente les radicaux cyclopentyle, tétrahydrofuryle ou le radical tétrahydro-thiényle,
Yc représente l'atome d'oxygène ou le radical -N_{H} ou -N-alkyle dans lequel le radical alkyle linéaire ou ramifié renferme au plus 4 atomes de carbone,
D₁c et D₂c soit, identiques ou différents, sont choisis parmi l'atome d'hydrogène, le radical hydroxyle, les radicaux alkyle et alcoxy linéaires ou ramifiés renfermant au plus 4 atomes de carbone et les radicaux -NH₂, -NH-COOtBu ou -NH-alkyle dans lequel le radical alkyle linéaire ou ramifié renferme au plus 4 atomes de carbone, soit forment ensemble le radical =O ou =N-Oalkyle, dans lequel le radical alkyle linéaire ou ramifié renferme au plus 4 atomes de carbone,
R₆c représente l'atome d'hydrogène, un atome d'halogène ou le radical hydroxyle,
lesdits produits de formule (Id) étant sous toutes les formes isomères possibles racémiques, énantiomères et diastéréo-isomères, ainsi que les sels d'addition avec les acides minéraux et organiques ou avec les bases minérales et organiques desdits produits de formule (Id).

On préfère tout particulièrement les produits dans lesquels R₂ représente cyclopentyle.

La présente invention a tout particulièrement pour objet les produits de formule (I) telle que définie ci-dessus, répondant aux formules suivantes :
- Dichlorhydrate de trans-4-[[[2-[(4-aminocyclohexyl)-amino]-9-cyclopentyl-9H-purin-6-y]-amino]-méthyl]-benzoate de butyle,
- Dichlorhydrate de trans-4-[[2-[(4-aminocyclohexyl)-amino]-9-cyclopentyl-9H-purin-6-yl]-amino]-benzoate d'éthyle,
- Trichlorhydrate de trans-N2-(4-aminocyclohexyl)-9-cyclo-pentyl-N6-[2-[(phénylméthyl)-amino]-éthyl]-9H-purin-2,6-diamine,
- Trichlorhydrate de trans-N2-(4-aminocyclohexyl)-N6-(2-aminoéthyl)-9-cyclopentyl-9H-purin-2,6-diamine,
- Trichlorhydrate de trans-N2-(4-aminocyclohexyl)-9-cyclo-pentyl-N6-[2-[[(4-méthoxyphényl)-méthyl]-amino]-éthyl]-9H-purin-2,6-diamine,
- Trichlorhydrate de trans-N2-(4-aminocyclohexyl)-N6-[2-[[[4-chloro-3-(trifluorométhyl)-phényl]-méthyl]-amino]-éthyl]-9-cyclopentyl-9H-purin-2,6-diamine,
- Trichlorhydrate de trans-N2-(4-aminocyclohexyl)-9-cyclo-pentyl-N6-[(diphénylméthyl)-amino]-éthyl]-9H-purin-2,6-diamine,
- Trichlorhydrate de trans-N2-(4-aminocyclohexyl)-N6-[2-[[(4-chlorophényl)-méthyl]-amino]-éthyl]-9-cyclopentyl-9H-purin-2,6-diamine,
- Dichlorhydrate de trans(+-)-4-[[2-[(4-aminocyclohexyl)-amino]-9-(tétrahydro-3-thiényl)-9H-purin-6-yl]-amino]-benzoate d'éthyle,
- Dichlorhydrate de trans(+-)-N2-(4-aminocyclohexyl)-9-(tétrahydro-3-thiényl)-N6-[4-(trifluorométhoxy)-phényl]-9H-purin-2,6-diamine,
- Dichlorhydrate de trans(+-)-N2-(4-aminocyclohexyl)-9-(tétrahydro-3-furanyl)-N6-[(4-trifluorométhoxy)-phényl]-9H-purin-2,6-diamine,

La présente invention a encore tout particulièrement pour objet les produits de formule (I) telle que définie ci-dessus, répondant aux formules suivantes :
- Dichlorhydrate de trans -3-[[2-[(4-aminocyclohexyl)-amino]-9-cyclopentyl-9H-purin-6-yl]-amino]-benzoate d'éthyle,
- Trichlorhydrate de trans-N2-(4-aminocyclohexyl)-9-cyclopentyl-N6-[2-[[(3,4-dichlorophényl)-amino]-méthyl]-éthyl]-9H-purine-2,6-diamine,
- Trichlorhydrate de trans-N2-(4-aminocyclohexyl)-9-cyclopentyl-N6-[2-[[(3,5-dichlorophényl)-méthyl]-amino]-éthyl]-9H-purine-2,6-diamine,
- Dichlorhydrate de trans-4-[[2-[(4-aminocyclohexyl)-amino]-9-cyclopentyl-9H-purin-6-yl]amino]benzèneacétonitrile,
- Dichlorhydrate de trans-N2-(4-aminocyclohexyl)-9-cyclopentyl-N6-[4-(4-morpholinyl)-phényl]-9H-purine-2,6-diamine,
- Dichlorhydrate de trans-4-[[2-[(4-aminocyclohexyl)-amino]-9-cyclopentyl-9H-purin-6-yl]amino]-benzonitrile,
- Dichlorhydrate de trans-N2-(4-aminocyclohexyl)-9-cyclopentyl-N6-(4-nitrophényl)-9H-purine-2,6-diamine,
- Dichlorhydrate de trans-N2-(4-aminocyclohexyl)-N6-(4-aminophényl)-9-cyclopentyl-9H-purine-2,6-diamine,
- Dichlorhydrate de trans-N2-(4-aminocyclohexyl)-9-cyclopentyl-N6-(4-méthoxyphényl)-9H-purine-2,6-diamine,
- Dichlorhydrate de trans-5-[[2-[(4-amino-cyclohexyl)-amino]-9-cyclopentyl-9H-purin-6-yl]-amino]-1,3-benzènedicarboxylate de diéthyle.

On peut noter que dans les produits préférés de la présente invention les substituants du radical cyclohexyle sont en trans l'un par rapport à l'autre.

La présente invention a également pour objet le procédé de préparation des produits de formule (I), telle que définie ci-dessus, caractérisé en ce que l'on soumet le composé de formule (II) : 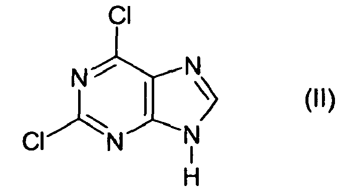 à une réaction avec un composé de formule (III) :

R₂'-OH (III)

dans laquelle R₂' a la signification indiquée ci-dessus pour R₂, dans laquelle les éventuelles fonctions réactives sont éventuellement protégées par des groupements protecteurs,
pour obtenir le produit de formule (IV) : 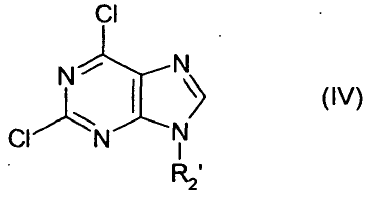 dans laquelle R₂' a la signification indiquée ci-dessus, produit de formule (IV) que l'on soumet aux réactions de l'une quelconque des voies 1 à 6 suivantes:
soit, selon la voie 1, l'on soumet le produit de formule (IV) à une réaction avec un composé de formule (V) :

   NH₂-(Z₁')n-R₁' (V)

   dans laquelle R₁' a la signification indiquée ci-dessus pour R₁, dans laquelle les éventuelles fonctions réactives sont éventuellement protégées par des groupements protecteurs, et n représente l'entier 0 ou 1 et lorsque n représente 1, alors Z₁' représente -CH₂ pour obtenir un produit de formule (VIII) : 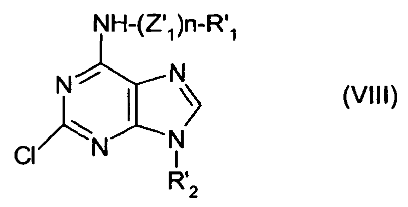 dans laquelle R₁', R₂' et Z₁' ont les significations indiquées ci-dessus,
soit, selon la voie 2, l'on soumet le produit de formule (IV) à une réaction avec un composé de formule (VI):

   NH₂-SO₂-R₁' (VI)

   dans laquelle R₁' a la signification indiquée ci-dessus, dans laquelle les éventuelles fonctions réactives sont éventuellement protégées par des groupements protecteurs, pour obtenir un produit de formule (IX) : 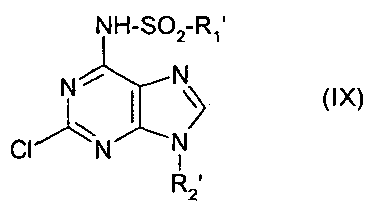 dans laquelle R₁' et R₂' ont les significations indiquées ci-dessus,
soit, selon la voie 3, l'on soumet le produit de formule (IV) à une réaction avec le composé de formule (VII):

   NH₂-(CH₂)₂-NH₂ (VII)

   pour obtenir un produit de formule (X) : 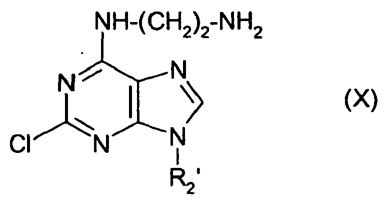 dans laquelle R₂' a la signification indiquée ci-dessus, produit de formule (X) que l'on soumet :
soit à une réaction avec un composé de formule (XI) :

   Cl-SO₂-R" (XI)

   dans laquelle -SO₂-R₁" représente les valeurs correspondantes de R_{1'} et R₁' a la signification indiquée ci-dessus, pour obtenir un composé de formule (XII) : 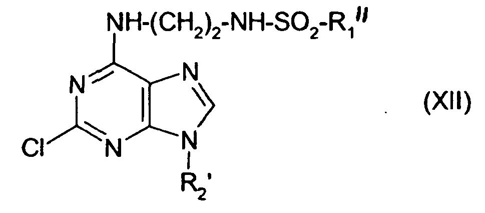 dans laquelle R₁" et R₂' ont les significations indiquées ci-dessus,
soit à une réaction avec un produit de formule (XI)_{A} :

   Cl-CO-R₁"' (XI)_{A}

   dans laquelle -CO-R₁'" représente les valeurs correspondantes de R_{1'} et R_{1'} a la signification indiquée ci-dessus, pour obtenir un produit de formule (XII)_{A} : 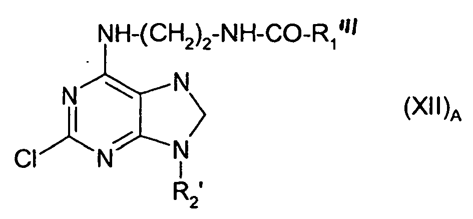 dans laquelle R₁'" et R₂' ont les significations indiquées ci-dessus,
soit à une réaction en présence d'un réducteur avec un produit de formule (XVII) :

   R₇-CHO (XVII)

   dans laquelle R₇ représente un radical aryle ou alkyle, ces radicaux étant tels que définis ci-dessus pour le radical R₁ dans lesquels les éventuelles fonctions réactives sont éventuellement protégées,
   pour obtenir un produit de formule (XIII) : 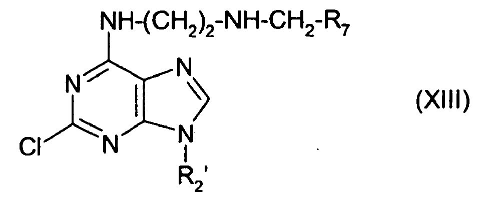 dans laquelle R₂' et R₇ ont les significations indiquées ci-dessus,
soit, selon la voie 4, l'on soumet le produit de formule (IV) à une réaction avec un composé de formule (XVIII) :

   R₁'-CO-NH₂ (XVIII)

   dans laquelle R₁' a la signification indiquée ci-dessus, pour obtenir un produit de formule (M₁) : 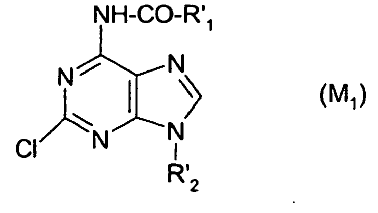 dans laquelle R₁' et R₂' ont les significations indiquées ci-dessus,
produits de formules (VIII), (IX), (XII), (XIII) et (M₁), que l'on peut soumettre aux réactions de l'une quelconque des voies a) ou b) suivantes:
a) soit à une réaction avec un composé de formule (XIV) : 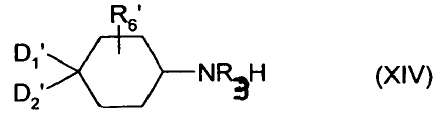 dans laquelle D'₁, D₂' et R₆' ont les significations indiquées précédemment respectivement pour D₁, D₂ et R₆ dans lesquelles les éventuelles fonctions réactives sont éventuellement protégées par des groupements protecteurs, et R₃ représente l'atome d'hydrogène ou un radical alkyle renfermant au plus 4 atomes de carbone, pour obtenir un produit de formule (Ix) : 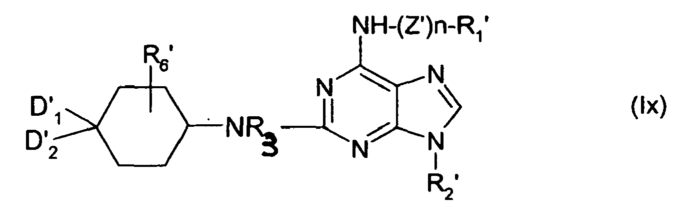 dans laquelle R₁', R₂', R₃, R₆', D₁' et D₂' ont les significations indiquées ci-dessus et Z' a la signification indiquée ci-dessus pour Z dans laquelle les éventuelles fonctions réactives sont éventuellement protégées par des groupements protecteurs,
   produit de formule (Ix) qui correspond donc à un produit de formule (I') dans laquelle Y représente -NR₃- les produits de formule (I') ayant la signification indiquée ci-dessus pour les produits de formule (I) dans laquelle les éventuelles fonctions réactives sont éventuellement protégées par des groupements protecteurs,
b) soit à une réaction avec un composé de formule (XV) : 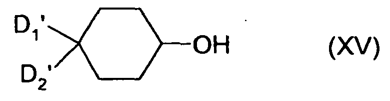 dans laquelle D₁' et D₂' ont les significations indiquées ci-dessus,
   pour obtenir un produit de formule (Iy) : 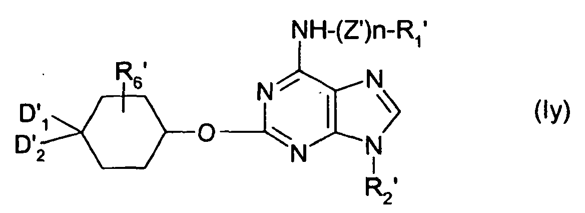 dans laquelle R₁', R₂', R₆', D₁', D₂' et Z' ont les significations indiquées ci-dessus,
   produit de formule (Iy) qui correspond donc à un produit de formule (I') tel que défini ci-dessus dans laquelle Y représente -O-,
produits de formules (Ix) et (Iy) qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction de réduction des composés nitrés en composés aminés,
j) une réaction d'élimination des groupements protecteurs que peuvent porter les fonctions réactives protégées,
k) une réaction de salification par un acide minéral ou organique ou par une base pour obtenir le sel correspondant,
l) une réaction de dédoublement des formes racémiques en produits dédoublés,
lesdits produits de formule (I) ainsi obtenus étant sous toutes les formes isomères possibles racémiques, énantiomères et diastéréoisomères.

On peut noter que de telles réactions de transformation de substituants en d'autres substituants peuvent également être effectuées sur les produits de départ ainsi que sur les intermédiaires tels que définis ci-dessus avant de poursuivre la synthèse selon les réactions indiquées dans le procédé décrit ci-dessus.

Dans des conditions préférentielles de mise en oeuvre de l'invention, le procédé décrit ci-dessus peut-être réalisé de la façon suivante :
La réaction du produit de formule (II) avec un produit de formule (III) pour donner un produit de formule (IV) peut être réalisée notamment en présence de DEAD, DIAD (diisopropyl azodicarboxylate)ou encore de triphénylphosphine P(phényl)₃ dans un solvant tel que THF ou CH₂-Cl₂ ou encore DMF.

Dans le produit de formule (III), le radical R₂ repré-sente notamment un radical alkyle, cycloalkyle, tétrahydro-furyle ou tétrahydrothiényle.

On peut citer notamment les produits de formule (III) suivants : cyclopentanol, 3-hydroxy-tétrahydrofuranne, 3-hydroxy-tétrahydrothiophène,2-hydroxybutanol et 3-hydroxy-pentanol

Les produits de formule (IV) ainsi obtenus sont soumis selon la voie 1) telle que définie ci-dessus à l'action du produit de formule (V) telle que définie ci-dessus dans laquelle n représente l'entier 0 et Z représente le radical -CH₂- quand n est égal à 1, notamment dans un alcool tel que le butanol à une température d'environ 80°C ou dans le DMF pour donner un produit de formule (VIII) telle que définie ci-dessus.

Les produits de formule (IV) sont soumis selon la voie 2) à l'action du produit de formule (VI) telle que définie ci-dessus dans laquelle Z représente -SO₂, notamment dans du THF, DME, Cs₂CO₃, K₂CO₃ ou encore Na₂CO₃ pour donner un produit de formule (IX) telle que définie ci-dessus.

Les produits de formule (IV) sont soumis selon la voie 3 à l'action du produit de formule (VII) telle que définie ci-dessus dans laquelle Z représente le radical -(CH₂)₂NHR₃-, notamment dans du butanol à une température d'environ 75°C pendant environ 2 ou 3 heures pour donner un produit de formule (X) telle que définie ci-dessus.

Le produit de formule (X) ainsi obtenu peut être soumis à l'action d'un produit de formule (XI) ou (XI)_{A} tel que défini ci-dessus dans DME, Cs₂CO₃ ou encore CH₂Cl₂ et N(Et)₃ pendant une heure environ à température ambiante pour donner respectivement un produit de formule (XII) ou (XII)_{A} telle que définie ci-dessus.

Le produit de formule (X) peut également être soumis à l'action d'un aldéhyde de formule (XVII) notamment dans dans un alcool tel que le méthanol ou l'éthanol, en présence de NaBH₄ ou NaBH₃CN. pour donner un produit de formule (XIII) telle que définie ci-dessus.

Pour les autres valeurs de Z, les produits correspondants sont préparés selon la voie 4 du procédé comme suit : les produits de formule (IV) sont soumis selon la voie 4 à l'action du produit de formule (XVIII) dans laquelle Z représente CO pour donner un produit de formule (M₁) telle que définie ci-dessus.

La réaction du produit de formule (IV) avec le produit de formule (XVIII) peut être réalisée dans les mêmes conditions que celles de la réaction du produit de formule (IV) avec le produit de formule VI pour donner le produit de formule (IX) dans lequel quand Z représente SO₂.

Les produits de formule (IV) sont soumis à l'action de l'ammoniac pour donner un produit de formule (XIX).

Les produits ainsi obtenus de formules (VIII), (IX), (XII), (XII)_{A}, (XIII) et (M₁), telles que définies ci-dessus sont soumis selon la voie a) à l'action d'un composé de formule (XIV) telle que définie ci-dessus dans laquelle Y représente -NR₅, pour une réaction de condensation qui le cas échéant peut être réalisée à une température d'environ 140°C: une telle réaction de condensation peut être suivie d'une réaction de salification en présence d'acide chlorhydrique par exemple ou encore d'acide tartrique, citrique ou méthane sulfonique, dans un alcool tel que par exemple l'éthanol ou le méthanol pour donner des produits de formule (Ix) telle que définie ci-dessus.

Les produits de formule (Ix) sont donc des produits de formule (I) dans laquelle les fonctions éventuellement réactives sont éventuellement protégées et dans laquelle Y représente -NR₅- telle que définie ci-dessus.

Les produits de formules (VIII),(IX), (XII), (XII)_{A}, (XIII) et (M₁), telles que définies ci-dessus peuvent également être soumis selon la voie b) à l'action d'un composé de formule (XV) telle que définie ci-dessus dans laquelle Y représente l'atome d'oxygène, pour une réaction de condensation par exemple en présence de NaH dans le THF ou DMF à température ambiante ou en chauffant : une telle réaction de condensation peut être suivie d'une réaction de salification en présence d'acide chlorhydrique par exemple ou encore d'acide tartrique, citrique ou méthane sulfonique, dans un alcool tel que par exemple l'éthanol ou le méthanol pour donner des produits de formule (Iy) telle que définie ci-dessus.

Les produits de formule (Iy) sont donc des produits de formule (I) dans laquelle les fonctions éventuellement réactives sont éventuellement protégées et dans laquelle Y représente -O- telle que définie ci-dessus.

La fonction amine des composés de formules (Ix) et (Iy) telles que définies ci-dessus, protégées par un groupe tel que Boc ou CH₂-phényle peut être libérée dans les conditions usuelles connues de l'homme du métier.

La réaction de saponification peut être réalisée selon les méthodes usuelles connues de l'homme du métier, telles que par exemple dans un solvant tel que le méthanol ou l'éthanol, le dioxane ou le diméthoxyéthane, en présence de soude ou de potasse.

Les réactions de réduction ou oxydation du produit de formule (Ix) en produit de formule (I) peuvent être réalisées selon les méthodes usuelles connues de l'homme du métier.

Selon les valeurs de R₁', R₂', R₅', R₆', R₃', Z', D₁' et D₂', les produits de formules (Ix) et (Iy) constituent ou non des produits de formule (I) et peuvent donner des produits de formule (I), ou être transformés en d'autres produits de formule (I) en étant soumis à une ou plusieurs des réactions a) à k) indiquées ci-dessus.

Ainsi les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthyl-silyle, tert-butyldiméthylsilyle, méthoxy-méthyle, tétrahydro-pyrannyle, benzyle ou acétyle,
- les groupements amino peuvent être protégés par exemple par les radicaux acétyle, trityle, benzyle, tert-butoxycarbonyle, benzyloxycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal ou l'éthylènedithiocétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :
- les fonctions acide peuvent être protégées par exemple sous forme d'esters formés avec les esters facilement clivables tels que les esters benzyliques ou terbutyliques ou des esters connus dans la chimie des peptides.

Les réactions auxquelles les produits de formules (Ix) et (Iy) telles que définies ci-dessus peuvent être soumis, si désiré ou si nécessaire, peuvent être réalisées, par exemple, comme indiqué ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
c) Les éventuels groupements alkylthio des produits décrits ci-dessus peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloro-perbenzoique ou encore par l'ozone, l'oxone, le périodate de sodium dans un solvant tel que par exemple le chlorure de méthylène ou le dioxanne à la température ambiante.
   L'obtention de la fonction sulfoxyde peut être favorisée par un mélange équimolaire du produit renfermant un groupement alkylthio et du réactif tel que notamment un peracide.
   L'obtention de la fonction sulfone peut être favorisée par un mélange du produit renfermant un groupement alkylthio avec un excès du réactif tel que notamment un peracide.
d) La réaction de transformation d'une fonction cétone en oxime peut être réalisée dans les conditions usuelles connues de l'homme de métier, telle que notamment une action en présence d'une hydroxylamine éventuellement O-substituée dans un alcool tel que par exemple l'éthanol, à température ambiante ou en chauffant.
e) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique.
   Les éventuelles fonctions carboxy libre des produits décrits ci-dessus peuvent être, si désiré, réduites en fonction alcool notamment par de l'hydrure de bore.
f) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
g) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
h) Les éventuelles fonctions nitrile des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit :
   J. Organometallic Chemistry., 33, 337 (1971) KOZIMA S.& coll.

   On peut noter que la réaction de transformation d'un carbamate en urée et notamment d'un sulfonylcarbamate en sulfonylurée, peut être réalisée par exemple au reflux d'un solvant comme par exemple le toluène en présence de l'amine adéquate.
   Il est entendu que les réactions décrites ci-dessus peuvent être effectuées comme indiqué ou encore, le cas échéant, selon d'autres méthodes usuelles connues de l'homme du métier.
i) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
   On trouvera une liste de différents groupements protec-teurs utilisables par exemple dans le brevet BF 2 499 995.
j) Les produits décrits ci-dessus peuvent, si désiré, faire l'objet de réactions de salification par exemple par un acide minéral ou organique ou par une base minérale ou organique selon les méthodes usuelles connues de l'homme du métier.
k) Les éventuelles formes optiquement actives des produits décrits ci-dessus peuvent être préparées par dédoublement des racémiques selon les méthodes usuelles connues de l'homme du métier.

Des illustrations de telles réactions définies ci-dessus sont données dans la préparation des exemples décrits ci-après.

Les produits de formule (I) tels que définis ci-dessus ainsi que leurs sels d'addition avec les acides présentent d'intéressantes propriétés pharmacologiques.

Les produits de la présente invention tels que définis ci-dessus, possèdent des propriétés inhibitrices de kinases d'une grande sélectivité.

Les cdk jouent un rôle central dans l'initiation, le développement et l'achèvement des évènements du cycle cellulaire et ainsi, les molécules inhibitrices de cdk sont susceptibles de limiter des proliférations cellulaires non désirées telles que celles observées dans les cancers, psoriasis, croissance de champignons, de parasites (animaux, protistes) : de telles molécules inhibitrices de cdk sont ainsi également susceptibles d'intervenir dans la régulation de maladies neurodégénératives telles que la maladie d'Alzheimer.

Des kinases particulièrement sensibles aux effets inhibiteurs des dérivés de la présente invention sont notamment les cdk1, cdk2, cdk4, cdk5 et cdk7.

Les produits de la présente invention sont donc doués de propriétés antimitotiques.

Les produits de la présente invention possèdent en plus de leurs propriétés inhibitrices spécifiques de kinases, des effets cellulaires intéressants tels que des propriétés antiprolifératives et notamment des effets sur l'apoptose.

On sait par des travaux décrits dans la littérature tel que dans WO 97/20842, que des rapports existent entre le cycle cellulaire et l'apoptose. Parmi les voies conduisant à l'apoptose, certaines sont dépendantes de kinases.

Les produits de la présente invention sont notamment utiles pour la thérapie de tumeurs.

Les produits de l'invention peuvent également ainsi augmenter les effets thérapeutiques d'agents anti-tumoraux couramment utilisés.

Les produits de formule (I) de la présente invention possèdent donc tout particulièrement des propriétés antimitotiques et anti-neurodégénératives.

Ces propriétés justifient leur application en thérapeutique et l'invention a particulièrement pour objet à titre de médicaments, les produits de formule (I)telle que défnie ci-dessus,lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a ainsi plus particulièrement pour objet à titre de médicaments, les produits tels que définis par la formule (I) : 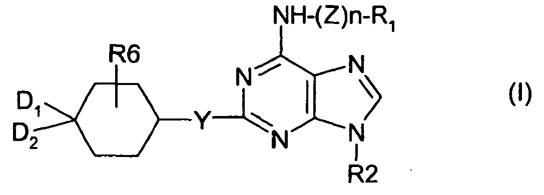 dans laquelle :
Z représente le radical divalent -CH₂-, -SO₂-, -CO-, -(CH₂)₂-NH-, -(CH₂)₂-Nalkyle, -(CH₂)₂-N-CH₂-phényle dans lesquels les radicaux phényle sont éventuellement substitués par un atome d'halogène, un radical hydroxyle, trifluorométhyle, alcoxy renfermant au plus 4 atomes de carbone ou carboxy libre, salifié ou estérifié,
n représente l'entier 0 ou 1,
R₁ est choisi parmi l'atome d'hydrogène et les radicaux phényle, -CH₂-phényle, -SO₂-phényle, -CO-phényle, pyridyle, alkyle et -SO₂-alkyle, dans lesquels les radicaux alkyle renferment au plus 4 atomes de carbone et sont éventuellement substitués par un radical carboxy libre, salifié ou estérifié, et tous les radicaux phényle sont éventuellement substitués par un ou plusieurs radicaux choisis parmi les atomes d'halogène et les radicaux hydroxyle, cyano, nitro, trifluorométhyle, trifluorométhoxy, thioalkyle et alcoxy renfermant au plus 4 atomes de carbone, alkyle renfermant au plus 4 atomes de carbone éventuellement substitué par un radical cyano, -COOH ou COOalk, les radicaux phényle, tétrazolyle, cycloalkyle interrompu par un ou plusieurs atomes d'oxygène ou d'azote, les radicaux -SO₂NH₂ et SO₂-NH-thiazolyle, les radicaux dioxol, carboxy libre, estérifié ou salifié et les radicaux -NHR₄ et -CONHR₄ dans lesquels R₄ représente un atome d'hydrogène, un radical alkyle renfermant au plus 4 atomes de carbone ou un radical cyclohexyle éventuellement substitué par un radical NH₂,
R₂ représente les radicaux cyclopentyle, tétrahydrofuryle ou le radical tétrahydrothiényle,
Y représente l'atome d'oxygène ou le radical -NH ou -Nalkyle dans lequel le radical alkyle linéaire ou ramifié renferme au plus 4 atomes de carbone,
D₁ et D₂ soit, identiques ou différents, sont choisis parmi l'atome d'hydrogène, le radical hydroxyle, les radicaux alkyle et alcoxy linéaires ou ramifiés renfermant au plus 4 atomes de carbone et les radicaux -NH₂, -NH-COOtBu ou
-NHalkyle dans lequel le radical alkyle linéaire ou ramifié renferme au plus 4 atomes de carbone, soit forment ensemble le radical =O ou =N-Oalkyle, dans lequel le radical alkyle linéaire ou ramifié renferme au plus 4 atomes de carbone,
R₆ représente l'atome d'hydrogène, un atome d'halogène ou le radical hydroxyle,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

L'invention a encore plus particulièrement pour objet à titre de médicaments, les produits de formule (I) telle que définie ci-dessus par la formule (Id): 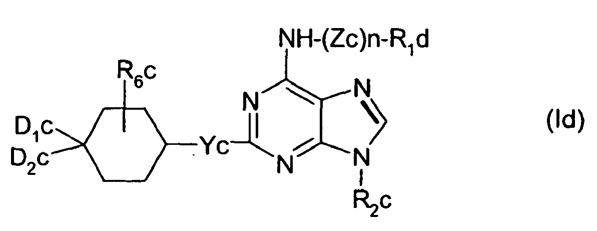 dans laquelle:
Zc représente le radical divalent -CH₂-, -SO₂- -CO-, -(CH₂)₂-NH-, -(CH₂)₂-Nalkyle, -(CH₂)₂-N-CH₂-phényle dans lesquels les radicaux phényle sont éventuellement substitués par un atome d'halogène, un radical hydroxyle, trifluoro-méthyle, alcoxy renfermant au plus 4 atomes de carbone ou carboxy libre, salifié ou estérifié,
n représente l'entier 0 ou 1,
R₁d est choisi parmi l'atome d'hydrogène et les radicaux phényle, -CH₂-phényle, -SO₂-phényle, -CO-phényle, alkyle et
-S0₂-alkyle, dans lesquels les radicaux alkyle renferment au plus 4 atomes de carbone et sont éventuellement substitués par un radical carboxy libre, salifié ou estérifié, et tous les radicaux phényle sont éventuellement substitués par un ou plusieurs radicaux choisis parmi les atomes d'halogène et les radicaux hydroxyle, cyano, nitro, trifluorométhyle, trifluorométhoxy, thioalkyle et alcoxy renfermant au plus 4 atomes de carbone, alkyle renfermant au plus 4 atomes de carbone éventuellement substitué par un radical cyano ou carboxy libre ou estérifié, les radicaux morpholinyle, phényle, tétrazolyle, -SO₂NH₂, SO₂-NH-thiazolyl salifié, dioxol, carboxy libre, estérifié ou salifié, -NHR₄c et -CONHR₄c dans lesquels R₄c représente un atome d'hydrogène, un radical alkyle renfermant au plus 4 atomes de carbone ou un radical cyclohexyle éventuellement substitué par un radical NH₂,
R₂c représente les radicaux cyclopentyle, tétrahydrofuryle ou le radical tétrahydro-thiényle,
Yc représente l'atome d'oxygène ou le radical -NH ou -N-alkyle dans lequel le radical alkyle linéaire ou ramifié renferme au plus 4 atomes de carbone,
D₁c et D₂c soit, identiques ou différents, sont choisis parmi l'atome d'hydrogène, le radical hydroxyle, les radicaux alkyle et alcoxy linéaires ou ramifiés renfermant au plus 4 atomes de carbone et les radicaux -NH₂, -NH-COOtBu ou
-NH-alkyle dans lequel le radical alkyle linéaire ou ramifié renferme au plus 4 atomes de carbone, soit forment ensemble le radical =O ou =N-Oalkyle, dans lequel le radical alkyle linéaire ou ramifié renferme au plus 4 atomes de carbone,
R₆c représente l'atome d'hydrogène, un atome d'halogène ou le radical hydroxyle,
lesdits produits de formule (Id) étant sous toutes les formes isomères possibles racémiques, énantiomères et diastéréo-isomères, ainsi que les sels d'addition avec les acides minéraux et organiques ou avec les bases minérales et organiques desdits produits de formule (Id).

L'invention a tout particulièrement pour objet, à titre de médicaments, les produits décrits ci-après dans les exemples et notamment les produits de formule (I) telle que définie ci-dessus, répondant aux formules suivantes :
- Dichlorhydrate de trans-4-[[[2-[(4-aminocyclohexyl)-amino]-9-cyclopentyl-9H-purin-6-y]-amino]-méthyl]-benzoate de butyle,
- Dichlorhydrate de trans-4-[[2-[(4-aminocyclohexyl)-amino]-9-cyclopentyl-9H-purin-6-yl]-amino]-benzoate d'éthyle,
- Trichlorhydrate de trans-N2-(4-aminocyclohexyl)-9-cyclo-pentyl-N6-[2-[(phénylméthyl)-amino]-éthyl]-9H-purin-2,6-diamine,
- Trichlorhydrate de trans-N2-(4-aminocyclohexyl)-N6-(2-aminoéthyl)-9-cyclopentyl-9H-purin-2,6-diamine,
- Trichlorhydrate de trans-N2-(4-aminocyclohexyl)-9-cyclo-pentyl-N6-[2-[[(4-méthoxyphényl)-méthyl]-amino]-éthyl]-9H-purin-2,6-diamine,
- Trichlorhydrate de trans-N2-(4-aminocyclohexyl)-N6-[2-[[[4-chloro-3-(trifluorométhyl)-phényl]-méthyl]-amino]-éthyl]-9-cyclopentyl-9H-purin-2,6-diamine,
- Trichlorhydrate de trans-N2-(4-aminocyclohexyl)-9-cyclo-pentyl-N6-[(diphénylméthyl)-amino]-éthyl]-9H-purin-2,6-diamine,
- Trichlorhydrate de trans-N2-(4-aminocyclohexyl)-N6-[2-[[(4-chlorophényl)-méthyl]-amino]-éthyl]-9-cyclopentyl-9H-purin-2,6-diamine,
- Dichlorhydrate de trans(+-)-4-[[2-[(4-aminocyclohexyl)-amino]-9-(tétrahydro-3-thiényl)-9H-purin-6-yl]-amino]-benzoate d'éthyle,
- Dichlorhydrate de trans(+-)-N2-(4-aminocyclohexyl)-9-(tétrahydro-3-thiényl)-N6-[4-(trifluorométhoxy)-phényl]-9H-purin-2,6-diamine,
- Dichlorhydrate de trans(+-)-N2-(4-aminocyclohexyl)-9-(tétrahydro-3-furanyl)-N6-[(4-trifluorométhoxy)-phényl]-9H-purin-2,6-diamine,

La présente invention a encore tout particulièrement pour objet, à titre de médicaments, les produits de formule (I) telle que définie ci-dessus, répondant aux formules suivantes :
- Dichlorhydrate de trans -3-[[2-[(4-aminocyclohexyl)-amino]-9-cyclopentyl-9H-purin-6-yl]-amino]-benzoate d'éthyle,
- Trichlorhydrate de trans-N2-(4-aminocyclohexyl)-9-cyclopentyl-N6-[2-[[(3,4-dichlorophényl)-méthyl]-amino]-éthyl]-9H-purine-2,6-diamine,
- Trichlorhydrate de trans-N2-(4-aminocyclohexyl)-9-cyclopentyl-N6-[2-[[(3,5-dichlorophényl)-méthyl]-amino]-éthyl]-9H-purine-2,6-diamine,
- Dichlorhydrate de trans-4-[[2-[(4-aminocyclohexyl)-amino]-9-cyclopentyl-9H-purin-6-yl]amino]benzène-acétonitrile,
- Dichlorhydrate de trans-N2-(4-aminocyclohexyl)-9-cyclopentyl-N6-[4-(4-morpholinyl)-phényl]-9H-purine-2,6-diamine,
- Dichlorhydrate de trans-4-[[2-[(4-aminocyclohexyl)-amino]-9-cyclopentyl-9H-purin-6-yl]amino]-benzonitrile,
- Dichlorhydrate de trans-N2-(4-aminocyclohexyl)-9-cyclopentyl-N6-(4-nitrophényl)-9H-purine-2,6-diamine,
- Dichlorhydrate de trans-N2-(4-aminocyclohexyl)-N6-(4-aminophényl)-9-cyclopentyl-9H-purine-2,6-diamine,
- Dichlorhydrate de trans-N2-(4-aminocyclohexyl)-9-cyclopentyl-N6-(4-méthoxyphényl)-9H-purine-2,6-diamine,
- Dichlorhydrate de trans-5-[[2-[(4-amino-cyclohexyl)-amino]-9-cyclopentyl-9H-purin-6-yl]-amino]-1,3-benzènedicarboxylate de diéthyle.

Les médicaments, objet de l'invention, trouvent, par exemple, comme antimitotiques, leur emploi dans la chimiothérapie des cancers, ou encore dans le traitement de psoriasis, de parasitoses telles que celles dues à des protistes ou à des champignons ou encore dans le traitement de la maladie d'Alzheimer ou dans le traitement de l'apoptose neuronale.

L'invention s'étend aux compositions pharmaceutiques contenant à titre de principe actif l'un au moins des médicaments tels que définis ci-dessus.

De telles compositions pharmaceutiques de la présente invention peuvent également, le cas échéant, renfermer des principes actifs d'autres médicaments antimitotiques tels que notamment ceux à base de taxol, cis-platine, les agents intercalants de l'ADN et autres.

Ces compositions pharmaceutiques peuvent être administrées par voie buccale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 0,05 à 5 g par jour chez l'adulte, ou de préférence de 0,1 à 2 g par jour.

Le produit de départ de formule (II) soit la dichloro-2,6-purine est connu et commercialisé.

Parmi les produits de départ de formules (III), (V), (VI), (VII), (XI), (XI)_{A}, (XIV), (XV) et (XVI), certains sont connus et peuvent être obtenus commercialement ou peuvent être préparés selon les méthodes usuelles connues de l'homme du métier.

Parmi les produits de départ commerciaux de formules (III), (V), (VI), (VII), (XI), (XI)_{A}, (XIV), (XV) et (XVI), on peut citer par exemple, les produits de formule (III) suivants : cyclopentanol, le 3-hydroxytétrahydrofuranne, le 3-propanol, le 3-hydroxythiophène ou encore le 2-butanol.

Parmi les produits commerciaux de formule (V), on peut citer le produit chlorhydrate de 4-(aminométhyl)-benzoate de méthyle, l'éthyl-4-aminobenzoate, le 4-aminobenzamide, le méthyl-3-aminobenzoate ou encore la 3-aminobenzamide.

Comme produits commerciaux de formule (XIV), on peut citer le trans-1,4-diaminocyclohexane ou encore trans-4-aminocyclohexanol.

On peut encore notamment préparer certains produits de départ à partir de produits de commerciaux par exemple en les soumettant à une ou plusieurs des réactions décrites ci-dessus en a) à l), réalisées dans les conditions également décrites ci-dessus.

On peut citer encore à titre d'exemple :
- comme produit de formule (VI), le phénylsulfonamide, le 3-bromophénylsulfonamide, le 4-terbutylphénylsulfonamide,
- comme produit de formule (VII), l'éthylènediamine,
- comme produit de formule (XI) le chlorure d'isopropylsulfonyle, le chlorure de paraméthoxyphénylsulfonyle ou encore le chlorure de trifluorométhanesulfonyle,
- comme produit de formule (XI)_{A}, le chlorure de 4-trifluorométhylbenzoyle, le chlorure de 4-anisoyle, le chlorure 4-chlorobenzoyle, le chlorure 2-chloro-4-nitro-benzoyle,
- comme produit de formule (XVII), le benzaldéhyde, le paraméthoxybenzaldéhyde ou encore le paracyanobenzaldéhyde.

La partie expérimentale ci-après donne des exemples de tels produits de départ.

La présente invention a enfin pour objet à titre de produits industriels nouveaux, les composés de formules (IX), (X), (XII), (XII)_{A}, (XIII) et (M₁).

L'invention a ainsi particulièrement pour objet les compositions pharmaceutiques contenant à titre de principe actif, l'un au moins des médicaments tels que définis ci-dessus.

L'invention a tout particulièrement pour objet les compositions pharmaceutiques telles que définies ci-dessus caractérisées en ce qu'elles sont utilisées comme médicaments antimitotiques, en particulier pour la chimiothérapie de cancers ou encore pour le traitement de psoriasis, de parasitoses telles que celles dues à des champignons ou à des protistes ou de la maladie d'Alzheimer.

L'invention a également tout particulièrement pour objet les compositions pharmaceutiques telles que définies ci-dessus caractérisées en ce qu'elles sont utilisées comme médicaments antineurodégénératifs notamment anti-apoptose neuronale.

L'invention a notamment pour objet l'utilisation des produits de formule (I) telle que définie ci-dessus pour la préparation de médicaments destinés à la chimiothérapie de cancers, au traitement de psoriasis, de parasitoses telles que celles dues à des champignons ou à des protistes, au traitement de la maladie d'Alzheimer ou au traitement d'affections neurodégénératives notamment l'apoptose neuronale.

Les exemples suivants illustrent l'invention sans toutefois la limiter.

### EXEMPLE 1 : Dichlorhydrate de trans-4-[[[2-[(4-aminocyclohexyl)amino]-9-cyclopentyl-9H-purin-6-yl]-amino]-méthyl]-benzoate de butyle.

### Stade 1 : 9-cyclopentyl-2,6-dichloro-9H-purine

On mélange 378 mg de dichloro-2,6-purine, 5 ml de tétrahydrofuranne, 0,27 ml de cyclopentanol, 787 mg de triphenylphosphine (P(phényl)3) et 0,46 ml de DEAD (diethylazodicarboxylate) et agite une nuit à température ambiante puis évapore à sec. Le produit brut est purifié par chromatographie sur colonne de silice avec pour éluant CH2Cl2 50, AcOEt 25, Cyclohexane 25. On obtient ainsi 400 mg de produit attendu.

### RMN dans CDCl₃

| | |
|---|---|
| 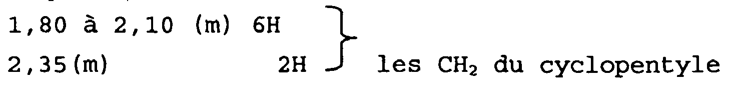 | |
| 4,98 (m) | =C-N-CH-CH₂ |
| 8,15 (s) 8,16 (s) | -CH=N |

### Spectre IR CHCl₃

1591 ; 1557 ; 1491 cm-1 hétérocycle
1747 cm-1 C=O

### Stade 2 : 4-[[(2-chloro-9-cyclopentyl-9H-purin-6-yl)-amino]-méthyl]-benzoate de méthyle

On mélange 450 mg du produit obtenu au stade 1 ci-dessus, 10 ml de butanol, 347 mg de chlorhydrate de 4-(aminométhyl)-benzoate de méthyle et 290 mg de carbonate de potassium, et agite à une température d'environ 100°C pendant environ 18 heures puis laisse revenir à température ambiante.
On ajoute alors 15 ml de H₂O, extrait par 2 x 50 ml de CH₂C1₂ (chlorure de méthylène), sèche, filtre et évapore. On empâte alors dans l'éther isopropylique et sèche sous vide à environ 50°C. On obtient ainsi 526 mg de produit attendu sous forme de cristaux incolore.

### Spectre IR CHCl₃

NH 3424 cm-1
>=0 1720 cm-1
hétérocycle et aromatique 1619 ; 1575 ; 1528 ; 1499 cm-1

### Stade 3 : Dichlorhydrate de trans-4-[[[2-[(4-aminocyclohexyl)-amino]-9-cyclopentyl-9H-purin-6-yl]-amino]-méthyl]-benzoate de butyle.

On mélange 50 mg du produit obtenu au stade 2 ci-dessus, 4 ml de butanol, 150 mg de trans-1,4-diaminocyclohexane, chauffe à environ 150°C pendant environ 24 heures, laisse revenir à température ambiante. On ajoute alors 4 ml d'éther, essore et rince à l'éther puis sèche à température ambiante.
On reprend dans 4 ml d'éthanol, ajoute 2 ml d'HCl (acide chlorhydrique) à 1,4N dans de l'éthanol puis évapore. On obtient ainsi 25 mg de produit attendu.

### RMN dans DMSO

| | |
|---|---|
| 0,92 (t) | |
| 1,40 (m) | |
| 1,67 (m) | |
| 4,23 (t) | O-C= |
| 1,20 à 3,10 | les C-CH₂ |
| 3,60 (masqué) | =C-N-CH + NH₃-CH |
| 4,75 (m) | phényl-N-CH |
| 4,85 (sl) | phényl-CH₂-N-C= |
| 7,55 à 7,90 AA'BB' | =C-phényl-O |
| 8,32 (sl) | N=C-CH-N |

### EXEMPLE 2 : Dichlorhydrate de trans(+-)-N2-(4-aminocyclohexyl)-N6-(phénylméthyl)-9-(tétrahydro-3-furanyl)-9H-purine-2,6-diamine.

### Stade 1 : (+-)-2,6-dichloro-9-(tetrahydro-3-furanyl)-9H-purine.

On mélange 945 mg de dichloro-2,6-purine, 660 mg de 3-hydroxytétrahydrofuranne, 7,96 g de triphénylphosphine (P(phenyl)3), 20 ml de tétrahydrofuranne puis ajoute en 10 minutes 1,16 g de DEAD (diéthylazodicarboxylate) et agite une nuit à température ambiante. On verse sur un solution aqueuse 1M de NaH₂PO₃. On extrait alors par 3 fois avec 20 ml d'acétate d'éthyle, lave avec 20 ml d'eau, puis avec 10 ml de solution aqueuse saturée de NaCl, sèche et évapore à sec.

Après chromatographie sur silice avec pour éluant chlorure de méthylène/acétate d'éthyle/CH₃CN en proportion de 70/15/15 puis une seconde chromatographie sur silice avec pour éluant chlorure de méthylène/acétate d'éthyle en proportion de 50/50, on obtient ainsi 878 mg de produit attendu sous forme de cristaux blancs.

### RMN dans DMSO

| | |
|---|---|
| 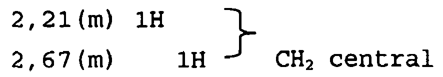 | |
| 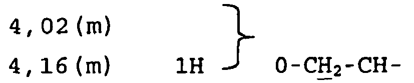 | |
| 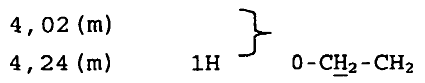 | |
| 5, 38 (m) 1H | CH₂-CH-N |
| 8,26 1H | H₂ |

### Stade 2 : (+-)-2-chloro-N-(phénylméthyl)-9-(tétrahydro-3-furanyl)-9H-purin-6-amine.

On mélange 139 mg du produit obtenu au stade 1 ci-dessus, 2 ml de butanol et 0,06 ml de benzylamine et chauffe à une température d'environ 95°C pendant 5 heures 30 minutes. On laisse alors revenir à température ambiante, laisse cristalliser, essore, lave par 10 ml d'isopropanol et sèche sous vide à environ 50°C. On obtient ainsi 157 mg de produit attendu sous forme de cristaux blancs.

### RMN dans DMSO

| | |
|---|---|
| 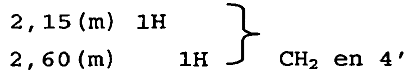 | |
| 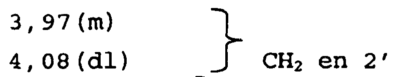 | |
| 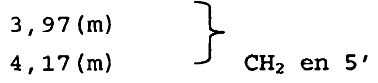 | |
| 4,83 (s large) | NH-CH₂-phényle |
| 6,50 (large) | NH-CH₂-phényle |
| 5, 30 (m) | H₃' |
| 7,25 à 7,40 (m) | 5H aromatiques |
| 7,84(s) | H₂ |

### Stade 3 : Dichlorhydrate de trans(+-)-N2-(4-aminocyclohexyl)-N6-(phényl-méthyl)-9-(tétrahydro-3-furanyl)-9H-purine-2,6-diamine.

On mélange 656 mg de trans-1,4-diaminocyclohexane et 133 mg du produit obtenu au stade 2 ci-dessus et porte à une température de 130 à 150°C pendant environ 5 heures puis laisse une nuit à température ambiante. On reprend alors par 10 ml d'eau et 20 ml d'acétate d'éthyle, laisse décanter, ré-extrait par 2 x 5 ml d'acétate d'éthyle, lave par 10 ml d'eau et 10 ml de chlorure de sodium en solution aqueuse saturée, sèche et évapore à sec. Après purification sur silice avec pour éluant méthanol/ammoniaque (NH₄OH) en proportion de 98/2, on ajoute 1,5 ml d'acide chlorhydrique à 1,4 N dans l'éthanol et laisse cristalliser. On dilue dans 2 ml d'acétate d'éthyle et laisse une heure à température ambiante. On essore, lave avec 5 ml d'acétate d'éthyle et sèche à une température d'environ 50°C. On obtient ainsi 98 mg de produit attendu sous forme de cristaux blanc/crème.

### RMN dans DMSO

| | |
|---|---|
| 1,40 (m) 4H | les H axiaux du cyclohexyle |
| 2,04 (d) 4H | les H équatoriaux du cyclohexyle |
| 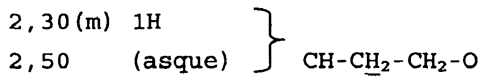 | |
| 3,02 (m,large) 1H | H₄ axial |
| 3,72 (t,large) 1H | H₁ axial |
| 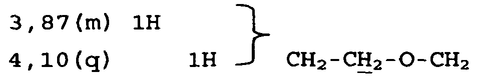 | |
| 3,99 (d) 2H | CH₂-CH₂-O-CH₂ |
| 4,90 (s large) | NH-CH₂-phényle |
| 5,08 (m) 1H | NH-CH₂- CH₂-O |
| 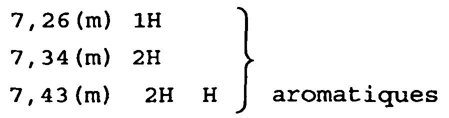 | |
| 8,08 (sl) >2H | NH₂ (salifié) |
| 8,23 (s) 1H | N=CH-N |
| 9,39 1H | mobile |

### PRODUIT 3 : (2R,3S)-2,3-dihydroxybutanedioate de trans-N2-(4-aminocyclohexyl)-9-(1-éthylpropyl)-N6-(phénylméthyl)-9H-purine-2,6-diamine.

### Stade 1 : 2,6-dichoro-9-(1-éthylpropyl)-9H-purine.

On mélange 1,32 mg de dichloro-2,6-purine, 2,75 g de triphénylphosphine (P(phényl)3), 35 ml de tétrahydrofuranne, 1,13 ml de 3-pentanol (10,5 mmoles), agite à température ambiante et ajoute en 15 minutes 1,63 ml de DEAD (diéthylazodicarboxylate) (10,5 mM) et agite environ 20 heures à température ambiante. On verse sur une solution aqueuse 1M de NaH₂PO₄, extrait par 3 fois avec 10 ml d'acétate d'éthyle, lave avec 10 ml d'eau puis avec 10 ml de chlorure de sodium en solution aqueuse saturée, sèche et évapore à sec. Après chromatographie sur silice avec pour éluant chlorure de méthylène/acétate d'éthyle en proportion de 90/10 puis une seconde chromatographie sur silice avec même éluant, on obtient ainsi 1,12 g de produit attendu sous forme de cristaux blancs.

### Stade 2 : 2-chloro-9-(1-éthylpropyl)-N-(phénylméthyl)-9H-purin-6-amine.

On mélange 191 mg du produit obtenu au stade 1 ci-dessus, 2,5 ml de butanol et 0,115 ml de benzylamine et chauffe à une température d'environ 90 à 110°C pendant 5 heures. On laisse alors revenir à température ambiante, laisse cristalliser, essore, lave par 10 ml d'isopropanol et sèche sous vide à environ 50°C. On obtient ainsi 148 mg de produit attendu sous forme de cristaux blancs.

### Stade 3 : (2R,3S)-2,3-dihydroxybutanedioate de trans-N2-(4-aminocyclohexyl)-9-(1-éthylpropyl)-N6-(phénylméthyl)-9H-purine-2,6-diamine.

On mélange 456 mg de trans-1,4-diaminocyclohexane et 131 mg du produit obtenu au stade 2 ci-dessus et porte à une température de 140 à 150°C pendant 4 heures puis laisse revenir à température ambiante On dilue alors avec 5 ml d'eau et 5 ml d'acétate d'éthyle, laisse décanter, ré-extrait par 2 x 10 ml d'acétate d'éthyle, lave par 10 ml d'eau et 5 ml de chlorure de sodium en solution aqueuse saturée, sèche et évapore à sec. Après purification sur silice avec pour éluant méthanol/ammoniaque (NH₄OH) en proportion de 98/2, on salifie par une solution 1M d'acide mésotartrique dans l'éthanol, laisse cristalliser pendant une nuit à température ambiante. On essore, lave avec 10 ml d'acétate d'éthyle et sèche à une température d'environ 50°C. On obtient ainsi 97 mg de produit attendu sous forme de cristaux beige rosé.

### RMN dans DMSO

| | |
|---|---|
| 0,70 (t) 6H | (CH₃-CH₂)₂-CH |
| 1, 81 (m) 4H | (CH₃-CH₂)₂-CH |
| 1, 22 (m) | |
| 1,37 (m) 4H | les H axiaux du cyclohexyle |
| 1,92 (d) | les H équatoriaux du cyclohexyle |
| 2,95 (t) | H₄ axial |
| 3,57 (m, large) | H₁ axial |
| 3,87 (s) 2H | NH-CH₂-phényle |
| 4,04 (m) 1H | =C-N-CH |
| 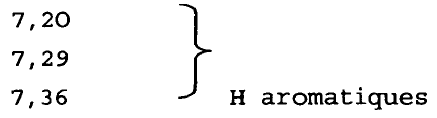 | |
| 7,85 (s) 1H | N-CH-N= |
| 6,16 (d) 1H | NH-CH |
| 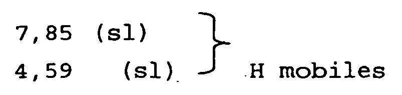 | |

### EXEMPLE 4 : Chlorhydrate de trans-9-cyclopentyl-N2-(4-hydroxycyclohexyl)-N6-(phénylméthyl)-9H-purine-2,6-diamine.

### Stade 1 : 2-chloro-9-cyclopentyl-N-(phénylmethyl)-9H-purin-6-amine.

On mélange 1,03 g du produit obtenu au stade 1 de l'exemple 1 ci-dessus, 15 ml de butanol et 0,54 ml de benzylamine et chauffe à une température d'environ 90 à 100°C pendant 4 heures. On laisse alors revenir à température ambiante et laisse une nuit. On dilue avec 10 ml d'isopropanol, laisse une heure à température ambiante, essore, lave par 20 ml d'isopropanol et sèche sous vide à environ 50°C. Après purification sur silice avec pour éluant chlorure de méthylène/acétate d'éthyle en proportion de 90/10 puis recristallisation dans un minimum d'isopropanol, on sèche sous vide à environ 50°C et obtient 114 mg de produit attendu sous forme de cristaux blancs.

### Stade 2 : Chlorhydrate de trans-9-cyclopentyl-N2-(4-hydroxycyclohexyl)-N6-(phénylméthyl)-9H-purine-2,6-diamine.

On mélange 1 g de trans-1,4-aminocyclohexanol que l'on porte à une température de 50 à 60°C puis ajoute 212 mg du produit obtenu au stade 1 ci-dessus et porte à une température de 140 à 150°C pendant environ 4 heures. On laisse alors revenir à une température de 100°C, ajoute 10 ml d'eau, laisse décanter, ajoute 10 ml d'eau, 20 ml d'acétate d'éthyle et porte à une température d'environ 70°C. On ajoute alors 10 ml d'eau et laisse une nuit à température ambiante. On laisse alors décanter, ré-extrait par 2 x 20 ml de chlorure de méthylène à 20 % de méthanol, joint les phases organiques, lave par 10 ml d'eau et 10 ml de chlorure de sodium en solution aqueuse saturée, sèche et évapore à sec. On dissout alors dans le minimum d'éthanol, ajoute de l'acide chlorhydrique à 1,4N dans l'éthanol et laisse cristalliser. On dilue dans 5 ml d'éthanol puis laisse une heure à température ambiante. On essore, lave avec 10 ml d'éthanol et sèche à une température d'environ 50°C. On obtient ainsi 215 mg de produit attendu sous forme de cristaux blancs.

### RMN dans DMSO

| | |
|---|---|
| 1,30 (m) 4H | les H axiaux centraux du cyclohexyle |

| | |
|---|---|
| 1, 70 (m) 2H | |
| 1,80 à 2,10 | |
| 2,18 (m) 2H | les CH₂ du cyclopentyle |
| 1,80 à 2,10 | les H équatoriaux centraux du cyclohexyle |
| 3,45 (m, large) 1H | H₄ axial |
| 3,71 (m, large) 1H | H₁ axial |
| 4,75 (m) 1H | -CH cyclopentyle |
| 4,89 (s, large) 2H | N-CH₂-phényle |
| 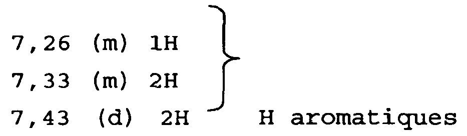 | |
| 8,28 | =N-CH=N |
| 9,41 à 5,94 1H | mobile |

### EXEMPLE 5 : Dichlorhydrate de trans(+-)-N2-(4-aminocyclohexyl)-N6-(phénylméthyl)-9-(tétrahydro-3-thiényl)-9H-purine-2,6-diamine.

### Stade 1 : 2,6-dichloro-9-(tétrahydro-3-thiényl)-9H-purine.

On mélange 312 mg de 3-hydroxythiophène, 380 mg de 2,6-dichloropurine, 6 ml de tétrahydrofuranne, 786 mg de triphénylphosphine (P(phënyl)3), mélange à température ambiante puis ajoute en 10 minutes 0,47 ml de DEAD (diéthylazodicarboxylate) et agite une nuit à température ambiante. On ajoute alors 10 ml de NaH2PO4 en solution aqueuse 1M, extrait par 3 fois avec 10 ml de chlorure de méthylène, lave avec 10 ml d'eau avec 5 de chlorure de sodium en solution aqueuse saturée, sèche et évapore à sec. Après purification sur silice avec pour éluant chlorure de méthylène/acétate d'éthyle en proportion de 75/25 puis 90/10, on empâte à température ambiante dans 5 ml d'éther isopropylique, essore, lave avec 5 ml d'éther isopropylique et sèche à température ambiante. On obtient ainsi 137 mg de produit attendu sous forme de cristaux blancs.

### Stade 2 : 2-chloro-N-(phénylméthyl)-9-(tétrahydro-3-thiényl)-9H-purin-6-amine.

On mélange 120 mg du produit obtenu au stade 1 ci-dessus, 2 ml de butanol et O, 105 ml de benzylamine et chauffe à une température d'environ 95 à 100°C pendant 10 heures. On laisse alors revenir à température ambiante et dilue alors dans 5 ml d'isopropanol, essore, lave par 5 ml d'isopropanol et sèche sous vide à environ 30°C. On obtient ainsi 132 mg de produit attendu sous forme de cristaux blancs.

### Stade 3 : Dichlorhydrate de trans(+-)-N2-(4-aminocyclohexyl)-N6-(phénylméthyl)-9-(tétrahydro-3-thiényl)-9H-purine-2,6-diamine.

On porte 400 mg de trans-1,4-diaminocyclohexane à une température de 60 à 70°C, ajoute alors 119 mg du produit obtenu au stade 2 ci-dessus et porte à une température de 130 à 140°C pendant 3 heures. On laisse revenir à température ambiante, ajoute 5 ml d'eau extrait par 3 x 10 ml d'acétate d'éthyle, lave par 10 ml d'eau et 5 ml de chlorure de sodium en solution aqueuse saturée, sèche et évapore à sec. Après purification sur silice avec pour éluant méthanol/ammoniaque (NH4OH) en proportion de 98/2, on salifie par de l'acide chlorhydrique à 1,4N dans l'éthanol et laisse cristalliser 2 jours à température ambiante. On essore, lave avec 10 ml d'éthanol et sèche à une température d'environ 50°C. On obtient ainsi 112 mg de produit attendu sous forme de cristaux blanc/crème.

### RMN dans DMSO

| | |
|---|---|
| 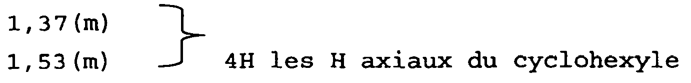 | |
| 2,04 (d) 4H | les H équatoriaux du cyclohexyle |
| 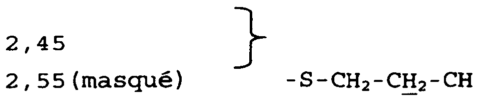 | |
| 2, 97 (m) | -S-CH₂-CH₂-CH |
| 3,02 (masqué) | H₄ supposé axial |
| 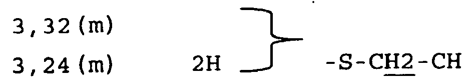 | |
| 3,73 (t) 1H | H₁ axial |
| 4,90 (s,l) | NH-CH₂-phényle |
| 5,07 (m) 1H | N=CH₂-N-CH- |
| 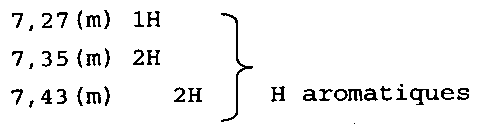 | |
| 8,33 (s) 1H | N=CH-N |
| 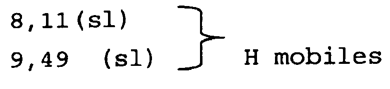 | |

### EXEMPLE 6 : Dichlorhydrate de trans-4-[[2-[(4-aminocyclohexyl)-amino]-9-cyclopentyl-9H-purin-6-yl]amino]-benzoate d'éthyle.

### Stade 1 : 4-[(2-chloro-9-cyclopentyl-9H-purin-6-yl)amino]-benzoate d'éthyle.

On introduit à température ambiante 86 mg du produit obtenu au stade 1 de l'exemple 1, 2 ml de nbutanol et 66 mg de éthyl-4-aminobenzoate et plonge dans un bain à la température d'environ 100°C pendant 7 heures sous agitation puis laisse revenir à température ambiante. On essore et rince à l'éther puis sèche sous vide. On obtient ainsi 74 mg de produit attendu sous forme de poudre beige.

### RMN dans DMSO

| | |
|---|---|
| 1,33 (t) 3H | -CO-CH₂-CH₃ |
| 4,30 (a) 2H | -CO-CH₂-CH₃ |
| 1, 72 (m) 2H | |
| 1,89 (m) 2H | |
| 2,01 (m) 2H | |
| 2,18 (m) 2H | CH₂ du cyclopentyle |
| 4,88 (q) 1H | CH₂-CH-N |
| 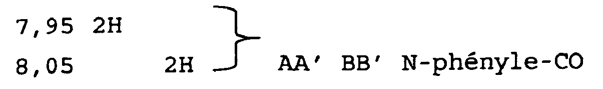 | |
| 8,53 (s) 1H | CH du cycle purine |
| 10,69 | NH |

### Stade 2 : Dichlorhydrate de trans-4-[[2-[(4-aminocyclohexyl)-amino]-9-cyclopentyl-9H-purin-6-yl]amino]-benzoate d'éthyle.

On porte 1,14 g de trans-1,4-diaminocyclohexane à environ 150°C puis ajoute 386 mg du produit obtenu au stade 1 ci-dessus : on laisse sous agitation pendant 3 heures 30 minutes puis laisse revenir à température ambiante. On ajoute alors 10 ml d'eau, essore, lave à l'eau et sèche à une température d'environ 50°C. On reprend 110 mg dans 10 ml de méthanol, ajoute 4 ml d'HCl à 1,4N dans l'éthanol puis concentre à ~4 ml : on laisse cristalliser, essore et lave à l'éthanol puis sèche à une température d'environ 50°C. On obtient ainsi 110 mg de produit attendu sous forme de poudre beige.

### RMN dans DMSO

| | |
|---|---|
| 1,32 (t) | CO₂-CH₂-CH₃ |
| 4,31 (q) | CO₂-CH₂-CH₃ |
| 1,39 (m) | |
| 1,52 (m) | CH₂ axiaux du cyclohexyle |
| 2,10 (m) | |
| 2,06 (m) | CH₂ équatoriaux du cyclohexyle |
| 3,03 (l) | |
| 3,68 (tl) | H axial isomère trans du cyclopentyle |
| 4,85 (l) | -N-CH du cyclopentyle |
| 2,16 (m) | CH₂ en alpha du cyclopentyle |
| 1,70 et 1,90 (m) | CH₂ en bêta du cyclopentyle |
| 7,98 (d) 2H | |
| 8,18 masqué 5H AA' BB' | -NH-phényle-C= + -N=CH- + -NH₂ |
| 7,58-9,28-11,13 | absorptions mobiles |

### EXEMPLE 7 : Trichlorhydrate de trans-N2-(4-aminocyclohexyl)-9-cyclopentyl-N6-[2-[(phénylméthyl)-amino]-éthyl]-9H-purine-2,6-diamine.

### Stade 1 : N-(2-aminoéthyl)-2-chloro-9-cyclopentyl-9H-purin-6-amine.

On mélange 3 g du produit obtenu au stade 1 de l'exemple 1, 21 ml de butanol, 7,5 ml de 1,2-éthane-diamine et porte à 75°C pendant 3 heures. On évapore le solvant et après chromatographie sur silice avec pour éluant chlorure de méthylène/méthanol/ammoniaque en proportion de 85/15/1,5, on obtient 2,73 g de produit attendu sous forme de résine jaune.

### Spectre IR CHCl₃

NH 3423 cm-1
>=O 1685 cm-1
hétérocycle 1619 ; 1576 ; 1530 ; 1498 cm-1

### Stade 2 : 2-chloro-9-cyclopentyl-N-[2-[(phénylméthyl)-amino]-éthyl]-9H-purin-6-amine.

On mélange 141 mg du produit obtenu au stade 1 ci-dessus, 2 ml de méthanol, 0,07 ml de benzaldéhyde, 0,1 ml d'acide acétique et 0,055 g de NaBH₃CN et laisse à température ambiante pendant environ 4 heures. On rajoute 10 ml AcOEt, lave par 2 x 5 ml H2O, puis 5 ml solution aqueuse saturé de NaCl. On sèche et évapore le solvant. Après chromatographie sur silice avec pour éluant CH₂Cl₂/méthanol/ammoniaque en proportion de 90/10/1, on obtient 100 mg de produit attendu.

### Stade 3 : Trichlorhydrate de trans-N2-(4-aminocyclohexyl)-9-cyclopentyl-N6-[2-[(phénylméthyl)-amino]-éthyl]-9H-purine-2,6-diamine.

On procède comme au stade 3 de l'exemple 1 à partir de 0,090 g du produit obtenu au stade 2 ci-dessus, 277 mg de trans-1,4-diaminocyclohexane, chauffe à environ 140°C pendant 2 heures. On purifie sur cartouche de silice avec CH₂Cl₂/méthanol/ammoniaque en proportion de 85/15/1,5. Le produit est salifié pour une solution de Hcl dans EOOH 1,4N. On obtient ainsi 70 mg de produit attendu.

### RMN dans DMSO

| | |
|---|---|
| 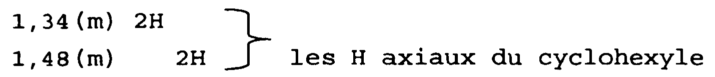 | |
| 1,70 (m) | |
| 1, 90 (m) | |
| 2,04 (m) | les CH₂ du cyclopentyle |
| 2,14 (m) | + les CH₂ équatoriaux du cyclohexyle |
| 3,03 (sl) | H₄ axial |
| 3,93 (sl) | H₁ axial |
| 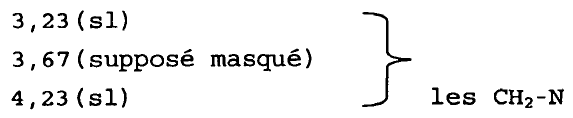 | |
| 4,75 (m) | CH du cyclopentyle |
| 7,42 (m) | |
| 7,56 (m) | -phényle- |
| 8,14 (s) | N-CH-N |
| 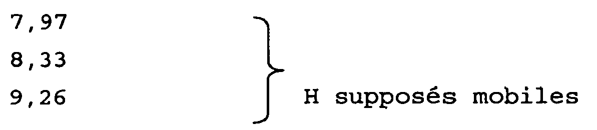 | |

### EXEMPLE 8 : Dichlorhydrate de trans-N-[2-[(4-aminocyclohexyl)-amino]-9-cyclopentyl-9H-purin-6-yl]-benzènesulfonamide.

### Stade 1 : N-[[2-chloro-9-cyclopentyl-9H-purin-6-yl]-amino]-benzènesulfonamide.

On mélange 257 mg du produit obtenu au stade 1 de l'exemple 1 ci-dessus, 4 ml de diméthoxyéthane (DME), 157 mg de benzènesulfonamide et 390 mg de carbonate de césium (CS₂CO₃) et agite à une température d'environ 100°C pendant 2 heures. On ajoute 4 ml d'acide chlorhydrique 2N et 4 ml d'eau, extrait par 30 ml d'acétate d'éthyle, sèche, filtre et évapore à sec. On empâte alors dans 5 ml d'éther et sèche sous vide à environ 50°C. On obtient ainsi 237 mg de produit attendu sous forme de cristaux beige.

### Stade 2 : Dichlorhydrate de trans-N-[2-[(4-aminocyclohexyl)-amino]-9-cyclopentyl-9H-purin-6-yl]-benzènesulfonamide.

On porte 570 mg de trans-1,4-diaminocyclohexane à une température d'environ 140°C puis ajoute 188 mg du produit obtenu au stade 1 ci-dessus et laisse à cette température pendant environ 5 heures. On laisse alors revenir à température ambiante. On ajoute 10 ml d'eau, essore et sèche sous vide à une température d'environ 50°C. Après chromatographie sur silice avec pour éluant chlorure de méthylène/méthanol/ammoniaque en proportion de 70/30/1, on empâte dans 5 ml d'éther et sèche à une température d'environ 50°C. On obtient ainsi 40 mg de produit attendu sous forme de cristaux brun.

### RMN dans DMSO

| | |
|---|---|
| 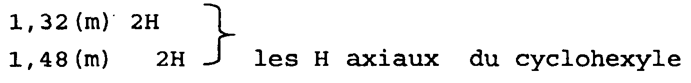 | |
| 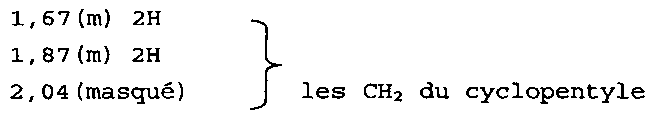 | |
| 3,04 (sl) 1H | H₄ axial |
| 3,56 (tl) 1H | H₁ axial |
| 4,70 (m) 1H | CH du cyclopentyle |
| 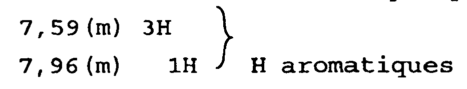 | |
| 8,12 (s) 1H | N=CH-N |
| 8,04 | H supposés mobiles |

### PRODUIT 9 : (2R,3S)-2,3-dihydroxybutanedioate de trans (+-)-N2-(4-aminocyclohexyl)-9-(1-méthylpropyl)-N6-(phénylméthyl)-9H-purine-2,6-diamine.

### Stade 1 : (+-)-2,6-dichloro-9-(1-méthylpropyl)-9H-purine

On procède comme au stade 1 de l'exemple 3 et mélange 1,32 mg de dichloro-2,6-purine, 2,75 g de triphénylphosphine (P(phényl)3), 35 ml de tétrahydrofuranne et 0,96 ml de 2-butanol, agite à température ambiante et ajoute en environ 20 minutes 1,63 ml de DEAD (diéthylazodicarboxylate) et agite une nuit à température ambiante. On verse sur 10 ml d'une solution 1 M de NaH₂PO₄ extrait par 3 fois avec 10 ml d'acétate d'éthyle, lave avec 10 ml d'eau puis avec 5 ml de chlorure de sodium en solution aqueuse saturée, sèche et évapore à sec. Après chromatographie sur silice avec pour éluant chlorure de méthylène/acétate d'éthyle en proportion de 90/10 puis une autre chromatographie sur silice avec pour éluant cyclohexane/acétate d'éthyle/chlorure de méthylène en proportion de 70/15/15, on obtient 1,27. g de produit attendu sous forme de cristaux blancs.

### Stade 2 : (+-)-2-chloro-9-(1-méthylpropyl)-N-(phénylméthyl)-9H-purin-6-amine.

On procède comme au stade 2 de l'exemple 3 et mélange 161 mg du produit obtenu au stade 1 ci-dessus, 3 ml de butanol et 0,132 ml de benzylamine et chauffe à une température d'environ 90 à 110°C pendant environ 5 heures. On laisse alors revenir à température ambiante, laisse cristalliser, dilue par 10 ml d'isopropanol, essore, lave par 10 ml d'isopropanol et sèche sous vide à environ 50°C. Après chromatographie sur silice avec pour éluant chlorure de méthylène/acétate d'éthyle en proportion de 50/50, on obtient ainsi 179 mg de produit attendu sous forme de cristaux blancs.

### Stade 3 : (2R,3S)-2,3-dihydroxybutanedioate de trans(+-) -N2-(4-aminocyclohexyl)-9-(1-méthylpropyl)-N6-(phénylméthyl)-9H-purine-2,6-diamine.

On procède comme au stade 3 de l'exemple 3 et porte 388 mg de trans-1,4-diaminocyclohexane à une température de 150°C et rajoute 107 mg du produit obtenu au stade 2 ci-dessus et laisse à une température de 140 à 150°C pendant environ 17 heures puis laisse revenir à température ambiante. On reprend alors avec 10 ml d'acétate d'éthyle/eau en proportion de 50/50, laisse décanter, ré-extrait par 2 x 10 ml d'acétate d'éthyle, lave par 10 ml d'eau et 5ml de chlorure de sodium en solution aqueuse saturée, sèche et évapore à sec. Après purification sur silice avec pour éluant méthanol/ammoniaque (NH4OH) en proportion de 98/2, on ajoute 10 ml d'une solution 1M d'acide m-tartrique dans l'éthanol et laisse cristalliser. Puis on essore, lave avec 2 x 1 ml d'éthanol et sèche à une température d'environ 50°C. On obtient ainsi 78 mg de produit attendu sous forme de cristaux beige.

### RMN dans DMSO

| | |
|---|---|
| 0, 73 (t) 3H | CH₃-CH₂-CH-CH₃ |
| 1,69 à 1,99 (m) | CH₃-CH₂-CH-CH₃ |
| 1,44 (d) | CH₃-CH₂-CH-CH₃ |
| 4,27 (m) 1H | CH₃-CH₂-CH-CH |
| 1,11 à 1,41 (m) | les H axiaux du cyclohexyle |
| 1,91 (d) | les H équatoriaux du cyclohexyle |
| 2,95 (t) | H₄ axial |
| 3,58 (m) | H₁ axial |
| 4,59 (sl) 2H | phényle-CH₂-NH |
| 6,17 (d) <1H | NH-CH |
| 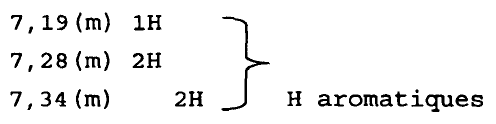 | |
| 7,77-7,74 | N=CH-N |
| 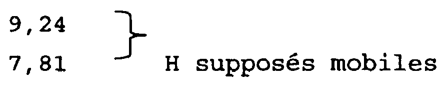 | |

### EXEMPLE 10 : Dichlorhydrate de trans-N-(2-((2-((4-aminocyclohexyl)amino)-9-cyclopentyl-9H-purin-6-yl)-amino)-éthyl)-4-méthyl-benzènesulfonamide.

### Stade 1 : N-[2-[(2-chloro-9-cyclopentyl-9H-purin-6-yl)-amino]-éthyl]-4-méthyl-benzènesulfonamide.

On mélange 280 mg du produit obtenu au stade 1 de l'exemple 7, 3 ml de chlorure de méthylène, 0,17 ml de NEt₃ (triéthylamine) et 230 mg de chlorure de l'acide 4-méthyl-benzènesulfonique puis agite à température ambiante pendant environ une demi-heure. On ajoute alors 2 ml d'eau, extrait par 2 x 5 ml de chlorure de méthylène, lave avec 5 ml d'eau, sèche et évapore. On empâte à l'éther, essore et sèche. On obtient ainsi 345 mg de produit attendu.

### Stade 2 : Dichlorhydrate de trans-N-(2-((2-((4-aminocyclohexyl)amino)-9-cyclopentyl-9H-purin-6-yl)-amino)-éthyl)-4-méthyl-benzenesulfonamide.

On mélange 320 mg du produit obtenu au stade 1 ci-dessus et 844 mg de trans-1,4-diaminocyclohexane et chauffe à environ 140°C pendant environ 3 heures puis descend à 80°C, ajoute alors 5 ml AcOEt, puis 10 ml d'eau à chaud. On laisse revenir à température ambiante, extrait avec 2 x 10 ml d'acétate d'éthyle, lave par 10 ml de solution de chlorure de sodium saturée puis sèche. Après purification par chromatographie sur silice avec pour éluant chlorure de méthylène /méthanol /ammoniaque en proportion de 90/10/1, on ajoute 1,5 ml d'acide chlorhydrique/éthanol 1,4N, laisse cristalliser puis filtre, rince avec 5 ml d'éther et sèche sous vide. On obtient ainsi 173 mg de produit attendu sous forme de cristaux blancs.

### RMN dans DMSO

| | |
|---|---|
| 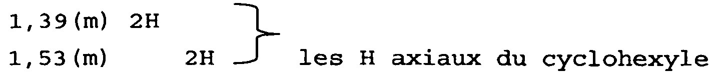 | |
| 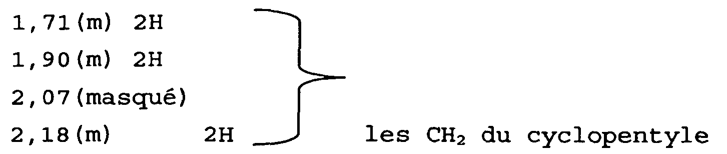 | |
| 2,34 (s) 3H | phényle-CH₃ |
| 3,10 (m) 3H | 1(CH₂) ₂-NF + H₄ supposé axial |
| 3,71 (m) 3H | 1(CH₂)₂-NH + H₁ supposé axial |
| 4,76 (m) 1H | CH du cyclopentyle |
| 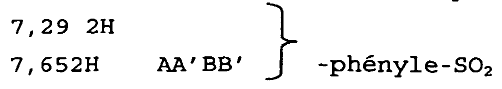 | |
| 8,10 (sl) <3H | NH₂ + N=CH-N |
| 7,54 | |
| 8,26 | |
| 8,81 | H supposés mobiles |

### EXEMPLE 11 : Dichlorhydrate de trans(+-)[[2-[(4-aminocyclohexyl)-amino]-9-(tétrahydro-3-furanyl)-9H-purin-6-y]-amino]-benzoate d'éthyle.

### Stade 1 : (+-)-4-[[2-chloro-9-(tétrahydro-3-furanyl)-9H-purin-6-yl]-amino]-benzoate d'éthyle.

On procède comme au stade 2 de l'exemple 2 à partir de 181 mg du produit obtenu au stade 1 de l'exemple 2 et 3 ml de butanol et en utilisant 124 ml de 4-aminobenzoate d'éthyle à la place de la benzylamine. On obtient ainsi 214 mg de produit attendu sous forme de cristaux blancs.

### RMN dans DMSO

| | |
|---|---|
| 2,31 (m) | |
| 2,55 (m) 2H | CH-CH₂-CH₂ |
| 3,91 (m) 2H | O-CH₂-CH |
| 5,22 (m) 2H | O-CH₂-CH |
| 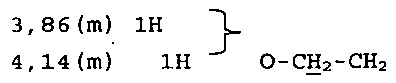 | |
| 1,33 (t) | |
| 4,30 (q) | CO₂-CH₃-CH₂ |
| 7,95 à 8,03 AA'BB' | N-phényle-C= |
| 8,41 (s) 1H | N=CH-N |
| 10,71 (s) | =C-NH |

### Stade 2 : Dichlorhydrate de trans(+-)[[2-[(4-aminocyclohexyl)-amino]-9-(tétrahydro-3-furanyl)-9H-purin-6-y]-amino]-benzoate d'éthyle.

On procède comme au stade 3 de l'exemple 2 à partir de 200 mg du produit obtenu au stade 1 ci-dessus et 600 mg de trans-1,4-diaminocyclohexane. Après purification sur silice avec pour éluant méthanol/triéthylamine (TEA) en proportion de 95/5, ou salifie par 1 à 2 ml d'acide chlorhydrique à 1,4N dans l'éthanol. On dilue dans 3 ml d'acétate d'éthyle, laisse deux heures à température ambiante, essore, lave avec 5 ml d'acétate d'éthyle et sèche à une température d'environ 50°C. On obtient ainsi 110 mg de produit attendu sous forme de cristaux beige rosé.

### RMN dans DMSO

| | |
|---|---|
| 1,32 (t) | CO₂-CH₂-CH₃ |
| 4,30 (q) | CO₂-CH₂-CH₃ |
| 1,30 à 1,60 | les H axiaux du cyclohexyle |
| 2,07 | les H équatoriaux du cyclohexyle |
| 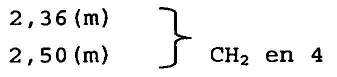 | |
| 3,02 | CH₂-N+ |
| 3,69 | CH-NC= |
| 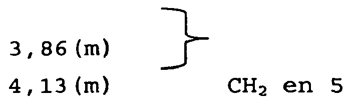 | |
| 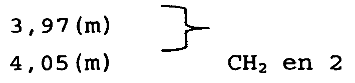 | |
| 5,17 (sl) | H₃ |
| 7,96 à 8,17 | -phényle-O- |
| 8,17 | faible absorption mobile NH3+ |
| 9,11 (s) à 11,07 (s) | NH |
| 7,56 | autres H mobiles |

### EXEMPLE 12 : Trichlorhydrate de trans-N2-(4-aminocyclohexyl)-N6-(2-aminoéthyl)-9-cyclopentyl-9H-purine-2,6-diamine.

On mélange 160 mg du produit obtenu au stade 1 de l'exemple 7, 10 ml de butanol et 690 mg de trans-1,4-diaminocyclohexane, chauffe à environ 150°C pendant environ 4 jours. Après chromatographie sur silice avec pour éluant chlorure de méthylène/méthanol/ammoniaque en proportion de 70/25/05. On salifie par une solution de HCl à 1,4N dans l'éthanol et on obtient ainsi 60 mg de produit attendu.

### RMN dans DMSO

| | |
|---|---|
| 1,25 à 1,55 | les H axiaux du cyclohexyle |
| 1,60 à 2,23 | les H équatoriaux du cyclohexyle |
| | et les CH2 du cyclopentyle |
| 3,11 | CH-+N |
| 3,77 | =C-NH-CH- |
| 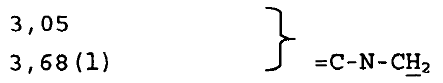 | |
| 4,77(sl) | N=C-N-CH |
| 8,16(sl) | H mobiles |

### EXEMPLE 13 : (2R,3S)-2,3-dihydroxybutanedioate de trans-(+-)-N2-(4-aminocyclohexyl)-N6-[(3-iodophényl)-méthyl]-9-(tétrahydro-3-furanyl)-9H-purine-2,6-diamine.

### Stade 1 : (+-)-2-chloro-N-[(3-iodophényl)-méthyl]-9-(tétrahydro-3-furanyl)-9H-purin-6-amine.

On procède comme au stade 2 de l'exemple 2 à partir de 133 mg du produit obtenu au stade 1 de l'exemple 2 et 2 ml de butanol et en utilisant 0,2 mg de 3-iodo-benzèneméthanamine (1,1 éq) à la place de la benzylamine. On obtient ainsi 208 mg de produit attendu sous forme de cristaux blancs.

### RMN dans CDCl3

| | |
|---|---|
| 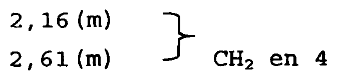 | |
| 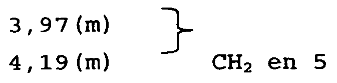 | |
| 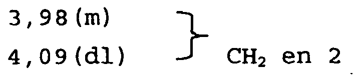 | |
| 5, 31 (m) | H₃ |
| 6,57 (sl) | NH |
| 7, 07 (t) | H₅' |
| 7, 34 (d) | |
| 7, 62 (d) | H₄' et H₆' |
| 7,73 (sl) | H₂' |
| 7,90 (s) | CH=N |

### Stade 2 : (2R,3S)-2,3-dihydroxybutanedioate de trans(+-) -N2-(4-aminocyclohexyl)-N6-[(3-iodophényl)-méthyl]-9-(tétrahydro-3-furanyl)-9H-purine-2,6-diamine.

On procède comme au stade 3 de l'exemple 2 à partir de 187 mg du produit obtenu au stade 1 ci-dessus et 470 mg de trans-1,4-diaminocyclohexane. Après purification sur silice avec pour éluant méthanol/ammoniaque (NH₄OH) en proportion de 98/2 et salification avec une solution 1m d'acide m-tartrique dans l'éthanol, on abandonne une nuit à température ambiante, essore, lave avec 10 ml d'acétate d'éthyle et sèche à une température d'environ 50°C. On obtient ainsi 137 mg de produit attendu sous forme de cristaux beige.

### RMN dans DMSO

| | |
|---|---|
| 1,29 (m) | les CH₂ axiaux du cyclohexyle |
| 1,93 (d) | les CH₂ équatoriaux du cyclohexyle |
| 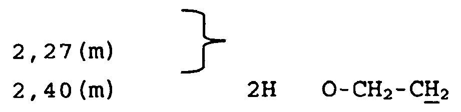 | |
| 2, 95 (tl) 1H | H₄ axial |
| 3,60 (m) 1H | H₁ axial |
| 3,77 à 4,00 4H | en excès |
| 4,08 1H | les CH₂-O |
| 4,56 (sl) 2H | phényle-CH₂-NH |
| 4,94 (m) 1H | -N-CH-CH₂-O |
| 6,31 (d) <1H | NH-CH |
| 7,11 (t) 1H | H₅' |
| 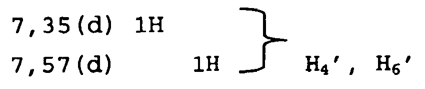 | |
| 7,71 (s) 1H | H₂' |
| 7,73 (s) 1H | N=CH-N |
| 7,99 | H supposé mobile |

### EXEMPLE 14 : Sel de sodium de l'acide trans-4((2-(4-amino-cyclohexyl)-amino)-9-cyclopentyl-9H-purin-6-yl) amino)-benzoïque.

On introduit à température ambiante 240 mg du produit de l'exemple 6, 10 ml d'éthanol puis 1 ml de soude (+-)-2-chloro-N-propyl-9-(tétrahydro-3-furanyl)-9H-purin-6-amine, agite à température ambiante pendant environ 20 heures, chauffe à environ 95°C pendant environ 3 heures puis laisse une nuit à température ambiante puis évapore à sec. On empâte dans l'acide acétique puis l'éther, sèche à environ 50°C et obtient ainsi 244 mg de produit attendu sous forme de cristaux beige.

### RMN dans DMSO

| | |
|---|---|
| 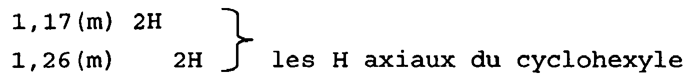 | |
| 1,67 (m) | |
| 1, 83 (m) | |
| 1,98 (m) 12H | les H équatoriaux du cyclohexyle + |
| 2,08 (m) | les CH₂ du cyclopentyle |
| 2,53 (masqué) | H₄ axial |
| 3,65 (ml) 1H | H₁ axial |
| 4,68 (m) 1H | CH du cyclopentyle |
| 6,43 (d) <1H | -HN-CH- |
| 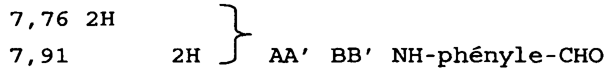 | |
| 7,88 (s) 1H | -N=CH-N- |
| 9,28 | H supposé mobile |

### EXEMPLE 15 : Dichlorhydrate de trans(+-)-N2-(4-aminocyclohexyl)-N6-propyl-9-(tétrahydro-3-furanyl)-9H-purine-2,6-diamine.

### Stade 1 : (+-)-2-chloro-N-propyl-9-(tétrahydro-3-furanyl)-9H-purin-6-amine.

On procède comme au stade 2 de l'exemple 2 à partir de 181 mg du produit obtenu au stade 1 de l'exemple 2 et 3 ml de butanol et en utilisant 0,062 ml de 1-propanamine à la place de la benzylamine. On obtient ainsi 136 mg de produit attendu sous forme de cristaux beige.

### RMN dans DMSO

| | |
|---|---|
| 1, 02 (t) | CH₃-CH₂-CH₂-NH₂ |
| 1, 71 (m) | CH₃-CH₂-CH₂-NH₂ |
| 3, 59 (m) | CH₃-CH₂-CH₂-NH₂ |
| 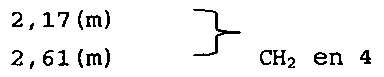 | |
| 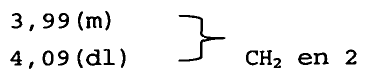 | |
| 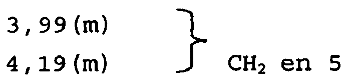 | |
| 6,04 et 5,80 | NH dédoublé |
| 7,89 (s) | CH=N |

### Stade 2 : Dichlorhydrate de trans(+-)-N2-(4-aminocyclohexyl)-N6-propyl-9-(tétrahydro-3-furanyl)-9H-purine-2,6-diamine.

On procède comme au stade 3 de l'exemple 2 à partir de 109 mg du produit obtenu au stade 1 ci-dessus et 445 mg de trans-1,4-diaminocyclohexane. Après purification sur silice avec pour éluant méthanol/ammoniaque (NH₄OH) en proportion de 98/2, on salifie par de l'acide chlorhydrique à 1,4N dans l'éthanol. On obtient ainsi 78 mg de produit attendu sous forme de cristaux.

### RMN dans DMSO

| | |
|---|---|
| 0,94 (t) 3H | CH₃-CH₂-CH₂-NH- |
| 1,65 (m) 2H | CH₃-CH₂-CH₂-NH |
| 3,58 (sl, masqué) | CH₃-CH₂-CH₂-NH |
| 1,37 à 1,52 | les H axiaux du cyclohexyle |
| 2,08 | les H équatoriaux du cyclohexyle |
| 3,05 (nl) 1H | H₄ axial |
| 3,72 (tl) 1H | H₁ axial |
| 8,12 (sl) | N=CHN = H mobiles (NH₂) |
| 9,32 (sl) <1H | H mobile |

### EXEMPLE 16 : Dichlorhydrate de trans-N-[2-[(4-aminocyclohexyl)-amino]-9-cyclopentyl-9H-purin-6-yl]-4-(1-méthyléthyl)-benzènesulfonamide.

### Stade 1 : N-[[2-chloro-9-cyclopentyl-9H-purin-6-yl] amino]-4-(1-méthyléthyl)-benzènesulfonamide.

On procède comme au stade 1 de l'exemple 8 et mélange 257 mg du produit obtenu au stade 1 de l'exemple 1, 4ml de diméthoxyéthane (DME), 390 mg de carbonate de césium (CS₂CO₃) et 199 mg de 4-(1-méthyléthyl)-benzène-sulfonamide à la place du benzènesulfonamide et agite à une température d'environ 100°C pendant environ 2 heures 30 minutes. On laisse revenir à température ambiante, acidifie avec 4 ml d'acide chlorhydrique 2N, extrait par 2 x 10 ml d'acétate d'éthyle, sèche et évapore à sec. On cristallise dans 5 ml d'éther, essore et sèche à température ambiante. On obtient ainsi 187 mg de produit attendu sous forme de cristaux incolores.

### Stade 2 : Dichlorhydrate de trans-N-[2-[(4-aminocyclohexyl)amino]-9-cyclopentyl-9H-purin-6-yl]-4-(1-méthyléthyl)-benzènesulfonamide.

On procède comme au stade 2 de l'exemple 8 et porte 456 mg de trans-1,4-diaminocyclohexane, à une température d'environ 150°C, ajoute 168 mg du produit obtenu au stade 1 ci-dessus et maintient à cette température de 150°C pendant environ 3 heures 30 minutes puis laisse alors revenir à température ambiante. On ajoute 10 ml d'eau, essore, lave avec 5 ml d'eau et sèche sous vide à une température d'environ 50°C. On acidifie à pH=4-5, extrait par 10 ml d'acétate d'éthyle puis évapore à sec le produit dans la phase aqueuse. On dissout dans 5 ml d'éthanol, ajoute 5 ml d'éthanol/acide chlorhydrique 1,4N, évapore à sec, empâte dans 5 ml d'éther et obtient ainsi 42 mg de produit attendu sous forme de cristaux.

### RMN dans DMSO

| | |
|---|---|
| 1,22 (d) 6H | CH₃-CH |
| 2, 98 (m) 1H | CH₃-CH |
| 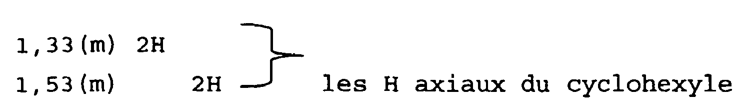 | |
| 1,68 (m) 2H | |
| 1,88 (m) 2H | les CH₂ du cyclopentyle |
| 2,06 (m) 8H | + les CH₂ équatoriaux du cyclohexyle |
| 3,00 (masqué) | H₄ axial |
| 3,57 (m) | H₁ axial |
| 4,71 (m) 1H | CH du cyclopentyle |
| 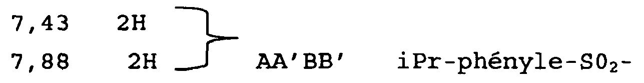 | |
| 8,11 (s) 1H | N=CH-N |
| 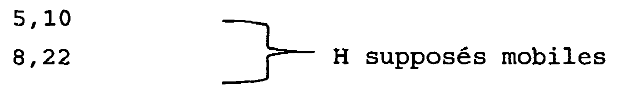 | |

### EXEMPLE 17 : Trichlorhydrate de trans-N2-(4-aminocyclohexyl)-9-cyclopentyl-N6-[2-[[(4-méthoxyphényl)-méthyl]-amino]-éthyl]-9H-purine-2,6-diamine.

### Stade 1 : 2-chloro-9-cyclopentyl-N-[2-[[(4-méthoxyphényl)-méthyl]-amino]-éthyl]-9H-purin-6-amine.

On procède comme au stade 2 de l'exemple 7 à partir de 280 mg du produit obtenu au stade 1 de l'exemple 7, 4 ml de méthanol, 0,2 ml de 4-méthoxy-benzaldéhyde, 0,2 ml d'acide acétique et 100 mg de NaBH₃CN et agite à température ambiante pendant environ 6 heures. On rajoute 10 ml AcOEt, lave par 2 x 5 ml H₂O, puis 5 ml d'une solution aqueuse saturé de NaCl. On sèche et évapore le solvant Après chromatographie sur silice avec pour éluant CH₂Cl₂/méthanol/ammoniaque en proportion de 90/10/1. On obtient ainsi 208 mg de produit attendu.

### Stade 2 : Trichlorhydrate de trans-N2-(4-aminocyclohexyl)-9-cyclopentyl-N6-[2-[[(4-méthoxyphényl)-méthyl]-amino]-éthyl]-9H-purine-2,6-diamine.

On procède comme au stade 3 de l'exemple 7 à partir de 186 mg du produit obtenu au stade 1 ci-dessus, 490 mg de trans-1,4-diaminocyclohexane, chauffe à environ 140°C pendant 10 heures. On purifie sur silice avec pour éluant CH₂Cl₂/MeOH/NH₄OH (85/15/1,5) puis salifie par une solution d'acide chlorhydrique à 1,4N dans l'éthanol. On obtient ainsi 60 mg de produit attendu.

### RMN dans DMSO

| | |
|---|---|
| 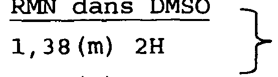 | |
| 1,55 (m) 2H | les H axiaux du cyclohexyle |
| 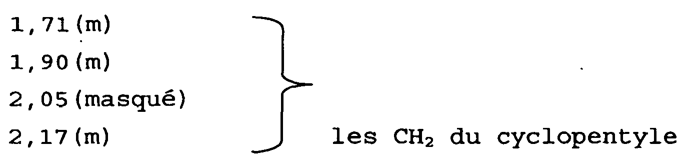 | |
| 2,06 (m) | les H équatoriaux du cyclohexyle |
| 3,04 (m,l) | H₄ axial |
| 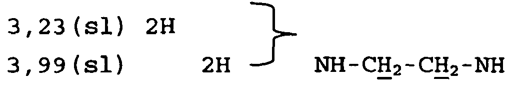 | |
| 3,71 (m) | H₁ axial |
| 3,78 (s) | O-CH₃ |
| 4,15 (s) 2H | NH-CH₂-phényle |
| 4,77 (m) | CH du cyclopentyle |
| 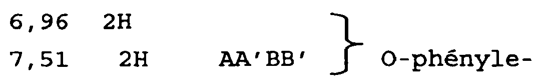 | |
| 8,24 (s) 1H | N=CH-N- |
| 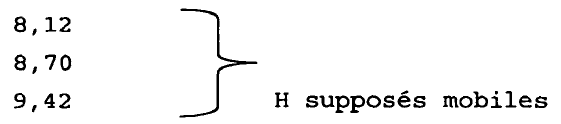 | |

### EXEMPLE 18 : Trichlorhydrate de trans-N2-(4-aminocyclohexyl)-9-cyclopentyl-N6-[2-[[(7-méthoxy-1,3-benzodioxol-5-yl)-méthyl]-amino]-éthyl]-9H-purine-2,6-diamine.

### Stade 1 : 2-chloro-9-cyclopentyl-N-[2-[[(7-méthoxy-1,3-benzodioxol-5-yl)-méthyl]-amino]-éthyl]-9H-purin-6-amine.

On procède comme au stade 2 de l'exemple 7 à partir de 280 mg du produit obtenu au stade 1 de l'exemple 7, 4 ml de méthanol, 250 mg de 7-méthoxy-1,3-benzodioxole-5-carboxaldéhyde à la place du benzaldéhyde, 0,2 ml d'acide acétique et 0,4 ml de tétrahydrofuranne et laisse pendant 4 heures à température ambiante. On ajoute alors 100 mg de NaBH3CN et agite à température ambiante pendant environ 3 heures. On rajoute 10 ml AcOEt, lave par 2 x 5 ml H₂O, puis 5 ml solution aqueuse saturé de NaCl. On sèche et évapore le solvant. Après purification par chromatographie sur silice avec pour éluant chlorure de méthylène/méthanol/ammoniaque en proportion de 90/10/1, on obtient ainsi 311 mg de produit attendu sous forme de cristaux beige.

### Stade 2 : Trichlorhydrate de trans-N2-(4-aminocyclohexyl)-9-cyclopentyl-N6-[2-[[(7-méthoxy-1,3-benzodioxol-5-yl)-méthyl]-amino]-éthyl]-9H-purine-2,6-diamine.

On procède comme au stade 3 de l'exemple 7 à partir de 65 mg du produit obtenu au stade 1 ci-dessus, 743 mg de trans-1,4-diaminocyclohexane, chauffe à environ 140°C pendant 3 heures. On traite par 10 ml d'eau et extrait à 2 x 10 ml d'acétate d'éthyle, lave avec 10 ml de NaCl (solution aqueuse saturée) et sèche sur MgSO₄. On salifie avec HCL/EtOH 1,4N, filtre lave avec 5 ml EtOH puis sèche à ~50°C. On obtient ainsi 161 mg de produit attendu.

### RMN dans DMSO

| | |
|---|---|
| 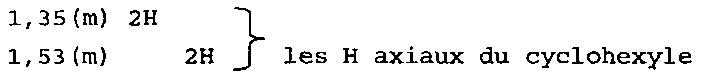 | |
| 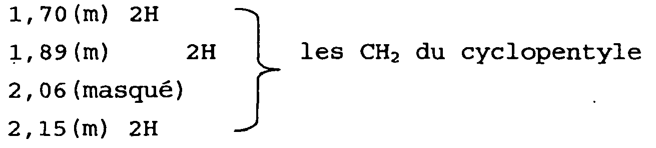 | |
| 3,03 (m,l) 1H | H₄ axial |
| 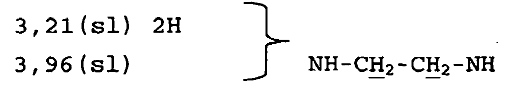 | |
| 3,70 (tl) | H₁ axial |
| 3,83 (s) | O-CH₃ |
| 4,12 (s) 2H | NH-CH₂-phényle |
| 4,76 (m) 1H | CH du cyclopentyle |
| 6,01 (s) 2H | O-CH₂-O |
| 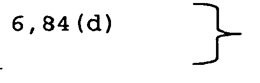 | |
| 6, 96 (d) | H₄, H₆ |
| 8,24 (s) 1H | N=CH-N- |
| 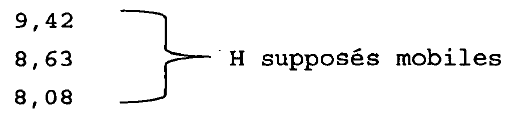 | |

### EXEMPLE 19 : Trichlorhydrate de trans-N2-(4-aminocyclohexyl)-N6-[2-[[[4-chloro-3-trifluoromethyl)-phényl]-méthyl]-amino]-éthyl]-9-cyclopentyl-9H-purine-2,6-diamine.

### Stade 1 : 2-chloro-N-[2-[[[4-chloro-3-(trifluorométhyl)-phényl]-méthyl]-amino]-éthyl]-9-cyclopentyl-9H-purin-6-amine.

On procède comme au stade 2 de l'exemple 7 à partir de 280 mg du produit obtenu au stade 1 de l'exemple 7, 4 ml de méthanol, 292 mg de 4-chloro-3-(trifluorométhyl)benzaldéhyde à la place du benzaldéhyde et 0,2 ml d'acide acétique puis agite à température ambiante pendant environ 3 heures. On ajoute alors 100 mg de NaBH₃CN et agite à température ambiante pendant environ 3 heures. On rajoute 10 ml AcOEt, lave par 2 x 5 ml H₂O puis 5 ml de solution aqueuse saturé de NaCl. On sèche et évapore le solvant. Après purification par chromatographie sur silice avec pour éluant chlorure de méthylène/méthanol/ammoniaque en proportion de 90/10/1, on obtient 367 mg de produit attendu sous forme de cristaux beige.

### Stade 2 : Trichlorhydrate de trans-N2-(4-aminocyclohexyl)-N6-[2-[[[4-chloro-3-(trifluorométhyl)-phényl]-méthyl]-amino]-éthyl]-9-cyclopentyl-9H-purine-2,6-diamine.

On procède comme au stade 3 de l'exemple 7 à partir de 320 mg du produit obtenu au stade 1 ci-dessus et 770 mg de trans-1,4-diaminocyclohexane et chauffe à environ 140°C pendant 3 heures. Après purification par chromatographie sur silice avec pour éluant chlorure de méthylène/méthanol/ammoniaque en proportion de 85/25/1,5 on salifie avec HCl/EtOH 1,4N, filtre, lave avec 5 ml EtOH puis sèche à ~50°C. On obtient ainsi 166 mg de produit attendu.

### RMN dans DMSO

| | |
|---|---|
| 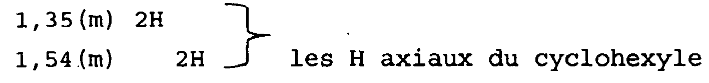 | |
| 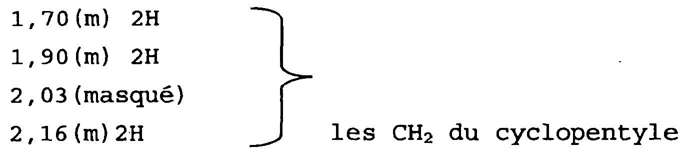 | |
| 2,03 (m) | les H équatoriaux du cyclohexyle |
| 3,03 (m,l) 1H | H₄ axial |
| 3,72 (tl) | H₁ axial |
| 9,28 (sl) 2H | |
| 4,00 (sl) | NH-CH₂-CH₂-NH |
| 4,34 (sl) 2H | NH-CH₂-phényle |
| 4,78 (m) 1H | CH du cyclopentyle |
| 7,94 (dd) 1H | H₅' |
| 7,77 (d) 1H | H₆' |
| 8,15 (d) 1H | H₃' |
| 8,33 (s) 1H | N=CH-N- |
| 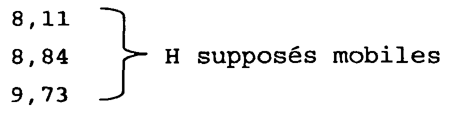 | |

### EXEMPLE 20 : Trichlorhydrate de trans-N2-(4-aminocyclohexyl)-9-cyclopentyl-N6-[(diphénylméthyl)-amino]-éthyl]-9H-purine-2,6-diamine.

### Stade 1 : 2-chloro-9-cyclopentyl-N-[2-[(diphénylméthyl)-amino]-éthyl]-9H-purin-6-amine.

On procède comme au stade 2 de l'exemple 7 à partir de 141 mg du produit obtenu au stade 1 de l'exemple 7, 2 ml de méthanol, 0,7 ml (15 éq) de benzaldéhyde et 0,1 ml d'acide acétique puis agite à température ambiante pendant environ 3 heures. On ajoute alors 55 mg de cyanoborohydrure de sodium (NaBH₃CN) et agite à température ambiante pendant 1 heure. On rajoute 10 ml AcOEt, lave par 2 x 5 ml H₂O, puis 5 ml de solution aqueuse saturée de NaCl. On sèche et évapore le solvant. Après purification par chromatographie sur silice avec pour éluant chlorure de méthylène/acétate d'éthyle en proportion de 70/30, le produit obtenu est empâté dans l'hexane et on obtient ainsi 143 mg de produit attendu.

### Stade 2 : Trichlorhydrate de trans-N2-(4-aminocyclohexyl)-9-cyclopentyl-N6-[(diphénylméthyl)-amino]-éthyl]-9H-purine-2,6-diamine.

On procède comme au stade 3 de l'exemple 7 à partir de 90 mg du produit obtenu au stade 1 ci-dessus et 222 mg de trans-1,4-diaminocyclohexane et chauffe à environ 140°C pendant environ 2 heures. Après purification par chromatographie sur silice avec pour éluant chlorure de méthylène/méthanol/ammoniaque en proportion de 85/15/1,5 on salifie avec HCl/EtOH 1,4N, filtre, lave avec 5 ml d'EtOH puis sèche à ~50°C.

### RMN dans DMSO

| | |
|---|---|
| 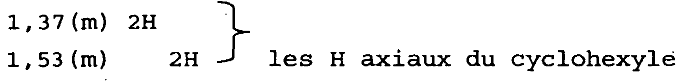 | |
| 1,72 (m) | |
| 1,90 (m) | |
| 2,04 (m) | les H équatoriaux du cyclohexyle et |
| 2,18 (m) | les CH₂ du cyclopentyle |
| 3,04 (sl) 1H | H₄ axial |
| 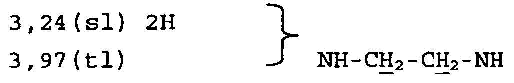 | |
| 3,68 (tl) 1H | H₁ axial |
| 4,29 (sl) 2H | NH-CH₂-phényle |
| 4,75 (m) 1H | =C-N-CH |
| 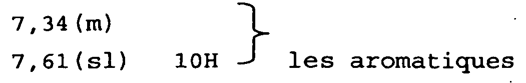 | |
| 8,19 | N=CH-N- |
| 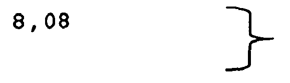 | |
| 8,68 | H supposés mobiles |

### EXEMPLE 21 : Dichlorhydrate de l'acide trans-4-[[[2-[(4-aminocyclohexyl)-amino]-9-cyclopentyl-9H-purin-6-yl]-amino]-méthyl]-benzoïque

On procède comme au stade 3 de l'exemple 1 à partir de 741 mg de trans-1,4-diaminocyclohexane que l'on chauffe à environ 140°C puis ajoute 500 mg du produit obtenu au stade 2 de l'exemple 1 et laisse à cette température pendant environ 3 heures puis laisse revenir à température ambiante. On rajoute 5 ml H₂O puis filtre. On reprend le précipité dans 10 ml d'éthanol puis ajoute 3 ml d'acide chlorhydrique à 1,4N dans l'éthanol, filtre l'insoluble et évapore à sec. On empâte le résidu à l'éther, sèche à température ambiante et obtient ainsi 45 mg de produit attendu.

### RMN dans DMSO

| | |
|---|---|
| 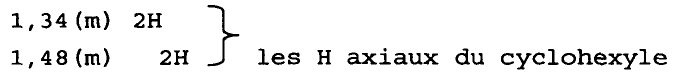 | |
| 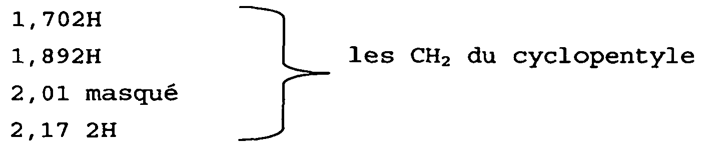 | |
| 2,01 (m) | les H équatoriaux du cyclohexyle |
| 3,01 (sl) 1H | H₄ axial |
| 3,68 (tl) 1H | H₁ axial |
| 4,76 (m) 1H | CH du cyclopentyle |
| 4,91 (sl) 2H | HN-CH₂-phényl |
| 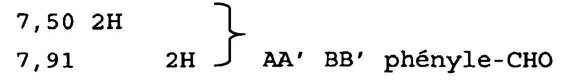 | |
| 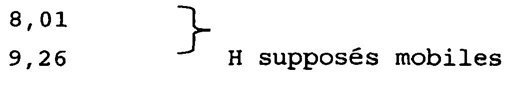 | |

### EXEMPLE 22 : Trichlorhydrate de trans 4-[[[2[[2-[(4-amino-cyclohexyl)-amino]-9-cyclopentyl-9H-purin-6-yl]-amino]-éthyl]-amino]-méthyl]-benzoate de méthyle.

### Stade 1 : 4-[[[2-[(2-chloro-9-cyclopentyl-9H-purin-6-yl)-amino]-éthyl]-amino]-méthyl]-benzoate de méthyle.

On procède comme au stade 2 de l'exemple 7 à partir de 280 mg du produit obtenu au stade 1 de l'exemple 7, 4 ml de méthanol, 230 mg de 4-formyl-benzoate de méthyle à la place du benzaldéhyde et 0,2 ml d'acide acétique puis agite à température ambiante pendant 5 heures. On ajoute alors 100 mg de NaBH3CN et agite à température ambiante pendant environ 1 heure. On rajoute 10 ml AcOEt, lave par 2 x 5 ml H₂O, puis 5 ml solution aqueuse saturé de NaCl. On sèche et évapore le solvant. Après purification par chromatographie sur silice avec pour éluant chlorure de méthylène/méthanol/ammoniaque en proportion de 95/05/0,5, on obtient 260 mg de produit attendu.

### Stade 2 : Trichlorhydrate de trans 4-[[[2[[2-[(4-aminocyclohexyl)-amino]-9-cyclopentyl-9H-purin-6-yl]-amino]-éthyl]-amino]-méthyl]-benzoate de méthyle.

On procède comme au stade 3 de l'exemple 7 à partir de 256 mg du produit obtenu au stade 1 ci-dessus et 700 mg de trans-1,4-diaminocyclohexane et chauffe à environ 140°C pendant environ 4 heures. On extrait alors par 3 x 10 ml d'acétate d'éthyle et lave par 10 ml de solution aqueuse saturée de chlorure de sodium. Après purification par chromatographie sur silice avec pour éluant chlorure de méthylène/méthanol/ammoniaque en proportion de 85/15/1,5, on salifie avec HCl/EtOH 1,4N, filtre lave avec 5 ml EtOH puis sèche à ~50°C. On obtient ainsi 70 mg de produit attendu.

### RMN dans DMSO

| | |
|---|---|
| 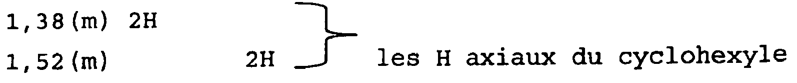 | |
| 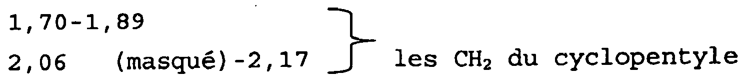 | |
| 2,04 (m) | les H équatoriaux du cyclohexyle |
| 3,04 (sl) 1H | H₄ axial |
| 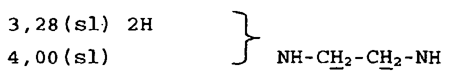 | |
| 3,72 (tl) 1H | H₁ axial |
| 4,32 (sl) 2H | NH-CH₂-phényle |
| 4,77 | CH du cyclopentyle |
| 3,88 (s) | CO₂-CH₃ |
| 7,74 2H | |
| 7,98 2H AA'BB' | phényle-CO₂CH₃ |
| 7,69 2H | |
| 7,89 2H AA'BB' | phényle-CO₂H |
| 8,33 | N=CH-N |
| 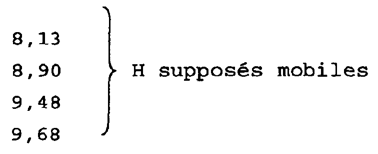 | |

### EXEMPLE 23 : Trichlorhydrate de trans-N2-(4-aminocyclohexyl)-N6-[4-cyanophényl)-méthyl]-amino]-éthyl]-9-cyclopentyl-9H-purine-2,6-diamine.

### Stade 1 : 2-chloro-N-[2-[[(4-cyanophényl)-méthyl]-amino]-éthyl]-9-cyclopentyl-9H-purin-6-amine.

On procède comme au stade 2 de l'exemple 7 à partir de 280 mg du produit obtenu au stade 1 de l'exemple 7, 184 mg de 4-cyano-benzaldéhyde à la place du benzaldéhyde, 4 ml de méthanol et 0,2 ml d'acide acétique et 0,5 ml de tétra-hydrofuranne puis agite à température ambiante pendant environ 5 heures. On ajoute alors 100 mg de NaBH₃CN et agite à température ambiante pendant 1 heure. On rajoute 10 ml AcOET, lave par 2 x 5 ml H₂O puis 5 ml solution aqueuse saturé de NaCl. On sèche et évapore le solvant. Après purification par chromatographie sur silice avec pour éluant chlorure de méthylène/méthanol/hydroxylamine en proportion de 95/05/0,33, on obtient 347 mg de produit attendu sous forme de cristaux blancs.

### Stade 2 : Trichlorhydrate de trans-N2-(4-aminocyclohexyl)-N6-[4-cyanophényl)-méthyl]-amino]-éthyl]-9-cyclopentyl-9H-purin-2,6-diamine.

On procède comme au stade 3 de l'exemple 7 à partir de 250 mg du produit obtenu au stade 1 ci-dessus et 720 mg. de trans-1,4-diaminocyclohexane et chauffe à environ 140°C pendant 4 heures. On reprend avec 10 ml H₂O et extrait alors 3 x 10 ml d'acétate d'éthyle et lave par 10 ml de chlorure de sodium saturé. Après purification par chromatographie sur silice avec pour éluant chlorure de méthylène/méthanol/hydroxylamine en proportion de (85/15/1,5), on salifie avec HCl/EtOH 1,4N, filtre lave avec 5 ml EtOH puis sèche à -50°C. On obtient ainsi 222 mg de produit attendu.

### RMN dans DMSO

| | |
|---|---|
| 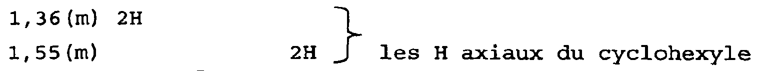 | |
| 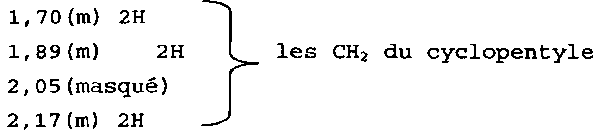 | |
| 2,04 (m) | les H équatoriaux du cyclohexyle |
| 3,04 (sl) 1H | H₄ axial |
| 3,73 (tl) 1H | H₁ axial |
| 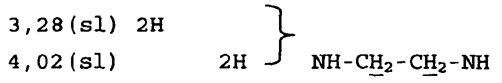 | |
| 4,33 (sl) 2H | NH-CH₂-phényle |
| 4,78 (m) 1H | CH du cyclopentyle |
| 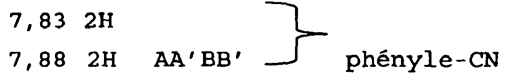 | |
| 8,39 (sl) 1H | N=CH-N |
| 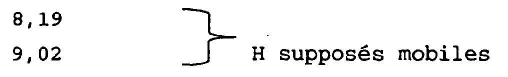 | |

### EXEMPLE 24 : Trichlorhydrate de trans-N2-(4-aminocyclohexyl)-9-cyclopentyl-N6-[2-[[3,4-5-triméthoxyphényl)-méthyl]-amino]-éthyl]-9H-purin-2,6-diamine.

### Stade 1 : 2-chloro-9-cyclopentyl-N-[2-[[(3,4,5-triméthoxyphényl)-méthyl]-amino]-éthyl]-9H-purin-6-amine.

On procède comme au stade 2 de l'exemple 7 à partir de 280 mg du produit obtenu au stade 1 de l'exemple 7, 275 mg de 3,4,5-triméthoxybenzaldéhyde à la place du benzaldéhyde, 4 ml de méthanol et 0,2 ml d'acide acétique puis agite à température ambiante pendant 5 heures. On ajoute alors 100 mg de NaBH₃CN et agite à température ambiante pendant environ 1 heure. On rajoute 10 ml AcOEt, lave par 2 x 5 ml H₂O, puis 5 ml solution aqueuse saturé de NaCl. On sèche et évapore le solvant. Après purification par chromatographie sur silice avec pour éluant chlorure de méthylène/méthanol/ammoniaque en proportion de 95/05/0,33, on obtient 305 mg de produit attendu sous forme de cristaux blancs.

### Stade 2 : Trichlorhydrate de trans-N2-(4-aminocyclohexyl)-9-cyclopentyl-N6-[2-[[3,4-5-triméthoxyphényl)-méthyl]-amino]-éthyl]-9H-purin-2,6-diamine.

On procède comme au stade 3 de l'exemple 7 à partir de 297 mg du produit obtenu au stade 1 ci-dessus et 735 mg de trans-1,4-diaminocyclohexane et chauffe à environ 140°C pendant 3 heures 30 minutes. On verse sur 10 ml H₂O et extrait alors par 3 x 10 ml d'acétate d'éthyle et lave par 10 ml de chlorure de sodium saturé. Après purification par chromatographie sur silice avec pour éluant chlorure de méthylène/méthanol/ammoniaque en proportion de 85/15/1,5, on salifie avec HCl/EtOH 1,4N, filtre lave avec 5 ml EtOH puis sèche à ~50°C.

### RMN dans DMSO

| | |
|---|---|
| 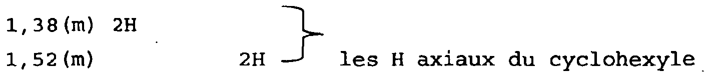 | |
| 2,07 (m) | les H équatoriaux du cyclohexyle |
| 3,04 (ml) 1H | H₄ axial |
| 3,73 (masqué partiellement) | H₁ axial |
| 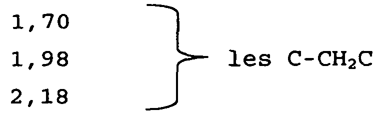 | |
| 4,78 | N-CH N-cyclopentyle |
| 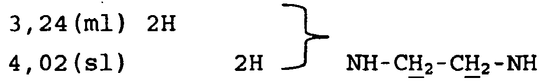 | |
| 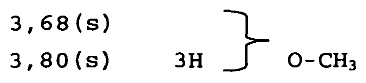 | |
| 4,15 | N-CH₂-C=H₂ les CH du cycle tétrasubstitué |
| 8,11 (l) -8,35 (l) 1H | N=CH-N |
| 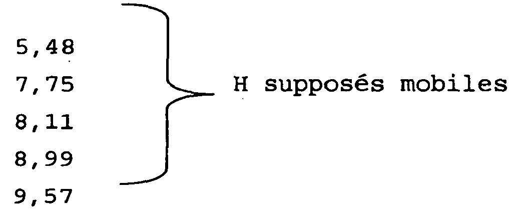 | |

### EXEMPLE 25 : Trichlorhydrate de trans-N2-(4-aminocyclohexyl)-N6-[2-[[(4-chlorophenyl)-méthyl]-amino]-éthyl]-9-cyclopentyl-9H-purin-2,6-diamine.

### Stade 1 : 2-chloro-N-[2-[[(4-chlorophényl)-méthyl]-amino]-éthyl]-9-cyclopentyl-9H-purin-6-amine.

On procède comme au stade 2 de l'exemple 7 à partir de 280 mg du produit obtenu au stade 1 de l'exemple 7, 197 mg de 4-chlorobenzaldéhyde à la place du benzaldéhyde, 4 ml de méthanol et 0,2 ml d'acide acétique puis agite à température ambiante 5 heures. On ajoute alors 100 mg de NaBH₃CN et agite à température ambiante pendant environ 1 heure. On rajoute 10 ml AcOEt, lave par 2 x 5 ml H₂O, puis 5 ml solution aqueuse saturé de NaCl. On sèche et évapore le solvant. Après purification par chromatographie sur silice avec pour éluant chlorure de méthylène/méthanol/ammoniaque en proportion de 95/05/0,33, on obtient 258 mg de produit attendu.

### Stade 2 : Trichlorhydrate de trans-N2-(4-aminocyclohexyl)-N6-[2-[[(4-chlorophényl)-méthyl]-amino]-éthyl]-9-cyclopentyl-9H-purin-2,6-diamine.

On procède comme au stade 3 de l'exemple 7 à partir de 242 mg du produit obtenu au stade 1 ci-dessus et 680 mg de trans-1,4-diaminocyclohexane et chauffe à environ 140°C pendant environ 3 heures 30 minutes. On verse sur H₂O puis extrait alors par 3 x 10 ml d'acétate d'éthyle et lave par 10 ml de chlorure de sodium saturé. Après purification par chromatographie sur silice avec pour éluant chlorure de méthylène/méthanol /ammoniaque en proportion de 85/15/1,5, on salifie avec HCl/EtOH 1,4N, filtre lave avec 5 ml EtOH puis sèche à ~50°C.

### RMN dans DMSO

| | |
|---|---|
| 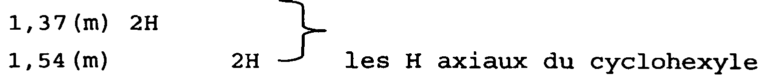 | |
| 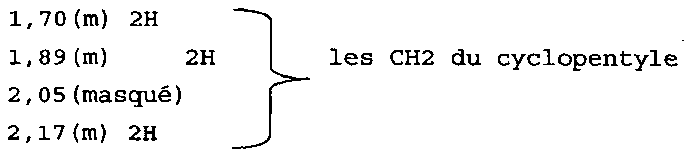 | |
| 2,05 (m) | les H équatoriaux du cyclohexyle |
| 3,05 (sl) 1H | H₄ axial |
| 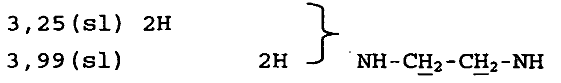 | |
| 3,77 (tl) 1H | H₁ axial |
| 4,12 (sl) 2H | HN-CH₂-phényle |
| 4,78 (m) 1H | CH du cyclopentyle |
| 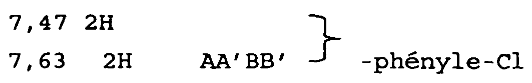 | |
| 8,35 (sl) 1H | N=CH-N |
| 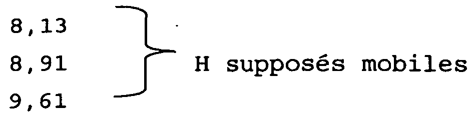 | |

### EXEMPLE 26 : Dichlorhydrate de trans-N-[2-[(4-aminocyclohexyl)-amino]-9-cyclopentyl-9H-purin-6-yl]-3-bromo-benzène-sulfonamide.

### Stade 1 : 3-bromo-N-[[2-chloro-9-cyclopentyl-9H-purin-6-yl]-amino]-benzènesulfonamide.

On procède comme au stade 1 de l'exemple 8 à partir de 257 mg du produit obtenu au stade 1 de l'exemple 1, 4 ml de diméthoxyéthane (DME), 390 mg de carbonate de césium (Cs₂CO₃) et 236 mg de 3-bromo-benzènesulfonamide à la place du benzènesulfonamide et agite à une température d'environ 100°C pendant environ 2 heures 30 minutes. On laisse revenir à température ambiante, acidifie avec 4 ml d'acide chlorhydrique 2N, extrait par 2 x 10 ml d'acétate d'éthyle, sèche et évapore à sec. On empâte dans 5 ml d'éther, essore et sèche à température ambiante. On obtient ainsi 356 mg de produit attendu sous forme de cristaux beige.

### Stade 2 : Dichlorhydrate de trans-N-[2-[(4-aminocyclohexyl)-amino]-9-cyclopentyl-9H-purin-6-yl]-3-bromo-benzène-sulfonamide.

On procède comme au stade 2 de l'exemple 8 et porte 400 mg de trans-1,4-diaminocyclohexane, à une température d'environ 150°C, ajoute 319 mg du produit obtenu au stade 1 ci-dessus et maintient à cette température de 150°C pendant une nuit. On laisse alors revenir à température ambiante puis chromatographie sur silice avec pour éluant CH₂CL/MeOH/NH₄OH (70/30/1), on ajoute alors 4 ml d'éthanol et 4 ml d'éthanol/acide chlorhydrique 1,4N, évapore à sec, empâte dans 10 ml d'éther/acétate d'éthyle en proportion de 50/50 et sèche sous vide à une température d'environ 50°C. On obtient ainsi 231 mg de produit attendu sous forme de cristaux beige.

### RMN dans DMSO

| | |
|---|---|
| 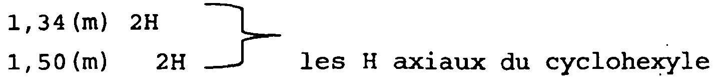 | |
| 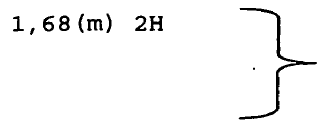 | |
| 1,87 (m) 2H | les CH₂ du cyclopentyle |
| 2,07 (masqué) | |
| 2,07 | les H équatoriaux du cyclohexyle |
| 3,05 (sl) 1H | H₄ axial |
| 3,60 (tl) 1H | H₁ axial |
| 4,71 (m) 1H | CH du cyclopentyle |
| 7,54 (t) 1H | H₅' |
| 7,80 (ddd) 1H | H₆' |
| 7,95 (dt) 1H | H₄' |
| 8,09 (t) 1H | H₂' |
| 8,18 (sl) | N=CH-N |
| 8,14 | H supposés mobiles |

### EXEMPLE 27 : Dichlorhydrate de trans-N-[2-[(4-aminocyclohexyl)-amino]-9-cyclopentyl-9H-purin-6-yl]-3-(trifluorométhyl)-benzènesulfonamide.

### Stade 1 : N-[[2-chloro-9-cyclopentyl-9H-purin-6-yl]-amino]-3-trifluorométhyl-benzènesulfonamide.

On procède comme au stade 1 de l'exemple 8 à partir de 257 mg du produit obtenu au stade 1 de l'exemple 1, 4 ml de diméthoxyéthane (DME), 390 mg de carbonate de césium (Cs₂CO₃) et 225 mg de 3-trifluorométhyl-benzènesulfonamide à la place du benzènesulfonamide et agite à une température d'environ 100°C pendant environ 2 heures 30 minutes. On laisse revenir à température ambiante, acidifie avec 4 ml d'acide chlorhydrique 2N, extrait par 2 x 10 ml d'acétate d'éthyle, sèche et évapore à sec. On empâte dans 10 ml d'éther, essore et sèche à température ambiante. On obtient ainsi 273 mg de produit attendu sous forme de cristaux incolore.

### Stade 2 : Dichlorhydrate de trans-N-[2-[(4-aminocyclohexyl)-amino]-9-cyclopentyl-9H-purin-6-yl]-3-(trifluorométhyl)-benzènesulfonamide.

On procède comme au stade 2 de l'exemple 8 et porte 286 mg de trans-1,4-diaminocyclohexane, à une température d'environ 150°C, ajoute 222 mg du produit obtenu au stade 1 ci-dessus et maintient à cette température de 150°C pendant 3 heures, puis à 110°C pendant une nuit puis à 150°C pendant 2 heures. On laisse alors revenir à température ambiante puis chromatographie sur silice avec pour éluant CH₂Cl₂/MeOH/NH₄OH (70/30/1), on ajoute alors 5 ml d'éthanol et 4 ml d'éthanol/acide chlorhydrique 1,4N, évapore à sec, empâte dans 5 ml d'éther/acétate d'éthyle en proportion de 50/50 et sèche sous vide à une température d'environ 50°C. On obtient ainsi 166 mg de produit attendu sous forme de cristaux beige.

### RMN dans DMSO

| | |
|---|---|
| 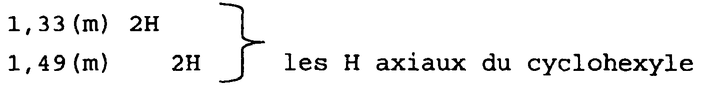 | |
| 1,67 (sl) 2H | |
| 1,86 (sl) 2H | |
| 2,08 (masqué) | les CH₂ du cyclopentyle |
| 2,05 | les H équatoriaux du cyclohexyle |
| 3,04 (sl) 1H | H₄ axial |
| 3,60 (tl) 1H | H₁ axial |
| 4,71 (sl) 1H | CH du cyclopentyle |
| 7,82 (tl) 1H | H₅' |
| 7,96 (dl) 1H | H₄' |
| 8,24 (m) 2H | H₆', H₂' |
| 8,16 (masqué) | N=CH-N |
| 8,16 | H supposés mobiles |

### EXEMPLE 28 : Dichlorhydrate de trans-N-[2-[(4-aminocyclohexyl)-amino]-9-cyclopentyl-9H-purin-6-yl]-4-(1,1-diméthyl-éthyl)-benzènesulfonamide.

### Stade 1 : N-[[2-chloro-9-cyclopentyl-9H-purin-6-yl]-amino]-4-(1,1-diméthyléthyl)-benzènesulfonamide.

On procède comme au stade 1 de l'exemple 8 à partir de 257 mg du produit obtenu au stade 1 de l'exemple 1, 4 ml de diméthoxyéthane (DME), 390 mg de carbonate de césium (CS₂CO₃) et 213 mg de 4-(1,1-diméthyléthyl)-benzènesulfonamide à la place du benzènesulfonamide et agite à une température d'environ 100°C pendant environ 4 heures. On laisse revenir à température ambiante et ajoute 4 ml d'acide chlorhydrique 2N et 5 ml d'eau, extrait par 40 ml d'acétate d'éthyle, sèche et évapore à sec. On empâte dans 10 ml d'éther, essore et sèche à température ambiante. On obtient ainsi 253 mg de produit attendu sous forme de cristaux beige.

### Stade 2 : Dichlorhydrate de trans-N-[2-[(4-aminocyclohexyl)-amino]-9-cyclopentyl-9H-purin-6-yl]-4-(1,1-diméthyléthyl)-benzènesulfonamide.

On procède comme au stade 2 de l'exemple 8 et porte 286 mg de trans-1,4-diaminocyclohexane, à une température d'environ 150°C, ajoute 217 mg du produit obtenu au stade 1 ci-dessus et maintient à cette température pendant environ 5 heures. On laisse alors revenir à température ambiante puis chromatographie sur silice avec pour éluant chlorure de méthylène/méthanol/ammoniaque en proportion de 70/30/1. On dissout dans 10 ml d'éthanol, ajoute alors 4 ml d'acide chlorhydrique/éthanol 1,4N, évapore à sec, empâte dans 10 ml d'éther et sèche à température ambiante. On obtient ainsi 107 mg de produit attendu sous forme de cristaux beige.

### RMN dans DMSO

| | |
|---|---|
| 1,31(s) | tBu |
| 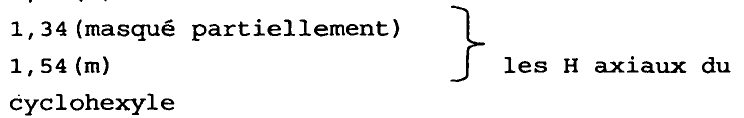 | |
| 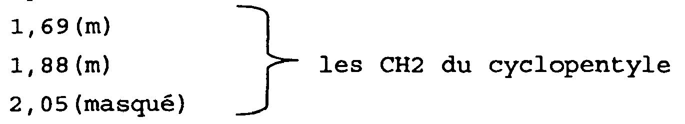 | |
| 2,05 | les H équatoriaux du cyclohexyle |
| 3,04 (sl) 1H | H₄ axial |
| 3,58 (tl) 1H | H₁ axial |
| 4,71 (m) 1H | CH du cyclopentyle |
| 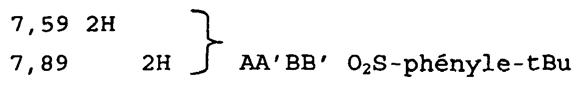 | |
| 8,16 (sl) >3H | N=CH-N + H supposés mobiles |

### EXEMPLE 29 : Dichlorhydrate de trans(+-)-N2-(4-aminocyclohexyl)-N6-propyl-9- (tétrahydro-3-thiényl)-9H-purin-6-amine.

### Stade 1 : 2-chloro-N-propyl-9-(tétrahydro-3-thiényl)-9H-purin-2,6-diamine.

On procède comme au stade 2 de l'exemple 5 à partir de 160 mg du produit obtenu au stade 1 de l'exemple 5, 3 ml de butanol et en utilisant 0,100 ml de 1-propanamine à la place de la benzylamine et porte à une température d'environ 80 à 85°C pendant 24 heures. On laisse revenir à température ambiante, évapore à sec, empâte dans 10 ml d'acétate d'éthyle à température ambiante, essore, lave avec 10 ml et sèche sous vide à une température d'environ 50°C. On obtient ainsi 123 mg de produit attendu sous forme de cristaux blancs.

### Stade 2 : Dichlorhydrate de trans(+-)-N2-(4-aminocyclohexyl)-N6-propyl-9-(tétrahydro-3-thiényl)-9H-purin-2,6-diamine.

On procède comme au stade 3 de l'exemple 5 à partir de 400 mg de trans-1,4-diaminocyclohexane et 106 mg du produit obtenu au stade 1 ci-dessus, porte à une température d'environ 140 à 145°C pendant environ 6 heures puis laisse revenir à température ambiante. Après purification sur silice avec pour éluant méthanol/ammoniaque (NH₄OH) en proportion de 98/2 et salification par de l'acide chlorhydrique à 1,4N dans l'éthanol, on obtient 30 mg de produit attendu sous forme de cristaux beige rosé.

### RMN dans DMSO

| | |
|---|---|
| 0, 94 (t) 3H | CH₂-CH₂-CH₂-NH- |
| 1,64 (m) 2H | CH₂-CH₂-CH₂-NH |
| 3, 55 (masqué) | CH₂-CH₂-CH₂-NH |
| 1,43 (m) 4H | les H axiaux du cyclohexyle |
| 2,07 (tl) 4H | les H équatoriaux du cyclohexyle |
| 2,43 (m) masqué | S-CH₂-CH₂ |
| 2,98 (m) 2H | S-CH₂-CH₂ |
| 3,05 (masqué) | H₄ supposé axial |
| 3,72 (tl) 1H | H₁ axial |
| 3,27 (m) | S-CH₂-CH |
| 5,03 (m) 1H | S-CH₂-CH |
| 8,01 (sl) 3H | N=CH-N + H mobiles |
| 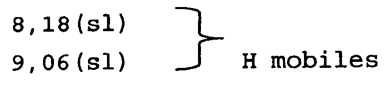 | |

### EXEMPLE 30 : Dichlorhydrate de trans(+-)-N2-(4-aminocyclohexyl)-N6-.[(3-iodophényl)-méthyl]-9-(tétrahydro-3-thiényl)-9H-purin-2,6-diamine.

### Stade 1 : 2-chloro-N-[(3-iodophényl)-méthyl]-9-(tétrahydro-3-thiényl)-9H-purin-6-amine.

On procède comme au stade 2 de l'exemple 5 à partir de 160 mg du produit obtenu au stade 1 de l'exemple 5, 3 ml de butanol et en utilisant 0,114 ml de 3-iodobenzèneméthanamine à la place de la benzylamine et porte à une température d'environ 80 à 85°C pendant environ 30 heures. On laisse revenir à température ambiante, dilue par 3 ml d'isopropanol, place environ une heure à une température d'environ 0°C, essore, lave avec 5 ml d'isopropanol et sèche sous vide à une température d'environ 50°C. Après purification sur silice avec pour éluant chlorure de méthylène/acétate d'éthyle à la proportion 90/10, on obtient ainsi 173 mg de produit attendu sous forme de cristaux blanc-jaune.

### Stade 2 : Dichlorhydrate de trans(+-)-N2-(4-aminocyclohexyl)-N6-[(3-iodophényl)-méthyl]-9-(tétrahydro-3-thiényl)-9H-purin-2,6-diamine.

On procède comme au stade 3 de l'exemple 5 à partir de 330 mg de trans-1,4-diaminocyclohexane et 137 mg du produit obtenu au stade 1 ci-dessus, porte à une température d'environ 140 à 145°C pendant environ 6 heures puis laisse revenir à température ambiante et laisse une nuit. Après purification sur silice avec pour éluant méthanol /ammoniaque (NH₄OH) en proportion de 98/2 et salification par de l'acide chlorhydrique à 1,4N dans l'éthanol, on essore, lave et sèche à une température d'environ 50°C. On obtient ainsi 74 mg de produit attendu sous forme de cristaux brun-rosé.

### RMN dans DMSO

| | |
|---|---|
| 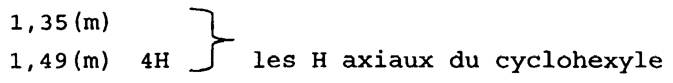 | |
| 2,02 (dl) 4H | les H équatoriaux du cyclohexyle |
| 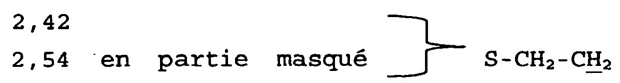 | |
| 2,97 (m) 3H | S-CH₂-CH₂ + H₄ supposé axial |
| 3,27 (m) 2H | S-CH₂-CH |
| 3,71 (tl) en partie masqué | H₁ axial |
| 4,80 (sl) 2H | NH-CH₂-phényle |
| 5,05 (m) 1H | N-CH-CH₂-S- |
| 7,16 (t) 1H | H₅' |
| 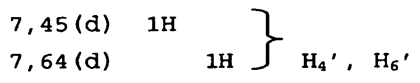 | |
| 7,80 (s) 1H | H₂' |
| 8,04 (sl) >2H | N=CH-N + H supposés mobiles |
| 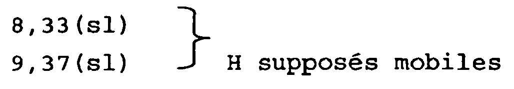 | |

### EXEMPLE 31 : Dichlorhydrate de trans(+-)-N2-(4-aminocyclohexyl)-N6-phényl-9-(tétrahydro-3-thiényl)-9H- purin-2,6-diamine.

### Stade 1 : 2-chloro-N-phényl-9-(tétrahydro-3-thiényl)-9H-purin-6-amine.

On procède comme au stade 2 de l'exemple 5 à partir de 160 mg du produit obtenu au stade 1 de l'exemple 5, 3 ml de butanol et en utilisant 0,055 ml d'aniline à la place de la benzylamine et porte à une température d'environ 80 à 85°C pendant 20 heures. On laisse revenir à température ambiante, dilue par 5 ml d'isopropanol, place environ une heure à une température d'environ 0°C, essore, lave avec 5 ml d'isopropanol et sèche sous vide à une température d'environ 50°C. Après purification sur silice avec pour éluant chlorure de méthylène/acétate d'éthyle à la proportion 90/10, on obtient ainsi 173 mg de produit attendu sous forme de cristaux blancs.

### Stade 2 : Dichlorhydrate de trans(+-)-N2-(4-aminocyclohexyl)-N6-phényl-9-(tétrahydro-3-thiényl)-9H-purin-2,6-diamine.

On procède comme au stade 3 de l'exemple 5 à partir de 536 mg de trans-1,4-diaminocyclohexane et 156 mg du produit obtenu au stade 1 ci-dessus, porte à une température d'environ 140 à 145°C pendant environ 4 heures 30 minutes puis laisse revenir à température ambiante. Après purification sur silice avec pour éluant méthanol/ammoniaque (NH₄OH) en proportion de 98/2 et salification par de l'acide chlorhydrique à 1,4N dans l'éthanol, on essore, lave et sèche à une température d'environ 50°C. On obtient ainsi 145 mg de produit attendu sous forme de cristaux beige rosé.

### RMN dans DMSO

| | |
|---|---|
| 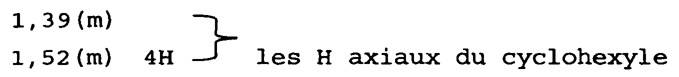 | |
| 2,07 4H | les H équatoriaux du cyclohexyle |
| 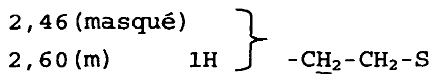 | |
| 3, 00 (m) 3H | CH₂-CH₂-S + H₄ supposé axial |
| 3,32 (m) 2H | -CH-CH₂-S |
| 5,13 (m) 1H | -CH-CH₂-S |
| 3,71 (m) 1H | H₁ axial |
| 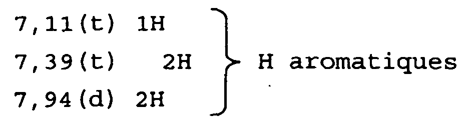 | |
| 8,08 (sl) | >2H N=CH-N + H mobiles |
| 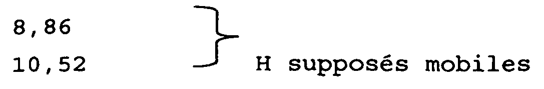 | |

### EXEMPLE 32 : Dichlorhydrate de trans(+-)-4-[[2-[(4-aminocyclohexyl)-amino]-9-(tétrahydro-3-thiényl)-9H-purin-6-yl]-amino]-benzoate d'éthyle.

### Stade 1 : 4-((2-chloro-9-(tétrahydro-3-thiényl)-9H-purin-6-yl)-amino)-benzoate d'éthyle.

On procède comme au stade 2 de l'exemple 5 à partir de 160 mg du produit obtenu au stade 1 de l'exemple 5, 3 ml de butanol et en utilisant 100 mg de 4-aminobenzoate d'éthyle à la place de la benzylamine et porte à une température d'environ 80 à 85°C pendant 30 heures. On laisse revenir à température ambiante, dilue par 3 ml d'isopropanol, place environ une heure à une température d'environ 0°C, essore, lave avec 5 ml d'isopropanol et sèche sous vide à une température d'environ 50°C. Après purification sur silice avec pour éluant chlorure de méthylène/acétate d'éthyle à la proportion 90/10, on obtient ainsi 202 mg de produit attendu sous forme de cristaux blanc-crème.

### Stade 2 : Dichlorhydrate de trans(+-)-4-[[2-[(4-aminocyclohexyl)-amino]-9-(tétrahydro-3-thiényl)-9H-purin-6-yl]-amino]-benzoate d'éthyle.

On procède comme au stade 3 de l'exemple 5 à partir de 513 mg de trans-1,4-diaminocyclohexane que l'on porte à une température d'environ 70 à 75°C et ajoute 181 mg du produit obtenu au stade 1 ci-dessus, porte à une température d'environ 140 à 145°C pendant 4 heures 30 minutes puis laisse revenir à température ambiante. Après purification sur silice avec pour éluant méthanol/ammoniaque (NH₄OH) en proportion de 98/2 salification par de l'acide chlorhydrique à 1,4N dans l'éthanol, on essore, lave par 10 ml d'éthanol et sèche à une température d'environ 50°C. On obtient ainsi 150 mg de produit attendu sous forme de cristaux blancs.

### RMN dans DMSO

| | |
|---|---|
| 1,33 (t) 3H | CH₃-CH₂-0 |
| 4,31 (q) | CH₃-CH₂-O |
| 1,45 (m) 4H | les H axiaux du cyclohexyle |
| 2,08 (tl) 4H | les H équatoriaux du cyclohexyle |
| 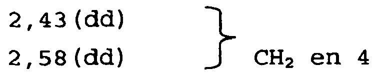 | |
| 2, 98 (m) 2H | CH₂ en 5 |
| 3, 00 (masqué) | H₄' axial |
| 3,31 (m) 2H | CH₂ en 2 |
| 3,70 (tl) 1H | H₁' axial |
| 5,12 (m) 1H | H₃ |
| 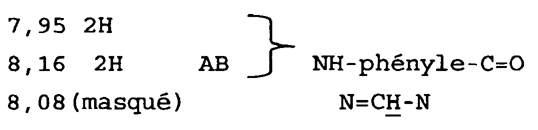 | |
| 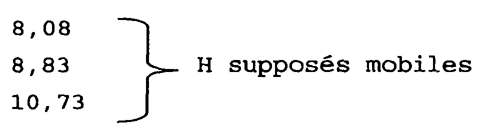 | |

### EXEMPLE 33 : Dichlorhydrate de trans(+-)-N2-(4-aminocyclohexyl)-9-(tétrahydro-3-thienyl)-N6-[4-(trifluorométhoxy)-phényl]-9H-purin-2,6-diamine.

### Stade 1 : 2-chloro-9-(tetrahydro-3-thiényl)-N-[4-(trifluorométhoxy)-phényl]-9H-purin-6-amine.

On procède comme au stade 2 de l'exemple 5 à partir de 160 mg du produit obtenu au stade 1 de l'exemple 5, 3 ml de butanol et en utilisant 0,081 ml de 4-(trifluoromethoxy)-benzenamine à la place de la benzylamine et porte à une température d'environ 80 à 85°C pendant environ 20 heures. On laisse revenir à température ambiante, dilue par 3 ml d'isopropanol, place environ 1 heure à une température d'environ 0°C, essore, lave avec 5 ml d'isopropanol et sèche sous vide à une température d'environ 50°C. Après purification sur silice avec pour éluant chlorure de méthylène/acétate d'éthyle à la proportion 90/10, on obtient ainsi 203 mg de produit attendu sous forme de cristaux blancs.

### Stade 2 : Dichlorhydrate de trans(+-)-N2-(4-aminocyclohexyl)-9-(tétrahydro-3-thiényl)-N6-[4-(trifluorométhoxy)-phényl]-9H-purin-2,6-diamine.

On procède comme au stade 3 de l'exemple 5 à partir de 186 mg du produit obtenu au stade 1 ci-dessus et 513 mg de trans-1,4-diaminocyclohexane, porte à une température d'environ 140 à 145°C pendant 4 heures 30 minutes puis laisse revenir à température ambiante. Après purification sur silice avec pour éluant méthanol/ammoniaque (NH₄OH) en proportion de 98/2 et salification par de l'acide chlorhydrique à 1,4N dans l'éthanol, on essore, lave par 10 ml d'éthanol et sèche à une température d'environ 50°C. On obtient ainsi 117 mg de produit attendu sous forme de cristaux beige rosé.

### RMN dans DMSO

| | |
|---|---|
| 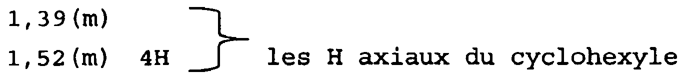 | |
| 2,06 (tl) 4H | les H équatoriaux du cyclohexyle |
| 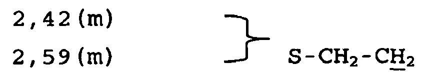 | |
| 2,99 (m) | S-CH₂-CH₂ |
| 3,00 (masqué) | H₄ supposé axial |
| 3,31(m) | S-CH₂-CH |
| 5,10 (m) 1H | S-CH₂-CH |
| 3,69 (tl) 1H | H₁ supposé axial |
| 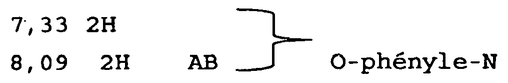 | |
| 8,04 >2H | N=CH-N + H mobiles |
| 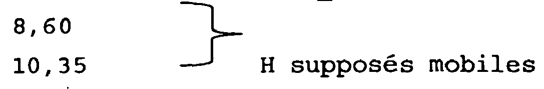 | |

### EXEMPLE 34 : Dichlorhydrate de trans(+-)-N2-(4-aminocyclohexyl)-N6-phényl-9-(tétrahydro-3-furanyl)-9H- purin-2,6-diamine.

### Stade 1 : (+-)-2-chloro-N-phényl-9-(tétrahydro-3-furanyl)-9H-purin-6-amine.

On procède comme au stade 2 de l'exemple 2 à partir de 200 mg (0,77 mM) du produit obtenu au stade 1 de l'exemple 2 et 3 ml de butanol et en utilisant 0,088 ml d'aniline (0,96 mmole) à la place de la benzylamine. On obtient ainsi 213 mg de produit attendu sous forme de cristaux blancs.

### Stade 2 : Dichlorhydrate de trans(+-)-N2-(4-aminocyclohexyl)-N6-phenyl-9-(tétrahydro-3-furanyl)-9H-purine-2,6-diamine.

On procède comme au stade 3 de l'exemple 2 à partir de 198 mg du produit obtenu au stade 1 ci-dessus et 716 mg de trans-1,4-diaminocyclohexane. Après purification sur silice avec pour éluant méthanol/ammoniaque (NH4OH) en proportion de 98/2 et salification par de l'acide chlorhydrique à 1,4N dans l'éthanol, on obtient 169 mg de produit attendu sous forme de cristaux beige rosé

### RMN dans DMSO

| | |
|---|---|
| 1,39 (m) 2H | |
| 1,53 (m) 2H | les H axiaux du cyclohexyle |
| 2,08 (m) 4H | les H équatoriaux du cyclohexyle |
| 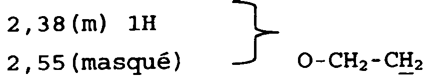 | |
| 3,03 (sl) 1H | H₄ axial |
| 3,73 (tl) 1H | H₁ axial |
| 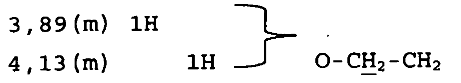 | |
| 4,04 (m) 2H | O-CH₂-CH |
| 5,14 (m) 1H | N-CH |
| 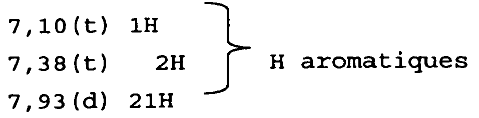 | |
| 8,06 (sl) <3H | CH-N + NH₂ |
| 8,68 à 10,36 | H mobiles |

### EXEMPLE 35 : Dichlorhydrate de trans(+-)-N2-(4-aminocyclohexyl)-9-(tétrahydro-3-furanyl)-N6-[(4-trifluorométhoxy)-phényl]-9H- purin-2,6-diamine.

### Stade 1 : (+-)-2-chloro-9-(tetrahydro-3-furanyl)-N-[4-(trifluorométhoxy)-phényl]-9H-purin-6-amine.

On procède comme au stade 2 de l'exemple 2 à partir de 200 mg (0,77 mmoles) du produit obtenu au stade 1 de l'exemple 2 et 3 ml de butanol et en utilisant 0,130 ml de 4-(trifluorométhoxy)-benzenamine (0,96 mmole) à la place de la benzylamine. On obtient ainsi 72 mg de produit attendu sous forme de cristaux blancs.

### Stade 2 : Dichlorhydrate de trans(+-)-N2-(4-aminocyclohexyl)-9-(tétrahydro-3-furalyl)-N6-[(4-trifluorométhoxy)-phényl]-9H-purin-2,6-diamine.

On procède comme au stade 3 de l'exemple 2 à partir de 153 mg du produit obtenu au stade 1 ci-dessus et 433 mg de trans-1,4-diaminocyclohexane. Après purification sur silice avec pour éluant méthanol/ammoniaque (NH₄OH) en proportion de 98/2 et salification par de l'acide chlorhydrique à 1,4N dans l'éthanol, on obtient 114 mg de produit attendu sous forme de cristaux blanc crème.

### RMN dans DMSO

| | |
|---|---|
| 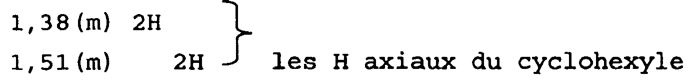 | |
| 2,07 (tl) 4H | les H équatoriaux du cyclohexyle |
| 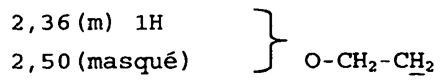 | |
| 3,03 (sl) 1H | H₄ axial |
| 3,70 (masqué) | H₁ supposé axial |
| 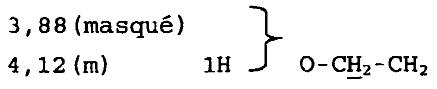 | |
| 4,02 (d) 2H | O-CH₂-CH |
| 5,10 (m) 1H | N-CH |
| 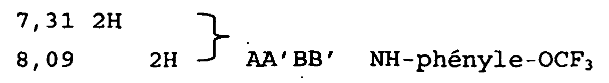 | |
| 8,00 (dl) <3H | N=CH-N + NH₂ |
| 8,41 (s) à 10,18 (s) | H mobiles |

### PRODUIT 36 : Dichlorhydrate de trans-N2-(4-aminocyclohexyl)-9-(1-éthylpropyl)-N6-propyl-9H-purin-2,6-diamine. Stade 1 : 2-chloro-9-(1-éthylpropyl)-N-propyl-9H-purin-6-amine.

On procède comme au stade 2 de l'exemple 3 à partir de 200 mg du produit obtenu au stade 1 de l'exemple 3 et 4 ml de butanol et en utilisant 0,129 ml de propylamine à la place de la benzylamine. On agite à température ambiante puis porte à une température de 80 à 85°C pendant 5 heures. On laisse revenir à température ambiante, évapore à sec puis empâte dans 5 ml de pentane à température ambiante, essore, lave et sèche à une température d'environ 50°C. Après purification sur silice avec pour éluant chlorure de méthylène/acétate d'éthyle en proportion de 90/10, on obtient 145 mg de produit attendu sous forme de cristaux blancs.

### Stade 2 : Dichlorhydrate de trans-N2-(4-aminocyclohexyl)-9-(1-éthylpropyl)-N6-propyl-9H-purin-2,6-diamine.

On procède comme au stade 3 de l'exemple 3 à partir de 121 mg du produit obtenu au stade 1 ci-dessus et 489 mg de trans-1,4-diaminocyclohexane et porte à une température de 140 à 150°C pendant 4 heures puis refroidit à 70-80°C et dilue alors avec 20 ml d'eau/acétate d'éthyle en proportion 50/50, laisse décanter, lave par 10 ml d'eau et 5 ml de chlorure de sodium en solution aqueuse saturée, sèche et évapore à sec. Après purification sur silice avec pour éluant méthanol/ammoniaque (NH₄OH) en proportion de 98/2 et salification par de l'acide chlorhydrique 1,4N dans l'éthanol, on obtient ainsi 114 mg de produit attendu sous forme de cristaux.

### RMN dans DMSO

| | |
|---|---|
| 0,77 (t) 6H | CH₃-CH₂-CH. |
| 0,95 (t) 3H | CH₃-(CH₂)₂ |
| 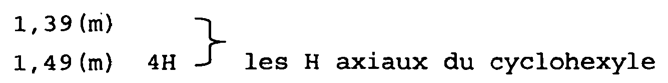 | |
| 1,65 (m) 2H | CH₃-CH₂-CH₂-NH |
| 1, 92 (m) 4H | CH₂-CH₃ |
| 2,07 (m) 4H | les H équatoriaux du cyclohexyle |
| 3,05 (sl) 1H | H₄ axial |
| 3,56 (sl) | NH-CH₂-CH₂-CH₃ |
| 3,68 (tl) 1H | H₁ axial |
| 4,19(m) | N-CH |
| 8,08 (sl) 3H | NH₂ + N=CH-N |
| 8,22 à 9,22 | H supposés mobiles |

### PRODUIT 37 : Dichlorhydrate de trans-N2-(4-aminocyclohexyl)-9-(1-éthylpropyl)-N6-phényl-9H-purin-2,6-diamine. Stade 1 : 2-chloro-9-(1-éthylpropyl)-N-phényl-9H-purin-6-amine.

On procède comme au stade 2 de l'exemple 3 à partir de 200 mg (0,77 mmole) du produit obtenu au stade 1 de l'exemple 3 et 4 ml de butanol et en utilisant 0,088 ml (0,96 mmole) d'aniline à la place de la benzylamine. On agite à température ambiante puis porte à une température de 80 à 85°C pendant 22 heures. On laisse revenir à température ambiante, dilue par 4 ml d'isopropanol, laisse pendant deux jours à une température d'environ 0°C puis essore, lave avec 10 ml d'isopropanol et sèche à une température d'environ 50°C. Après purification sur silice avec pour éluant chlorure de méthylène/acétate d'éthyle en proportion de 90/10, on obtient 104 mg de produit attendu sous forme de cristaux blancs.

### Stade 2 : Dichlorhydrate de trans-N2-(4-aminocyclohexyl)-9-(1-éthylpropyl)-N6-phényl-9H-purin-2,6-diamine.

On procède comme au stade 3 de l'exemple 3 à partir de 97 mg du produit obtenu au stade 1 ci-dessus et 350 mg de trans-1,4-diaminocyclohexane et porte à une température de 140 à 150°C environ 4 heures puis refroidit à 70-80°C et dilue alors avec 20 ml d'eau/acétate d'éthyle en proportion 50/50, laisse décanter, lave par 10 ml d'eau et 5 ml de chlorure de sodium en solution aqueuse saturée, sèche et évapore à sec. Après purification sur silice avec pour éluant méthanol/ammoniaque (NH₄OH) en proportion de 98/2 et salification par de l'acide chlorhydrique à 1,4N dans l'éthanol, on obtient ainsi 66 mg de produit attendu sous forme de cristaux.

### RMN dans DMSO

| | |
|---|---|
| 0,82 (t) 6H | CH₃-CH₂- |
| 1,96 (masqué) | CH₃-CH₂ |
| 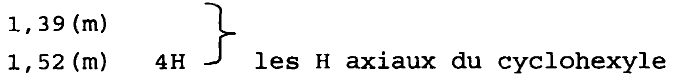 | |
| 2,08 (m) | les H équatoriaux du cyclohexyle |
| 3,02 (sl) 1H | H₄ axial |
| 3,70 (tt) 1H | H₁ axial |
| 4,32 (m) | N-CH-CH₂- |
| 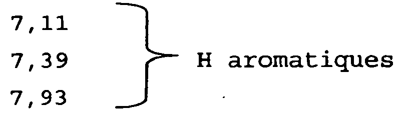 | |
| 8,03 (sl) >2H | NH₂ + N=CH-N |
| 8,78 à 10,40 | H mobiles |

### EXEMPLE 38 : Dichlorhydrate de trans-N-[2-[(4-aminocyclohexyl)-amino]-9-cyclopentyl-9H-purin-6-yl]-4-éthoxy-benzènesulfonamide.

### Stade 1 : N-[[2-chloro-9-cyclopentyl-9H-purin-6-yl]-amino]-4-éthoxy-benzènesulfonamide.

On procède comme au stade 1 de l'exemple 8 à partir de 257 mg du produit obtenu au stade 1 de l'exemple 1, 4 ml de diméthoxyéthane (DME), 390 mg de carbonate de césium (Cs₂CO₃) et 201 mg de 4-éthoxy-benzènesulfonamide à la place du benzènesulfonamide et agite à une température d'environ 100°C pendant environ 2 heures 30 minutes. On laisse revenir à température ambiante, acidifie avec 4 ml d'acide chlorhydrique 2N, extrait par 2 x 10 ml d'acétate d'éthyle, sèche, filtre et évapore à sec. On empâte dans 10 ml d'éther et sèche à température ambiante. On obtient ainsi 325 mg de produit attendu sous forme de cristaux beige.

### Stade 2 : Dichlorhydrate de trans-N-[2-[(4-aminocyclohexyl)-amino]-9-cyclopentyl-9H-purin-6-yl]-4-éthoxy-benzène-sulfonamide.

On procède comme au stade 2 de l'exemple 8 et porte 400 mg de trans-1,4-diaminocyclohexane, à une température d'environ 150°C, ajoute 295 mg du produit obtenu au stade 1 ci-dessus et maintient à cette température pendant environ 7 heures 30 minutes. Puis on laisse revenir à température ambiante. On chromatographie sur silice avec pour éluant chlorure de méthylène/méthanol/ammoniaque en proportion de 70/30/1 puis 90/10/1 pour purifier le produit. On ajoute alors 10 ml d'éthanol et 3 ml d'acide chlorhydrique/éthanol 1,4N, évapore à sec, empâte dans 10 ml d'éther et sèche sous vide à une température d'environ 60°C. On obtient ainsi 146 mg de produit attendu sous forme de cristaux beige.

### RMN dans DMSO

| | |
|---|---|
| 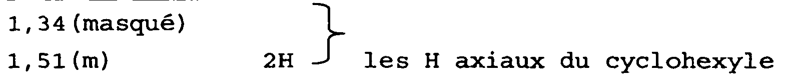 | |
| 1,34 (t) | CH₃-CH₂-O |
| 4,12 (a) 2H | CH₃-CH₂-O |
| 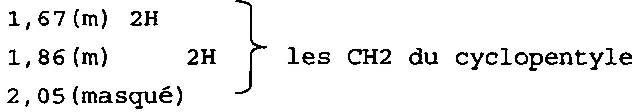 | |
| 2,05 (dl) | les H équatoriaux du cyclohexyle |
| 3,03 (sl) 1H | H₄ axial |
| 3,57 (tl) 1H | H₁ axial |
| 4,71 (m) 1H | CH du cyclopentyle |
| 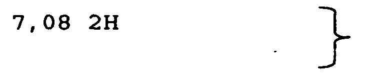 | |
| 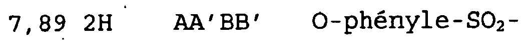 | |
| 8,11 | N=CH-N + NH₂ |
| 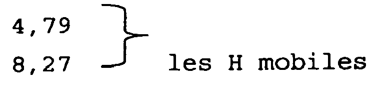 | |

### EXEMPLE 39 : Dichlorhydrate de trans-N-[2-[(4-aminocyclo-hexyl)-amino]-9-cyclopentyl-9H-purin-6-yl]-4-bromo-benzène-sulfonamide.

### Stade 1 : 4-bromo-N-[[2-chloro-9-cyclopentyl-9H-purin-6-yl]-amino]-benzènesulfonamide.

On procède comme au stade 1 de l'exemple 8 à partir de 257 mg du produit obtenu au stade 1 de l'exemple 1, 4 ml de diméthoxyéthane (DME), 390 mg de carbonate de césium (Cs₂CO₃) et 236 mg de 4-bromo-benzenesulfonamide à la place du benzènesulfonamide et agite à une température d'environ 100°C pendant environ 2 heures 30 minutes. On laisse revenir à température ambiante, acidifie avec 4 ml d'acide chlorhydrique 2N, extrait par 2 x 10 ml d'acétate d'éthyle, sèche, filtre et évapore à sec. On empâte dans 10 ml d'éther et sèche à température ambiante. On obtient ainsi 226 mg de produit attendu sous forme de cristaux beige.

### Stade 2 : Dichlorhydrate de trans-N-[2-[(4-aminocyclohexyl)-amino]-9-cyclopentyl-9H-purin-6-yl]-4-bromo-benzène-sulfonamide.

On procède comme au stade 2 de l'exemple 8 et porte 228 mg de trans-1,4-diaminocyclohexane, à une température d'environ 150°C, ajoute 182 mg du produit obtenu au stade 1 ci-dessus et maintient à cette température pendant environ 8 heures. On chromatographie sur silice avec pour éluant chlorure de méthylène/méthanol/ammoniaque en proportion de 80/20/1, ajoute alors 10 ml d'éthanol, 4 ml d'acide chlorhydrique/éthanol 1,4N et laisse une nuit. Puis on évapore à sec, empâte dans 10 ml d'éther et sèche sous vide à une température d'environ 60°C. On obtient ainsi 74 mg de produit attendu sous forme de cristaux beige.

### RMN dans DMSO

| | |
|---|---|
| 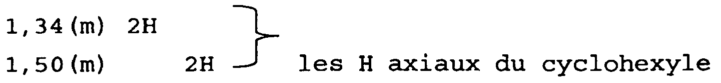 | |
| 2,07 (m) 4H | les H équatoriaux du cyclohexyle |
| 3,04 (sl) 1H | H₄ axial |
| 3,58 (tl) 1H | H₁ axial |
| 4,70 (m) 1H | N-CH du cyclopentyle |
| 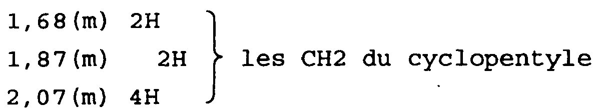 | |
| 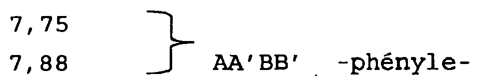 | |
| 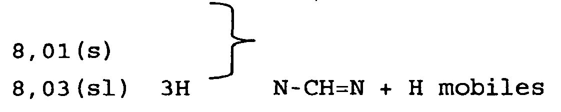 | |

### EXEMPLE 40 : Dichlorhydrate de trans-N-[2-[(4-aminocyclohexyl)-amino]-9-cyclopentyl-9H-purin-6-yl]-4-méthyl-benzène-sulfonamide.

### Stade 1 : N-[[2-chloro-9-cyclopentyl-9H-purin-6-yl]-amino]-4-méthyl-benzènesulfonamide.

On procède comme au stade 1 de l'exemple 8 à partir de 257 mg du produit obtenu au stade 1 de l'exemple 1, 4 ml de diméthoxyéthane (DME), 390 mg de carbonate de césium (CS₂CO₃) et 171 mg de 4-méthyl-benzènesulfonamide à la place du benzènesulfonamide et agite à une température d'environ 100°C pendant environ 2 heures 30 minutes. On laisse revenir à température ambiante, acidifie avec 4 ml d'acide chlorhydrique 2N, extrait par 2 x 10 ml d'acétate d'éthyle, sèche, filtre et évapore à sec. On empâte dans 10 ml d'éther et sèche à température ambiante. On obtient ainsi 292 mg de produit attendu sous forme de cristaux beige.

### Stade 2 : Dichlorhydrate de trans-N-[2-((4-aminocyclohexyl)-aminol-9-cyclopentyl-9H-purin-6-yl]-4-méthyl-benzène-sulfonamide.

On procède comme au stade 2 de l'exemple 8 et porte 285 mg de trans-1,4-diaminocyclohexane, à une température d'environ 150°C, ajoute 196 mg du produit obtenu au stade 1 ci-dessus et maintient à cette température pendant environ 7 heures. On chromatographie sur silice avec pour éluant chlorure de méthylène/méthanol/ammoniaque en proportion de 80/20/1, ajoute alors 8 ml d'éthanol, 4 ml d'acide chlorhydrique/éthanol 1,4N et laisse une nuit. Puis on évapore à sec, empâte dans 10 ml d'éther et sèche sous vide à une température d'environ 60°C. On obtient ainsi 112 mg de produit attendu sous forme de cristaux beige.

### RMN dans DMSO

| | |
|---|---|
| 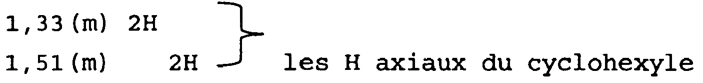 | |
| 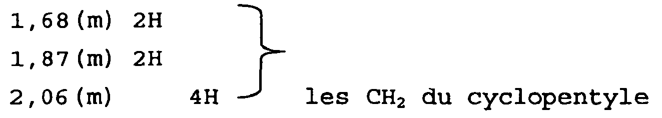 | |
| 2,06 (dl) 4H | les H équatoriaux du cyclohexyle |
| 3,02 (sl) 1H | H₄ axial |
| 3,58 (tl) 1H | H₁ axial |
| 4,71 (m) 1H | CH du cyclopentyle |
| 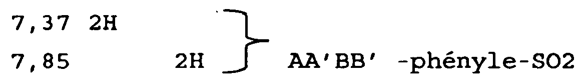 | |
| 8,12 <3H | N-CH=N + NH₂ |
| 8,16 (s) 1H | H mobile |

### PRODUIT 41 : Dichlorhydrate de trans-N2-(4-aminocyclohexyl-9-(1-éthylpropyl)-N6-[(3-iodophényl)-méthyl]-9H-purin-2,6-diamine.

### Stade 1 : 2-chloro-9-(1-ethylpropyl)-N-[(3-iodophényl)-méthyl)-9H-purin-6-amine.

On procède comme au stade 2 de l'exemple 3 à partir de 200 mg du produit obtenu au stade 1 de l'exemple 3 et 4 ml de butanol et en utilisant 0,123 ml de 3-iodo-benzèneméthanamine à la place de la benzylamine. On agite à température ambiante puis porte à une température de 80 à 85°C pendant 22 heures. On laisse revenir à température ambiante, dilue par 4 ml d'isopropanol, laisse pendant trois heures à une température d'environ 0°C puis essore, lave avec 10 ml d'isopropanol et sèche à une température d'environ 50°C. Après purification sur silice avec pour éluant cyclohexane/acétate d'éthyle/chlorure de méthylène en proportion de 70/15/15, on obtient 235 mg de produit attendu sous forme de cristaux blancs.

### Stade 2 : Dichlorhydrate de trans-N2-(4-aminocyclohexyl-9-(1-éthylpropyl)-N6-[(3-iodophényl)-méthyl]-9H-purin-2,6-diamine.

On procède comme au stade 3 de l'exemple 3 à partir de 226 mg du produit obtenu au stade 1 ci-dessus et 565 mg de trans-1,4-diaminocyclohexane et porte à une température de 140 à 150°C pendant 4 heures puis refroidit à 70-80°C et dilue alors avec 20 ml d'eau/acétate d'éthyle en proportion 50/50, laisse décanter, lave par 10 ml d'eau et 5 ml de chlorure de sodium en solution aqueuse saturée, sèche et évapore à sec. Après purification sur silice avec pour éluant méthanol/ammoniaque (NH4OH) en proportion de 98/2 et salification par de l'acide chlorhydrique à 1,4N dans l'éthanol, on obtient 160 mg de produit attendu sous forme de cristaux.

### RMN dans DMSO

| | |
|---|---|
| 0,79 (t) 6H | CH₃-CH₂-CH |
| 1,93 (m) 4H | CH₃-CH₂-CH |
| 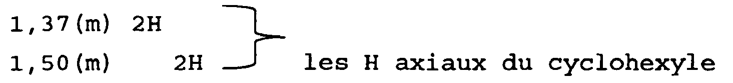 | |
| 2,05 (m) 4H | les H équatoriaux du cyclohexyle |
| 3,03 (sl) 1H | H₄ axial |
| 3,70 (tl) 1H | H₁ axial |
| 4,24 (m) | CH-(CH₂-CH₃)₂ |
| 4,83 (sl) 2H | NH-CH₂-phényle |
| 7,17 (t) 1H | H₅' |
| 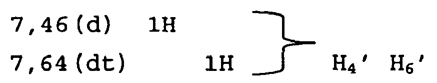 | |
| 7,82 (s) 1H | H₂' |
| 8,04 (sl) 3H | NH₂ + N=CH-N |
| 8,31 à 9,32 | H mobiles |

### PRODUIT 42 : Dichlorhydrate de trans-N2-(4-aminocyclohexyl)-9-(1-éthylpropyl)-N6-[4-(trifluorométhoxy)-phényl]-9H-purin-2,6-diamine.

### Stade 1 : 2-chloro-9-(1-éthylpropyl)-N-[4-(trifluorométhoxy)-phényl]-9H-purin-6-amine.

On procède comme au stade 2 de l'exemple 3 à partir de 200 mg du produit obtenu au stade 1 de l'exemple 3 et 4 ml de butanol et en utilisant 0, 13 ml de 4-(trifluorométhoxy)-benzenamine à la place de la benzylamine. On agite à température ambiante puis porte à une température de 80 à 85°C pendant 22 heures. On laisse revenir à température ambiante, dilue par 4 ml d'isopropanol, laisse pendant quatre heures à une température d'environ 0°C puis essore, lave avec 10 ml d'isopropanol et sèche à une température d'environ 50°C. Après purification sur silice avec pour éluant cyclohexane/acétate d'éthyle/chlorure de méthylène en proportion de 70/15/15, on obtient 169 mg de produit attendu sous forme de cristaux blancs.

### Stade 2 : Dichlorhydrate de trans-N2-(4-aminocyclohexyl)-9-(1-éthylpropyl)-N6-[4-(trifluorométhoxy)-phényl]-9H-purin-2,6-diamine.

On procède comme au stade 3 de l'exemple 3 à partir de 160 mg du produit obtenu au stade 1 ci-dessus et 456 mg de trans-1,4-diaminocyclohexane et porte à une température de 140 à 150°C pendant 4 heures puis refroidit à 70-80°C et dilue alors avec 20 ml d'eau/acétate d'éthyle en proportion 50/50 laisse décanter, lave par 10 ml d'eau et 5 ml de chlorure de sodium en solution aqueuse saturée, sèche et évapore à sec. Après purification sur silice avec pour éluant méthanol/ammoniaque (NH₄OH) en proportion de 98/2 et salification par de l'acide chlorhydrique à 1,4N dans l'éthanol, on obtient 149 mg de produit attendu sous forme de cristaux.

### RMN dans DMSO

| | |
|---|---|
| 0,77 (t) 6H | (CH₃-CH₂)₂-CH |
| 1,95 (m) | (CH₃-CH₂)₂-CH |
| 1,34 (m) 2H | |
| 1,50 (m) 2H | les H axiaux du cyclohexyle |
| 2,03 (m) 4H | les H équatoriaux du cyclohexyle |
| 3,02 (sl) 1H | H₄ axial |
| 3,64 (tl) 1H | H₁ axial |
| 4,30 (m) 1H | CH-(CH₂-CH₃)₂ |
| 7,38 | |
| 8,09 2H AA'BB' | F₃CO-phényle-N |
| 8,07 | NH₂ + N=CH-N |
| 8,93 à 10,76 | H supposés mobiles |

### PRODUIT 43 : Dichlorhydrate de trans-4-[[2-[(4-aminocyclohexyl)-amino]-9-(1-éthylpropyl)-9H-purin-6-yl]-amino]-benzoate d'éthyle.

### Stade 1 : 4-[[2-chloro-9-(1-éthylpropyl)-9H-purin-6-yl]-amino]-benzoate d'éthyle.

On procède comme au stade 2 de l'exemple 3 à partir de 200 mg du produit obtenu au stade 1 de l'exemple 3 et 4 ml de butanol et en utilisant 158 mg de 4-aminobenzoate d'éthyle à la place de la benzylamine. On agite à température ambiante puis porte à une température de 80 à 85°C pendant 22 heures. On laisse revenir à température ambiante, dilue par 4 ml d'isopropanol, laisse pendant deux jours à une température d'environ 0°C puis essore, lave avec 10 ml d'isopropanol et sèche à une température d'environ 50°C. Après purification sur silice avec pour éluant chlorure de méthylène/acétate d'éthyle en proportion de 90/10, on obtient 209 mg de produit attendu sous forme de cristaux blancs.

### Stade 2 : Dichlorhydrate de trans-4-[[2-[(4-aminocyclohexyl)-amino]-9-(1-éthylpropyl)-9H-purin-6-yl]-amino]-benzoate d'éthyle

On procède comme au stade 3 de l'exemple 3 à partir de 198 mg du produit obtenu au stade 1 ci-dessus et 582 mg de trans-1,4-diaminocyclohexane et porte à une température de 140 à 150°C pendant 4 heures puis refroidit à 70-80°C et dilue alors avec 20 ml d'eau/acétate d'éthyle en proportion 50/50, laisse décanter, lave par 10 ml d'eau et 5 ml de chlorure de sodium en solution aqueuse saturée, sèche et évapore à sec. Après purification sur silice avec pour éluant méthanol/ammoniaque (NH₄OH) en proportion de 98/2 et salification par de l'acide chlorhydrique à 1,4N dans l'éthanol, on obtient 184 mg de produit attendu sous forme de cristaux.

### RMN dans DMSO

| | |
|---|---|
| 0,80 (t) 6H | CH3-CH2-CH |
| 2,00 (q) 4H | CH3-CH2-CH |
| 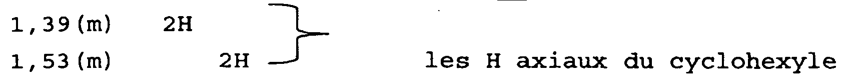 | |
| 2,09 (m) 4H | les H équatoriaux du cyclohexyle |
| 3,03 (sl) 1H | H₄ axial |
| 3,69 (ttl) 1H | H₁ axial |
| 4,32 (m) | N-CH-(CH₂)₂ + O-CH₂-CH₃ |
| 1,34 (t) 3H | O-CH₂-CH₃ |
| 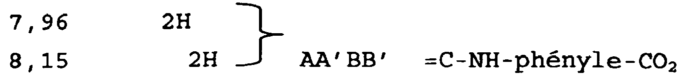 | |
| 8,08 | NH₂ + N=CH-N |
| 9,00 à 10,86 | H supposés mobiles |

### PRODUIT 44 : Dichlorhydrate de trans(+-)-N2-(4-aminocyclohexyl)-N6-[(3-iodophényl)-méthyl]-9-(1-méthylpropyl)-9H-purin-2,6-diamine.

### Stade 1 : (+-)-2-chloro-N-[(3-iodophényl)-méthyl]-9-(1-méthylpropyl)-9H-purin-6-amine.

On procède comme au stade 2 de l'exemple 9 à partir de 200 mg du produit obtenu au stade 1 de l'exemple 9 et 4 ml de butanol et en utilisant 0,128 ml de 3-iodo-benzèneméthanamine à la place de la benzylamine. On porte à une température de 80 à 85°C pendant environ 22 heures. On laisse revenir à température ambiante, dilue avec 4 ml d'isopropanol et place deux jours à une température d'environ 0°C. Puis on essore, lave avec 10 ml d'isopropanol et sèche à une température d'environ 50°C. Après purification sur silice avec pour éluant chlorure de méthylène/acétate d'éthyle en proportion de 90/10, on obtient 290 mg de produit attendu sous forme de cristaux blancs.

### Stade 2 : Dichlorhydrate de trans(+-)-N2-(4-aminocyclohexyl)-N6-[(3-iodophényl)-méthyl]-9-(1-méthylpropyl)-9H-purin-2,6-diamine.

On procède comme au stade 3 de l'exemple 9 à partir de 279 mg du produit obtenu au stade 1 ci-dessus et 720 mg de trans-1, 4-diaminocyclohexane. Après purification dans les mêmes conditions que pour l'exemple 9, on salifie par 10 ml HCl/éthanol 1,4N et évapore à sec. On obtient ainsi 232 mg de produit attendu sous forme de cristaux.

### RMN dans DMSO

| | |
|---|---|
| 0,80 (t) 3H | CH₃-CH₂- |
| 1,78 à 2,00 2H | CH₃-CH₂ |
| 1,27 à 1,58 (m) 4H | les H axiaux du cyclohexyle |
| 1,52 (d) 3H | CH₃-CH |
| 2,03 (m) 4H | les H équatoriaux du cyclohexyle |
| 3,03 (sl) 1H | H₄ axial |
| 3,69 (tl) 1H | H₁ axial |
| 4,49 (m) 1H | N-CH-CH₃ |
| 4,78 (sl) 2H | NH-CH₂-phényle |
| 7,16 (t) | H₅ |
| 7,46 (dl) 1H | |
| 7,64 (dt) 1H | H₄, H₆ |
| 7,80 (sl) 1H | H₂ |
| 8,06 (sl) <3H | NH₂ + N=CH-N |
| 8,41 à 9,42 | H supposés mobiles |

### EXEMPLE 45 : Trichlorhydrate de trans-N-(4-aminocyclohexyl)-2-[[[2-[[2-[(4-aminocyclohexyl)-amino]-9-cyclopentyl-9H-purin-6-yl]-amino]-éthyl]-amino]-sulfonyl]-benzamide.

### Stade 1 : 2[[[2-[(2-chloro-9-cyclopentyl-9H-purin-6-yl)-amino]-éthyl]-amino]-sulfonyl]-benzoate d'éthyle.

On procède comme au stade 1 de l'exemple 10 à partir de 280 mg du produit obtenu au stade 1 de l'exemple 7, 4 ml de chlorure de méthylène, 0,2 ml de triéthylamine et 400 mg de 2-(chlorosulfonyl)-benzoate de méthyle à la place de chlorure de l'acide 4-méthyl-benzènesulfonique puis agite à température ambiante pendant environ 30 minutes. On ajoute alors 5 ml d'eau, extrait par 2 x 10 ml de chlorure de méthylène, lave avec 5 ml de H₂O, sèche et évapore. Après chromatographie sur silice avec pour éluant chlorure de méthylène/acétate d'éthyle en proportion de 70/30, on obtient ainsi 129 mg de produit attendu.

### Stade 2 : Trichlorhydrate de trans-N-(4-aminocyclohexyl)-2-[[[2-[[2-[(4-aminocyclohexyl)-amino]-9-cyclopentyl-9H-purin-6-yl]-amino]-éthyl]-aminol-sulfonyl]-benzamide.

On procède comme au stade 2 de l'exemple 10 à partir de 111 mg du produit obtenu au stade 1 ci-dessus et 262 mg de trans-1,4-diaminocyclohexane et chauffe à environ 140°C pendant environ 3, heures puis descend à 80°C, ajoute alors par 5 ml AcOEt puis 10 ml d'eau à chaud, laisse revenir à température ambiante, extrait avec 2 x 10 ml d'acétate d'éthyle, lave par 10 ml de solution aqueuse de chlorure de sodium saturé puis sèche. Après purification par chromatographie sur silice avec pour éluant chlorure de méthylène/méthanol/ammoniaque en proportion de 80/20/2,5, on ajoute 5 ml d'acide chlorhydrique/éthanol 1,4N, laisse cristalliser puis filtre, rince avec ml d'éther et sèche sous vide. On obtient ainsi 58 mg de produit attendu sous forme de solide beige.

### RMN dans DMSO

| | |
|---|---|
| 1,29 à 1,62 8H | les H axiaux du cyclohexyle |
| 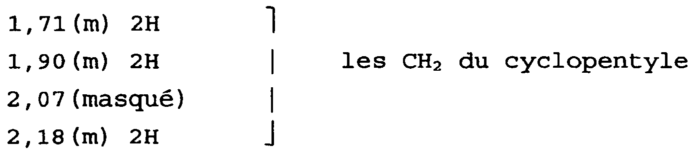 | |
| 2,07 (m) 8H | les H équatoriaux du cyclohexyle |
| 3,02 (sl) >2H en excès | les H₄-H₄' axiaux |
| 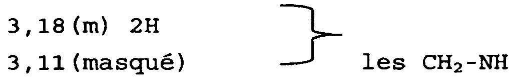 | |
| 3,71 (masqué) 3H | les H₁-H₁' supposés axiaux |
| 4,76 (m) 1H | CH du cyclopentyle |
| 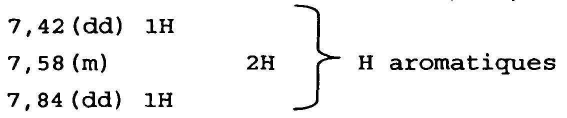 | |
| 8,17 | les NH₂ + N=CH-N |
| 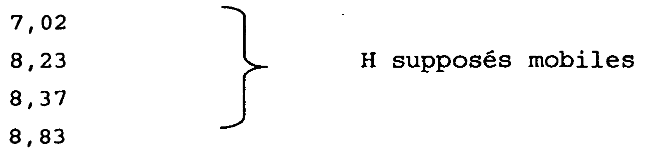 | |

### EXEMPLE 46 : Dichlorhydrate de trans-N-(2-[[2-[(4-aminocyclohexyl)-amino]-9-cyclopentyl-9H-purin-6-yl]-amino)-éthyl]-trifluorométhanesulfonamide.

### Stade 1 : N-[2-[(2-chloro-9-cyclopentyl-9H-purin-6-yl)-amino]-éthyl]-trifluorométhanesulfonamide.

On procède comme au stade 1 de l'exemple 10 à partir de 280 mg du produit obtenu au stade 1 de l'exemple 7, 3 ml de chlorure de méthylène, 0,17 ml de triéthylamine, 0,128 ml de chlorure de l'acide trifluorométhane-sulfonique à la place du chlorure de l'acide 4-méthyl-benzènesulfonique puis agite à température ambiante pendant environ 30 minutes. On ajoute alors 2 ml d'eau, extrait par 3 x 5 ml de chlorure de méthylène, lave avec 5 ml de chlorure de sodium saturé, sèche et évapore. On empâte avec 5 ml d'éther puis avec 5 ml de pentane essore, et sèche. On obtient ainsi 315 mg de produit attendu sous forme de solide beige.

### Stade 2 : Dichlorhydrate de trans-N-(2-[[2-[(4-aminocyclohexyl)-amino]-9-cyclopentyl-9H-purin-6-yl]-amino]-éthyl]-trifluoromethanesulfonamide.

On procède comme au stade 2 de l'exemple 10 à partir de 292 mg du produit obtenu au stade 1 ci-dessus et 810 mg de trans-1,4-diaminocyclohexane et chauffe à environ 140°C pendant environ 3 heures puis descend à 80°C, ajoute 5 ml AtOEt puis 10 ml d'eau à chaud, laisse revenir à température ambiante, extrait avec 2 x 10 ml d'acétate d'éthyle, lave par 10 ml de chlorure de sodium saturé, puis sèche. Après purification par chromatographie sur silice avec pour éluant chlorure de méthylène/méthanol/ammoniaque en proportion de 85/15/1,5, on ajoute 5 ml d'acide chlorhydrique/éthanol 1,4N, laisse cristalliser puis filtre, rince avec 5 ml d'éther et sèche sous vide. On obtient ainsi 167 mg de produit attendu sous forme de solide blanc.

### RMN dans DMSO

| | |
|---|---|
| 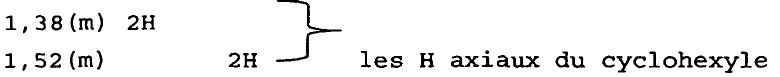 | |
| 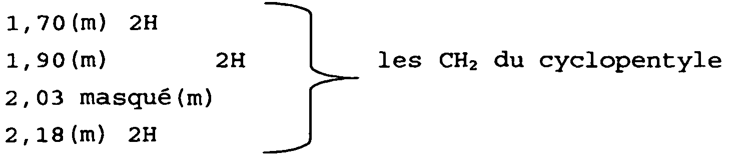 | |
| 2,07 (m) 4H | les H équatoriaux du cyclohexyle |
| 3,05 (sl) 1H | H₄ axial |
| 3,46 (tl) 2H | CH₂-NH-SO₂ |
| 3,74 (tl) | H₁ axial |
| 3,79 (masqué) | CH₂-NH-C=N |
| 4,77 (m) 1H | CH du cyclopentyle |
| 8,02 <3H | les NH₂ + N=CH-N |
| 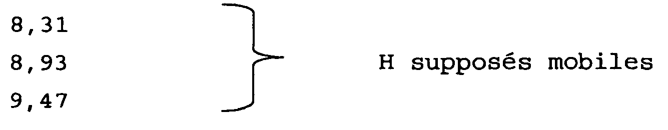 | |

### EXEMPLE 47 : Trichlorhydrate de trans-4-[(4-aminocyclohexyl)-amino]-N-[2-[[2-[(4-aminocyclohexyl)amino]-9-cyclopentyl-9H-purin-6-yl]-amino]-éthyl]-benzène-sulfonamide.

### Stade 1 : N-[2-[(2-chloro-9-cyclopentyl-9H-purin-6-yl)-amino]-éthyl]-4-fluoro-benzènesulfonamide.

On procède comme au stade 1 de l'exemple 10 à partir de 280 mg du produit obtenu au stade 1 de l'exemple 7, 3 ml de chlorure de méthylène, 0,17 ml de triéthylamine et 0,128 ml de chlorure de l'acide 4-fluorobenzène-sulfonique à la place du chlorure de l'acide 4-méthyl-benzènesulfonique puis agite à température ambiante pendant environ 30 minutes. On ajoute alors 2 ml d'eau, extrait par 3 x 5 ml de chlorure de méthylène, lave avec 5 ml de chlorure de sodium saturé, sèche et évapore. On empâte avec 5 ml d'éther puis avec 5 ml de pentane essore, et sèche. On obtient ainsi 360 mg de produit attendu.

### Stade 2 : Trichlorhydrate de trans-4-[(4-aminocyclohexyl)-amino]-N-[2-[[2-[(4-aminocyclohexyl)amino]-9-cyclopentyl-9H-purin-6-yl]-amino]-éthyl]-benzène-sulfonamide.

On procède comme au stade 2 de l'exemple 10 à partir de 173 mg du produit obtenu au stade 1 ci-dessus et 450 mg de trans-1,4-diaminocyclohexane et chauffe à environ 140°C pendant environ 3 heures puis laisse revenir à 80°C, ajoute 5 ml AcOEt puis 5 ml d'eau à chaud. On laisse revenir à température ambiante, extrait avec 2 x 10 ml d'acétate d'éthyle, lave par 10 ml de chlorure de sodium saturé puis sèche. Après purification par chromatographie sur silice avec pour éluant chlorure de méthylène/méthanol/ammoniaque en proportion de 75/22/03, on ajoute 5 ml d'acide chlorhydrique/éthanol 1,4N, laisse cristalliser puis filtre, rince avec 5 ml d'éther et sèche sous vide. On obtient ainsi 62 mg de produit attendu sous forme de cristaux blancs.

### RMN dans DMSO

| | |
|---|---|
| 1,15 à 1,61 (m) 8H | les H axiaux du cyclohexyle |
| 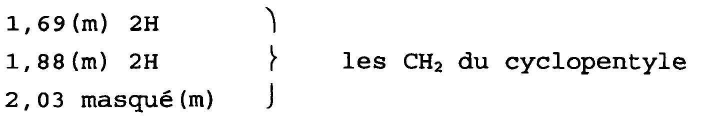 | |
| 2,17 (m) 2H | |
| 2,03 (m) 8H | les H équatoriaux du cyclohexyle |
| 2,99 (m) 4H | 1 CH₂-CH₂-NH + H₄-H₄' supposés axiaux |
| 3,22(tl) 1H | H₁' axial |
| 3,70 (m) 3H | 1 CH₂-CH₂-NH + H₁ supposé axial |
| 4,75 (m) 1H | CH du cyclopentyle |
| 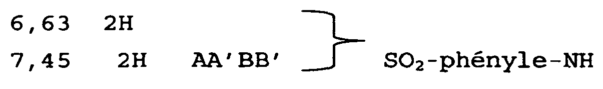 | |
| 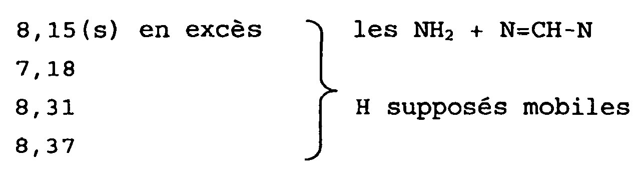 | |
| 8,96 | |

### EXEMPLE 48 : Dichlorhydrate de trans-N-(2-[[2-[[4-aminocyclohexyl)-amino]-9-cyclopentyl-9H-purin-6-yl]-amino]-éthyl]-4-(trifluorométhyl)-benzènesulfonamide.

### Stade 1 : N-[2-[(2-chloro-9-cyclopentyl-9H-purin-6-yl)-amino]-éthyl]-4-(trifluorométhyl)-benzènesulfonamide.

On procède comme au stade 1 de l'exemple 10 à partir de 280 mg du produit obtenu au stade 1 de l'exemple 7, 3 ml de chlorure de méthylène, 0,17 ml de triéthylamine et 280 mg de chlorure de l'acide 4-(trifluorométhyl)-benzènesulfonique à la place du chlorure de l'acide 4-méthylbenzènesulfonique, puis agite à température ambiante pendant environ 30 minutes. On ajoute alors 2 ml d'eau, extrait par 3 x 5 ml de chlorure de méthylène, lave avec 5 ml de chlorure de sodium saturé, sèche et évapore. On empâte avec 5 ml d'éther puis avec 5 ml de pentane, essore, et sèche. On obtient ainsi 375 mg de produit attendu.

### Stade 2 : Dichlorhydrate de trans-N-(2-[[2-[[4-aminocyclohexyl)-amino]-9-cyclopentyl-9H-purin-6-yl]-amino]-éthyl]-4-(trifluorométhyl)-benzènesulfonamide.

On procède comme au stade 2 de l'exemple 10 à partir de 188 mg du produit obtenu au stade 1 ci-dessus et 440 mg de trans-1,4-diaminocyclohexane et chauffe à environ 140°C pendant environ 3 heures. On laisse revenir à 80°C, ajoute 5 ml de AcOEt puis 5 ml d'eau à chaud. On laisse revenir à température ambiante puis extrait avec 2 x 5 ml d'acétate d'éthyle, lave par 5 ml de chlorure de sodium saturé, puis sèche. Après purification par chromatographie sur silice avec pour éluant chlorure de méthylène/méthanol/ammoniaque en proportion de 90/10/1, on ajoute 5 ml d'acide chlorhydrique/éthanol 1,4N, laisse cristalliser puis filtre, rince avec 5 ml d'éther et sèche sous vide. On obtient ainsi 148 mg de produit attendu sous forme de cristaux blancs.

### RMN dans DMSO

| | |
|---|---|
| 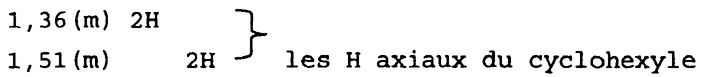 | |
| 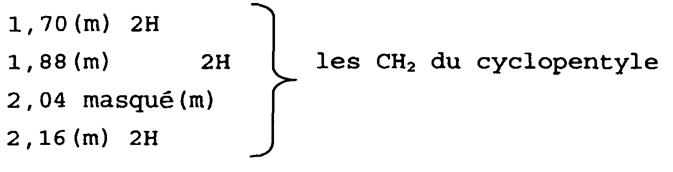 | |
| 2,06 | les H équatoriaux du cyclohexyle |
| 3,05 (sl) | H₄ axial |
| 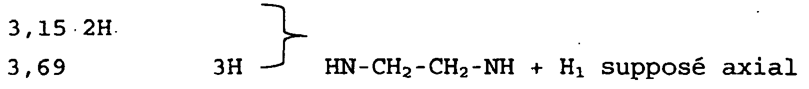 | |
| 4,75 (m) 1H | CH du cyclopentyle |
| 7,88 2H | |
| 8,00 2H AA'BB' | F₃C-phényle-SO₂ |
| 8,10 3H | les NH₂ + N=CH-N |
| 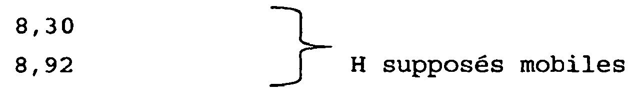 | |

### PRODUIT 49 : Dichlorhydrate de trans(+-)-N2-(4-aminocyclohexyl)-9-(1-méthylpropyl)-N6-propyl-9H-purin-2,6-diamine.

### Stade 1 : (+-)-2-chloro-9-(1-méthylpropyl)N-propyl-9H-purin-6-amine.

On procède comme au stade 2 de l'exemple 9 à partir de 200 mg du produit obtenu au stade 1 de l'exemple 9 et 4 ml de butanol et en utilisant 0,132 ml 1-propanamine à la place de la benzylamine. On agite à température ambiante puis porte à une température de 80 à 85°C pendant environ 22 heures. On laisse revenir à température ambiante. On reprend avec 5 ml d'acétate d'éthyle et évapore à sec. On empâte à température ambiante dans 5 ml de pentane, essore, lave avec 5 ml de pentane et sèche à une température d'environ 50°C. Après purification sur silice avec pour éluant chlorure de méthylène/acétate d'éthyle en proportion de 90/10, on obtient 203 mg de produit attendu sous forme de cristaux blancs.

### Stade 2 : Dichlorhydrate de trans(+-)-N2-(4-aminocyclohexyl)-9-(1-méthylpropyl)-N6-propyl-9H-purin-2,6-diamine.

On procède comme au stade 2 de l'exemple 44 à partir de 117 mg du produit obtenu au stade 1 ci-dessus et 500 mg de trans-1,4-diaminocyclohexane. On obtient ainsi 80 mg de produit attendu sous forme de cristaux.

### RMN dans DMSO

| | |
|---|---|
| 0,81 (t) 3H | CH₃-CH₂-CH |
| 0,96 (t) 3H | CH₃-CH₂-CH₂ |
| 1,38 à 1,47 4H | les H axiaux du cyclohexyle |
| 1,52 (d) 2H | CH₃-CH |
| 1,66 (m) 2H | CH₃-CH₂-CH₂ |
| 1,96 (m) 2H | CH₃-CH₂-CH |
| 2,08 (m) 4H | les H équatoriaux du cyclohexyle |
| 3,04 (sl) 1H | H₄ axial |
| 3,63 (sl) 2H | HN-CH₂- (chaîne) |
| 3,72 (tl) 1H | H₁ axial |
| 4,43 (m) 1H | N-CH- |
| 8,11 (sl) <3H | NH₂ + N=CH-N |
| 8,25 à 9,12 | H supposés mobiles |

### PRODUIT 50 : Dichlorhydrate de trans(+-)-N2-(4-aminocyclohexyl))-9-(1-méthylpropyl)-N6-[4-(trifluorométhoxy)-phényl]-9H-purin-2,6-diamine.

### Stade 1 : (+-)-2-chloro-9-(1-méthylpropyl)-N-[4-(trifluorométhoxy)-phényl]-9H-purin-6-amine.

On procède comme au stade 2 de l'exemple 9 à partir de 200 mg du produit obtenu au stade 1 de l'exemple 9 et 4 ml de butanol et en utilisant 0,135 ml de 4-(trifluorométhoxy)-benzènamine à la place de la benzylamine. On agite à température ambiante puis porte à une température de 80 à 85°C pendant environ 22 heures. On laisse revenir à température ambiante, dilue avec 4 ml d'isopropanol et place deux jours à une température d'environ 0°C. Puis on essore, lave avec 5 ml d'isopropanol et sèche à une température d'environ 50°C. Après purification sur silice avec pour éluant chlorure de méthylène/acétate d'éthyle en proportion de 90/10, on obtient 210 mg de produit attendu sous forme de cristaux blancs.

### Stade 2 : Dichlorhydrate de trans(+-)-N2-(4-aminocyclohexyl)-9-(1-méthylpropyl)-N6-[4-(trifluorométhoxy)-phényl]-9H-purin-2,6-diamine.

On procède comme au stade 2 de l'exemple 44 à partir de 201 mg du produit obtenu au stade 1 ci-dessus et 595 mg de trans-1,4-diaminocyclohexane. On obtient ainsi 139 mg de produit attendu sous forme de cristaux.

### RMN dans DMSO

| | |
|---|---|
| 0,84 (t) 3H | CH₃-CH₂ |
| 1,83 à 2,02 (m) 2H | CH₃-CH₂ |
| 1,38 à 1,53 4H | les H axiaux du cyclohexyle |
| 1,57 (d) 3H | CH₃-CH |
| 2,05 (m) 4H | les H équatoriaux du cyclohexyle |
| 3,02 (sl) 1H | H₄ axial |
| 3,69 (tl) 1H | H₁ axial |
| 4,54 (m) 1H | N-CH-CH₂- |
| 7,36 | |
| 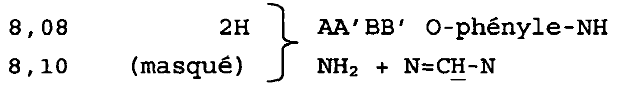 | |
| 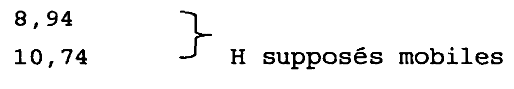 | |

### PRODUIT 51 : Dichlorhydrate de trans(+-)-4-[[2-[[4-aminocyclohexyl)-amino]-9-(1-méthylpropyl)-9H-purin-6-yl]amino]-benzoate d'éthyle.

### Stade 1 : (+-)-4-[[2-chloro-9-(1-méthylpropyl)-9H-purin-6-yl]-amino]-benzoate d'éthyle.

On procède comme au stade 2 de l'exemple 9 à partir de 200 mg du produit obtenu au stade 1 de l'exemple 9 et 4 ml de butanol et en utilisant 0,165 mg de 4-aminobenzoate d'éthyle à la place de la benzylamine. On agite à température ambiante puis porte à une température de 80 à 85°C pendant environ 22 heures. On laisse revenir à température ambiante, dilue avec 4 ml d'isopropanol et place deux jours à une température d'environ 0°C. Puis on essore, lave avec 5 ml d'isopropanol et sèche à une température d'environ 50°C. Après purification sur silice avec pour éluant chlorure de méthylène/acétate d'éthyle en proportion de 90/10, on obtient 289 mg de produit attendu sous forme de cristaux blancs.

### Stade 2 : Dichlorhydrate de trans(+-)-4-[[2-[[4-aminocyclohexyl)-amino]-9-(1-méthylpropyl)-9H-purin-6-yl]-amino]-benzoate d'éthyle.

On procède comme au stade 2 de l'exemple 44 à partir de 275 mg du produit obtenu au stade 1 ci-dessus et 840 mg de trans-1,4-diaminocyclohexane. On obtient ainsi 267 mg de produit attendu sous forme de cristaux.

### RMN dans DMSO

| | |
|---|---|
| 0,84 (t) 3H | CH₃-CH₂ |
| 1,85 à 2,04 (m) 2H | CH₃-CH₂ |
| 1,34 (t) 3H | CH₃-CH₂-O |
| 4,32 (q) 2H | CH₃-CH₂-O |
| 1,37 à 1,53 4H | les H axiaux du cyclohexyle |
| 1,57 (d) 3H | CH₃-CH |
| 2,09 (m) 4H | les H équatoriaux du cyclohexyle |
| 3,03 (sl) 1H | H₄ axial |
| 3,71 (tl) 1H | H₁ axial |
| 4,56 (m) 1H | N-CH-CH₂- |
| 7,96 2H | |
| 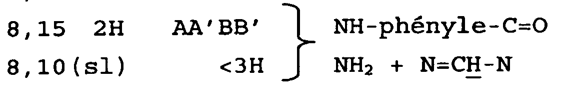 | |
| 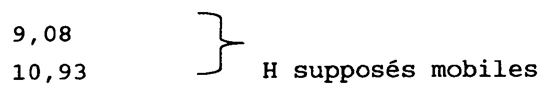 | |

### PRODUIT 52 : Dichlorhydrate de trans(+-)-N2-(4-aminocyclohexyl)-9-(1-méthylpropyl)-N6-phényl-9H-purin-2,6-diamine.

### Stade 1 : (+-)-2-chloro-9-(1-méthylpropyl)-N-phényl-9H-purin-6-amine.

On procède comme au stade 2 de l'exemple 9 à partir de 200 mg du produit obtenu au stade 1 de l'exemple 9 et 4 ml de butanol et en utilisant 0,091 ml d'aniline à la place de la benzylamine. On agite à température ambiante puis porte à une température de 80 à 85°C pendant environ 22 heures. On laisse revenir à température ambiante, dilue avec 4 ml d'isopropanol et place deux jours à une température d'environ 0°C. Puis on essore, lave avec 5 ml d'isopropanol et sèche à une température d'environ 80°C. Après purification sur silice avec pour éluant chlorure de méthylène/acétate d'éthyle en proportion de 90/10, on obtient 176 mg de produit attendu sous forme de cristaux blancs.

### Stade 2 : Dichlorhydrate de trans(+-)-N2-(4-aminocyclohexyl)-9-(1-méthylpropyl)-N6-phényl-9H-purin-2,6-diamine.

On procède comme au stade 2 de l'exemple 44 à partir de 166 mg du produit obtenu au stade 1 ci-dessus et 629 mg de trans-1,4-diaminocyclohexane. On obtient ainsi 158 mg de produit attendu sous forme de cristaux.

### RMN dans DMSO

| | |
|---|---|
| 0,84 (t) 3H | CH₃-CH₂ |
| 1,83 à 2,10 (m) 2H | CH₃-CH₂ |
| 1,29 à 1,61 4H | les H axiaux du cyclohexyle |
| 1,57 (d) 3H | CH₃-CH |
| 2,08 (m) 4H | les H équatoriaux du cyclohexyle |
| 3,02 (sl) 1H | H₄ axial |
| 3,70 (tl) 1H | H₁ axial |
| 4,55 (m) 1H | -CH-CH₂- |
| 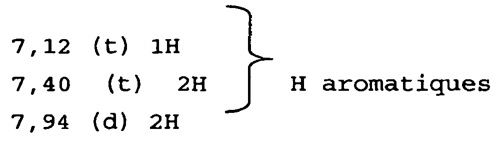 | |
| 8,10 (sl) <3H | NH₂ + N=CH-N |
| 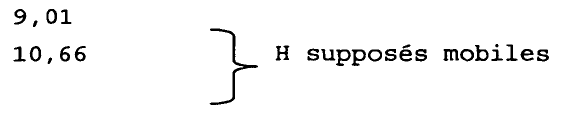 | |

### EXEMPLE 53 : Dichlorhydrate de trans-N-[2-[[2-[(4-aminocyclohexyl)-amino]-9-cyclopentyl-9H-purin-6-yl]-amino]-éthyl]-4-méthoxy-benzenesulfonamide.

### Stade 1 : N-[2-[(2-chloro-9-cyclopentyl-9H-purin-6-yl)-amino]-éthyl]-4-méthoxy-benzenesulfonamide.

On procède comme au stade 1 de l'exemple 10 à partir de 280 mg du produit obtenu au stade 1 de l'exemple 7, 4 ml de chlorure de méthylène, 0,2 ml de triéthylamine et 248 mg de chlorure de l'acide 4-méthoxy-benzènesulfonique à la place du chlorure de l'acide 4-méthylbenzènesulfonique puis agite à température ambiante pendant environ une nuit. On ajoute alors 2 ml d'eau, extrait par 3 x 5 ml de chlorure de méthylène, lave avec 5 ml de chlorure de sodium saturé, sèche et évapore. On empâte avec 5 ml d'éther puis avec 5 ml de pentane, essore, et sèche. Après chromatographie sur silice avec pour éluant chlorure de méthylène/CH₃CN en proportion de 70/30, on obtient ainsi 250 mg de produit attendu sous forme de solide beige.

### Stade 2 : Dichlorhydrate de trans-N-[2-[[2-[(4-aminocyclohexyl)-amino]-9-cyclopentyl-9H-purin-6-yl]-amino]-éthyl]-4-méthoxy-benzènesulfonamide.

On procède comme au stade 2 de l'exemple 10 à partir de 126 mg du produit obtenu au stade 1 ci-dessus et 319 mg de trans-1,4-diaminocyclohexane et chauffe à environ 140°C pendant environ 3 heures. On laisse revenir à 80°C, ajoute 5 ml AcOEt, ajoute 5 ml d'eau à chaud. On laisse revenir à température ambiante, extrait avec 2 x 5 ml d'acétate d'éthyle, lave par 5 ml de chlorure de sodium saturé, puis sèche. Après purification par chromatographie sur silice avec pour éluant chlorure de méthylène/méthanol/ammoniaque en proportion de 85/15/1,5, on ajoute 5 ml d'acide chlorhydrique/éthanol 1,4N, laisse cristalliser puis filtre, rince avec 5 ml d'éther et sèche sous vide. On obtient ainsi 95 mg de produit attendu sous forme de solide beige.

### RMN dans DMSO

| | |
|---|---|
| 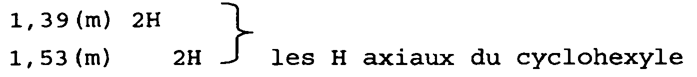 | |
| 2,05 | les H équatoriaux du cyclohexyle |
| 1,71 et 1,90 | CH₂-CH₂-CH-N |
| 2,00 et 2,17 | CH₂-CH₂-CH-N |
| 4,75 | CH₂-CH₂-CH-N |
| 3,03 (masqué) | H₄ axial |
| 3,71 (masqué) | H₁ axial |
| 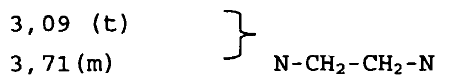 | |
| 3,82 (s) | phényle-O-méthyle |
| 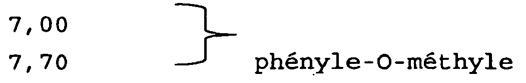 | |
| 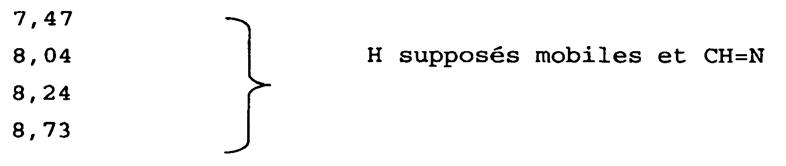 | |

### EXEMPLE 54 : Dichlorhydrate de trans-N-[2-[[2-[(4-aminocyclohexyl)-amino]-9-cyclopentyl-9H-purin-6-yl]-amino]-éthyl]-4-chloro-benzènesulfonamide.

### Stade 1 : 4-chloro-N-[2-[(2-chloro-9-cyclopentyl-9H-purin-6-yl)-amino]-éthyl]-benzènesulfonamide.

On procède comme au stade 1 de l'exemple 10 à partir de 280 mg du produit obtenu au stade 1 de l'exemple 7, 4 ml de chlorure de méthylène, 0,2 ml de triéthylamine et 255 mg de chlorure de l'acide 4-chlorobenzènesulfonique à la place du chlorure de l'acide 4-méthyl-benzènesulfonique puis agite à température ambiante pendant 30 minutes. On ajoute alors 2 ml d'eau, extrait par 3 x 5 ml de chlorure de méthylène, lave avec 5 ml de chlorure de sodium saturé, sèche et évapore. On empâte avec 5 ml d'éther puis avec 5 ml de pentane, essore, et sèche. Après chromatographie sur silice avec pour éluant chlorure de méthylène/CH₃CN en proportion de 70/30, on obtient 350 mg de produit attendu sous forme de solide beige.

### Stade 2 : Dichlorhydrate de trans-N-[2-[[2-[(4-aminocyclohexyl)-amino]-9-cyclopentyl-9H-purin-6-yl]-amino]-éthyl]-4-chloro-benzènesulfonamide.

On procède comme au stade 2 de l'exemple 10 à partir de 161 mg du produit obtenu au stade 1 ci-dessus et 403 mg de trans-1,4-diaminocyclohexane et chauffe à environ 140°C pendant environ 3 heures 30 minutes. On laisse revenir à 80°C, ajoute 5 ml de AcOEt puis 5 ml d'eau à chaud. On laisse revenir à température ambiante, extrait avec 2 x 5 ml d'acétate d'éthyle, lave par 5 ml de chlorure de sodium saturé, puis sèche. Après purification par chromatographie sur silice avec pour éluant chlorure de méthylène/méthanol/ammoniaque en proportion de 85/15/1,5, on ajoute 5 ml d'acide chlorhydrique/éthanol 1,4N, laisse cristalliser puis filtre, rince avec 5 ml d'éther et sèche sous vide. On obtient ainsi 113 mg de produit attendu sous forme de cristaux beige blanc.

### RMN dans DMSO

| | |
|---|---|
| 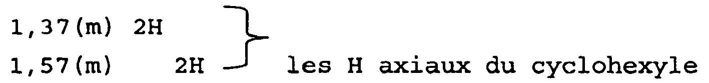 | |
| 2,04 | les H équatoriaux du cyclohexyle |
| 1,70 et 1,89 | CH₂-CH₂-CH-N |
| 2,00 et 2,17 | CH₂-CH₂-CH-N |
| 4,75 | CH₂-CH₂-CH-N |
| 3,05 (masqué) | H₄ axial |
| 3,70 (masqué) | H₁ axial |
| 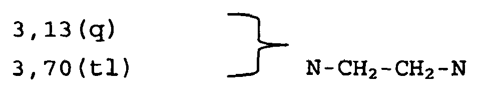 | |
| 7,84 (t) | NH-CH₂ |
| 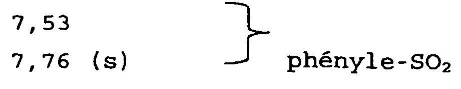 | |
| 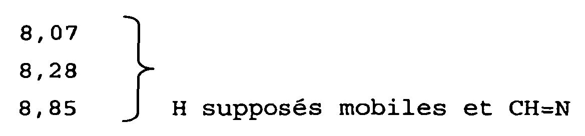 | |

### EXEMPLE 55 : Dichlorhydrate de trans-N-[2-[2-[(4-amino-cyclohexyl)-amino]-9-cyclopentyl-9H-purin-6-yl]-amino]-éthyl]-1-méthyl-éthanesulfonamide.

### Stade 1 : N-[2-[(2-chloro-9-cyclopentyl-9H-purin-6-yl)-amino]-éthyl]-1-méthyl-éthanesulfonamide.

On procède comme au stade 1 de l'exemple 10 à partir de 280 mg du produit obtenu au stade 1 de l'exemple 7, 4 ml de chlorure de méthylène, 0,2 ml de triéthylamine et 172 mg de chlorure de l'acide 1-méthyléthanesulfonique à la place du chlorure de l'acide 4-méthylbenzènesulfonique puis agite à température ambiante pendant environ 30 minutes. On ajoute alors 2 ml d'eau, extrait par 3 x 5 ml de chlorure de méthylène, lave avec 5 ml de chlorure de sodium saturé, sèche et évapore. On empâte avec 5 ml d'éther puis avec 5 ml de pentane, essore et sèche. Après chromatographie sur silice avec pour éluant chlorure de méthylène/CH₃CN en proportion de 70/30, on obtient ainsi 115 mg de produit attendu sous forme de cristaux blancs.

### Stade 2 : Dichlorhydrate de trans-N-[2-[2-[(4-aminocyclohexyl)-amino]-9-cyclopentyl-9H-purin-6-yl]-amino]-éthyl]-1-méthyl-éthanesulfonamide.

On procède comme au stade 2 de l'exemple 10 à partir de 157 mg du produit obtenu au stade 1 ci-dessus et 315 mg de trans-1,4-diaminocyclohexane et chauffe à environ 140°C pendant environ 3 heures 30 minutes. On descend à 80°C, rajoute 5 ml AcOEt, ajoute 5 ml d'eau à chaud. On laisse revenir à température ambiante, extrait avec 2 x 5 ml d'acétate d'éthyle, lave par 5 ml de chlorure de sodium saturé, puis sèche. Après purification par chromatographie sur silice avec pour éluant chlorure de méthylène/méthanol/ammoniaque en proportion de 85/15/1,5, on ajoute 5 ml d'acide chlorhydrique à 1,4N dans l'éthanol, laisse cristalliser puis filtre, rince avec 5 ml d'éther et sèche sous vide. On obtient ainsi 98 mg de produit attendu sous forme de cristaux blancs.

### RMN dans DMSO

| | |
|---|---|
| 1,23 (d) | CH₃-CH-CH₃ |
| 3,19 (m) | CH₃-CH-CH₃ |
| 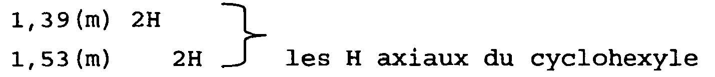 | |
| 2,06 | les H équatoriaux du cyclohexyle |
| | et les CH₂ du cyclohexyle |
| 1,71 et 1,90 | CH₂-CH₂-CH-N |
| 2,00 et 2,17 | CH₂-CH₂-CH-N |
| 4,76 (m) | CH₂-CH₂-CH-N |
| 3,05 (masqué) | H₄ axial |
| 3,73 (masqué) | H₁ axial |
| 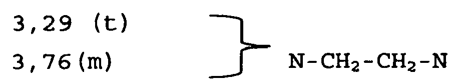 | |
| 6,94 | un des NH-CH₂ |
| 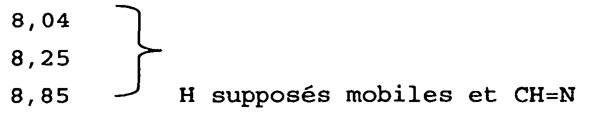 | |

### EXEMPLE 56 : Dichlorhydrate de trans-2-[[[2-[[2-[(4-aminocyclohexyl)-amino]-9-cyclopentyl-9H-purin-6-yl]-amino]-éthyl]-amino]-sulfonyl]-benzoate d'éthyle.

On procède comme au stade 1 de l'exemple 45 en utilisant à la place de 2-(chlorosulfonyl)-benzoate de méthyle, le O-chlorosulfonyle-benzoate d'éthyle. A partir du produit ainsi obtenu, on procède comme au stade 2 de l'exemple 45 et obtient ainsi le produit attendu.

### EXEMPLE 57 : Dichlorhydrate de trans-N-[2-[(4-aminocyclohexyl)-amino]-9-cyclopentyl-9H-purin-6-yl]-benzamide.

On procède comme au stade 1 de l'exemple 8 en utilisant à la place du benzènesulfonamide, le benzamide. A partir du produit ainsi obtenu, on procède comme au stade 2 de l'exemple 8 et on obtient ainsi le produit attendu.

### EXEMPLE 58 : Dichlorhydrate de l'acide trans-3-[[2-[(4-aminocyclohexyl)-aminol-9-cyclopentyl-9H-purin-6-yl]-amino]-benzoïque.

### Stade 1 : 3-[(2-chloro-9-cyclopentyl-9H-purin-6-yl) amino]-benzoate de méthyle.

On procède comme au stade 1 de l'exemple 6 en introduisant à température ambiante 257 mg du produit obtenu au stade 1 de l'exemple 1, 4 ml de butanol et 181 mg de méthyl-3-aminobenzoate et chauffe à une température d'environ 100°C pendant environ 5 heures. On évapore, empâte à l'éther puis sèche. On obtient ainsi 352 mg de produit attendu sous forme de cristaux blanc cassé.

### Spectre IR NUJOL

>=0 1722 cm-1
C=C + C=N + aromatique
1648; 1619; 1600; 1582; 1558; 1528; 1500cm-1

### RMN dans DMSO

| | |
|---|---|
| 1,72 (m) | |
| 1,89 (m) | |
| 2,01 (m) | |
| 2,19 (m) | les CH₂ cyclique du cyclopentyle |
| 3,88 (s) | -CO₂CH₃ |
| 4,87 (m) 1H | -N-CH (CH du cyclopentyle) |
| 8,52 (s) 1H | -CH=N |
| 7,51 (t) | H₅ |
| 7,68 (d) | H₆ |
| 8,13 (dl) | H₄ |
| 8,56 (sl) | H₂ |
| 10,58 (s) | NH |

### Stade 2 : Dichlorhydrate de l'acide trans-3-[[2-[(4-aminocyclohexyl)-amino]-9-cyclopentyl-9H-purin-6-yl]-amino]-benzoique.

On procède comme au stade 2 de l'exemple 6 et porte 400 mg de trans-1,4-diaminocyclohexane à une température d'environ 150°C et ajoute 260 mg du produit obtenu au stade 1 ci-dessus : on laisse sous agitation pendant 5 heures puis laisse une nuit à température ambiante. On ajoute alors 5 ml d'eau, extrait par 40 ml d'acétate d'éthyle, ajoute 10 ml de méthanol, sèche, filtre et évapore à sec. On reprend dans 4 ml d'HCl à 1,4N dans l'éthanol et 20 ml de méthanol, évapore à sec, puis empâte dans 5 ml d'éther et sèche à une température d'environ 60°C. On obtient ainsi 73 mg de produit attendu sous forme de solide beige.

### RMN dans DMSO

| | |
|---|---|
| 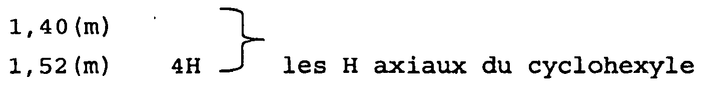 | |
| 1, 71 (m) 2H | |
| 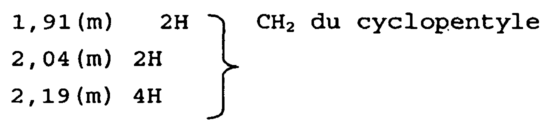 | |
| 2,04 (m) 4H | H équatoriaux du cyclohexyle |
| 2,98 (sl) 1H | H₄ axial |
| 3,77 (tl) 1H | H₁ axial |
| 4,82 (m) 1H | CH cyclopentyle |
| 7,50 (t) 1H | H₄' |
| 7,67 (d) 1H | H₃' |
| 8,06 (sl) <3H | NH₂ + N=CH-N |
| 8,13 | |
| 8,26 | H₁', H₅' |
| 8,44-8,83-10,58 | H supposés mobiles |

### EXEMPLE 59 : Dichlorhydrate de trans-3-[[2-[(4-aminocyclohexyl)-amino]-9-cyclopentyl-9H-purin-6-yl]amino]-benzoate d'éthyle

### Stade 1 : 3-[(2-chloro-9-cyclopentyl-9H-purin-6-yl) amino]-benzoate d'éthyle

On introduit à température ambiante 257 mg du produit obtenu au stade 1 de l'exemple 1, 4 ml de n-butanol et 200 mg de éthyl-3-aminobenzoate et plonge dans un bain à la température d'environ 100°C pendant 6 heures et demie sous agitation puis 16 heures à température ambiante, laisse revenir à température ambiante. On essore et rince à l'éther puis sèche sous pression réduite. On obtient ainsi 364 mg de produit attendu.

### Stade 2 : Dichlorhydrate de trans-3-[[2-[(4-aminocyclohexyl)-amino]-9-cyclopentyl-9H-purin-6-yl]amino]-benzoate d'ethyle

On porte 400 mg de trans-1,4-diaminocyclohexane à environ 140°C puis ajoute 270 mg du produit obtenu au stade 1 ci-dessus, laisse sous agitation pendant 5 heures puis laisse revenir à température ambiante. On ajoute alors 15 ml d'eau, extrait au dichlorométhane, lave à l'eau, sèche, évapore les solvants, chromatographie le résidu sur silice avec pour éluant méthanol/ammoniaque (98/2) reprend le résidu dans l'éthanol. On le salifie à l'aide d'une solution de HCl dans EtOH 1,4N et sèche à une température d'environ 60°C le chlorhydrate attendu. On obtient ainsi 156 mg de produit attendu.

### RMN dans DMSO

| | |
|---|---|
| 1,35 (t) 3H, 4, 35 (q) 2H | CO₂-CH₂-CH₃ |
| 1,43 (m) 4H | CH₂ axiaux du cyclohexyle |
| 1,71 (m) -1,91 (m) -2,06 (masqué) -2,20 (m) | CH₂ du cyclopentyle |
| 2,07 (m) H | équatoriaux du cyclohexyle |
| 3,00 (s) 1H, H4' | axial |
| 3,70 (t) 1H, H1' | axial |
| 4,84 (m) 1H | CH du cyclopentyle |
| 7,53 (t) 1H, | H₅ |
| 7,68 (dt) 1H, | H₄ |
| 8,09 (sl) <3H | N=CH + H mobiles |
| 8,32 (t) 1H, | H₂ |
| 8,37(d) 1H, | H₆ |
| 8,89-10,71 | H mobiles |

### EXEMPLE 60 : Dichlorhydrate de trans-4-((2-((4-aminocyclohexyl)amino)-9-cyclopentyl-9H-purin-6-yl)amino)-benzamide

### Stade 1 : 4-[(2-chloro-9-cyclopentyl-9H-purin-6-yl) amino]-benzamide.

On procède comme au stade 1 de l'exemple 6 en introduisant à température ambiante 193 mg du produit obtenu au stade 1 de l'exemple 1, 2 ml de butanol et 68 mg de 4-aminobenzamide et agite à une température d'environ 100°C pendant environ 20 heures. On évapore, empâte à l'éther puis sèche. On obtient ainsi 170 mg de produit attendu sous forme de cristaux.

### Spectre IR NUJOL

> = 0 1653 cm-1
système conjugué + aromatique
1616;1605;1555;1517;1491cm-1

### RMN dans DMSO

| | |
|---|---|
| 1,73 (m) 2H | |
| 1,90 (m) 2H | |
| 2,02 (m) 2H | les CH₂ du cyclopentyle |
| 2,19 (m) 2H | |
| 4,87 (m) 1H | CH du cyclopentyle |
| 8,43 (s) 1H | N=CH-N- |
| 10,33 (s) 1H | |
| 7,22 (sl) | H supposés mobiles |
| 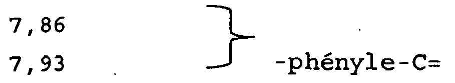 | |

### Stade 2 : Dichlorhydrate de trans-4-((2-((4-aminocyclohexyl)-amino)-9-cyclopentyl-9H-purin-6-yl)amino)-benzamide.

On procède comme au stade 2 de l'exemple 6 et introduit à température ambiante sous agitation 800 mg de trans-1,4-diaminocyclohexane puis chauffe à environ 150°C jusqu'à fusion et ajoute 249 mg du produit obtenu au stade 1 ci-dessus. On laisse sous agitation pendant 4 heures 30 minutes puis laisse revenir à température ambiante. On ajoute alors 10 ml d'eau, extrait par 10 ml de chlorure de méthylène contenant 25 % de méthanol, sèche, filtre et évapore à sec. On reprend dans 5 ml d'éthanol, ajoute 2 ml d'HCl à 1,4N dans l'éthanol puis évapore. On laisse cristalliser, essore et lave à l'éthanol puis sèche à une température d'environ 60°C. On obtient ainsi 207 mg de produit attendu sous forme de cristaux incolore.

### RMN dans DMSO

| | |
|---|---|
| 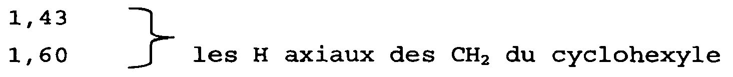 | |
| 2,08 | les H équatoriaux des CH₂ du cyclohexyle |
| 1,75 (m) 2H | |
| 1,95 (m) 2H | |
| 2,05 (m) 2H | les CH₂ du cyclopentyle |
| 2, 20 (m) 2H | |
| 3,04 (m) | H₄' axial |
| 3,76 (tt) | H₁' axial |
| 4,84 (m) | H angulaire du cyclopentyle |
| 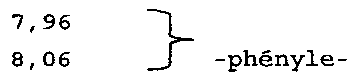 | |
| 8,10 | H mobiles et H₂ |
| 8,88 (s), 10,65 (s), 7,3 | H mobiles |

### EXEMPLE 61 : Dichlorhydrate de trans-3-[[[2-[(4-aminocyclohexyl)-amino]-9-cyclopentyl-9H-purin-6-yl] amino]méthyl]-benzamide.

On procède comme à l'exemple 6 en utilisant au stade 1 de l'exemple 6 à la place de l'éthyl-4-aminobenzoate, le 3 méthylaminobenzamide. En procédant ensuite comme au stade 2 de l'exemple 6, on obtient ainsi le produit attendu.

### EXEMPLE 62 : Dichlorhydrate de trans-3-[[[2-[(4-aminocyclohexyl)-amino]-9-cyclopentyl-9H-purin-6-yl]amino] méthyl]-benzoate d'éthyle.

On procède comme à l'exemple 1 en utilisant au stade 2 de l'exemple 1 à la place du chlorhydrate de 4-(aminométhyl)-benzoate de méthyle, le chlorhydrate de 3-(aminométhyl)-benzoate de méthyle.

### EXEMPLE 63 : Dichlorhydrate de trans-N-(2-(([4-aminocyclohexyl)-amino)-9-cyclopentyl-9H-purin-6-yl)-3-pyridine-sulfonamide.

### Stade 1 : N-[[2-chloro-9-cyclopentyl-9H-purin-6-yl] amino]-3-pyridinesulfonamide

On procède comme au stade 1 de l'exemple 8 à partir de 514 mg du produit obtenu au stade 1 de l'exemple 1, 8 ml de diméthoxyéthane (DME), 780 mg de carbonate de césium (CS₂CO₃) et 316 mg de 3-pyridinesulfonamide à la place du benzènesulfonamide et agite à une température d'environ 100°C pendant environ 5 heures 30 minutes et laisse une nuit à température ambiante. On ajoute alors 4 ml d'acide chlorhydrique 2N, essore le précipité formé, rince avec 5 ml d'eau et sèche sous vide à une température d'environ 50°C. On obtient ainsi 414 mg de produit attendu sous forme de cristaux beige.

### Stade 2 : Dichlorhydrate de trans-N-(2-(([4-aminocyclohexyl)amino)-9-cyclopentyl-9H-purin-6-yl)-3-pyridine-sulfonamide.

On procède comme au stade 2 de l'exemple 8 et porte 400 mg de trans-1,4-diaminocyclohexane, à une température d'environ 140°C, ajoute 265 mg du produit obtenu au stade 1 ci-dessus et maintient à cette température pendant environ 3 heures 30 minutes. On chromatographie sur silice avec pour éluant méthanol/ammoniaque en proportion de 98/2, ajoute alors 4 ml d'éthanol et 2 ml de méthanol puis 4 ml d'acide chlorhydrique/éthanol 1,4N, filtre le léger insoluble puis évapore à sec, empâte dans 10 ml d'éther et sèche sous vide à une température d'environ 60°C. On obtient ainsi 148 mg de produit attendu sous forme de cristaux brun.

### RMN dans DMSO

| | |
|---|---|
| 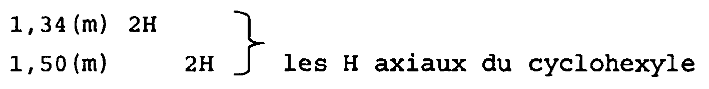 | |
| 1,68 (m) 2H | |
| 1,88 (m) 2H | les CH₂ du cyclopentyle |
| 2,05 (masqué) | |
| 2,06 | les H équatoriaux du cyclohexyle |
| 3,03 (sl) 1H | H₄ axial |
| 3,59 (tl) 1H | H₁ axial |
| 4,72 (m) 1H | CH du cyclopentyle |
| 7,62 (dd) | H₅' |
| 8,33 (dt) 1H | H₄' |
| 8,77 (dd) 1H | H₆' |
| 9,12 (d) | H₂' |
| 8,13 (sl) <3H | N=CH-N + NH₂ |
| 8,22 (s) 1H | H supposé mobile |

### EXEMPLE 64 : Dichlorhydrate de trans-4-[[2-[(4-aminocyclohexyl)amino]-9-cyclopentyl-9H-purin-6-yl]amino]-butanoate d'éthyle.

On procède comme à l'exemple 1 en utilisant au stade 2 de l'exemple 1 à la place du chlorhydrate de 4-(amino-méthyl)-benzoate de méthyle, le ∝-aminobutyrate d'éthyle puis on procède comme au stade 3 de l'exemple 1 à partir du produit ainsi obtenu.

### EXEMPLE 65 : Dichlorhydrate de trans-4-[[9-cyclopentyl-2-[[4-[[(1,1-diméthyléthoxy)carbonyl]aminolcyclohexyl] méthylamino]-9H-purin-6-yl]amino]-benzoate d'éthyle.

On procède comme au stade 2 de l'exemple 6 en utilisant à la place du trans 1-4 diaminocyclohexane, le trans 1-(NBOC)-4-(N-méthyl)cyclohexane. On obtient ainsi le produit attendu.

### EXEMPLE 66 : Dichlorhydrate de trans-4-[[2-[(4-aminocyclohexyl)méthylamino]-9-cyclopentyl-9H-purin-6-yl]amino]-benzoate d'éthyle.

On procède à partir du produit de l'exemple 65 en déprotégeant l'amine N-BOC par l'action d'acide trifluoroacétique. On obtient ainsi le produit attendu.

### EXEMPLE 67 : Dichlorhydrate de trans-9-cyclopentyl-N2-(4-hydroxy-4-méthylcyclohexyl)-N6-(phénylmethyl)-9H-purine-2,6-diamine.

On procède comme à l'exemple 1 en utilisant au stade 2 de l'exemple 1 à la place du chlorhydrate de 4-(amino-méthyl)-benzoate de méthyle, la benzylamine et au stade 3 de l'exemple 1, le 1-méthyl-trans-1,4 aminocyclohexanol à la place du trans 1-4 diaminocyclohexane.

### EXEMPLE 68 : Dichlorhydrate de N2-(4-amino-2-hydroxy-cyclohexyl)-9-cyclopentyl-N6-(phénylméthyl)-9H-purine-2,6-diamine.

On procède comme à l'exemple 1 en utilisant au stade 2 de l'exemple 1 à la place du chlorhydrate de 4-(amino-méthyl)-benzoate de méthyle, la benzylamine et au stade 3 de l'exemple 1 le 2-hydroxy-trans-1,4 diaminocyclohexane à la place du trans 1-4 diaminocyclohexane.

### EXEMPLE 69 : Dichlorhydrate de N2-(4-amino-3-hydroxy-cyclohexyl)-9-cyclopentyl-N6-(phénylméthyl)-9H-purine-2,6-diamine.

On procède comme à l'exemple 1 en utilisant au stade 2 de l'exemple 1 à la place du chlorhydrate de 4-(amino-méthyl)-benzoate de méthyle, la benzylamine et au stade 3 de l'exemple 1, le 3-hydroxy-trans-1,4 diaminocyclohexane à la place du trans 1-4 diaminocyclohexane.

### EXEMPLE 70 : Dichlorhydrate de trans-N2-(4-amino-3-fluorocyclohexyl)-9-cyclopentyl-N6-(phénylméthyl)-9H-purine-2,6-diamine.

On procède comme à l'exemple 1 en utilisant au stade 2 de l'exemple 1 à la place du chlorhydrate de 4-(amino-méthyl)-benzoate de méthyle, la benzylamine et au stade 3 de l'exemple 1, le 3-fluoro-trans-1,4 diaminocyclohexane à la place du trans 1-4 diaminocyclohexane.

### EXEMPLE 71 : Dichlorhydrate de trans-2-[(4-aminocyclohexyl)oxy]-9-cyclopentyl-N-(phénylméthyl)-9H-purin-6-amine.

On procède comme à l'exemple 1 en utilisant au stade 2 de l'exemple 1 à la place du chlorhydrate de 4-(amino-méthyl)-benzoate de méthyle, la benzylamine et au stade 3 de l'exemple 1, à la place du trans 1-4 diaminocyclohexane, le trans-1,4 Bocaminocyclohexanol en présence d'hydrure de sodium (NaH)dans le dimethylformamide.

### EXEMPLE 72 : Dichlorhydrate de 4-[[9-cyclopentyl-6-[(phényl-méthyl)amino]-9H-purin-2-yl]amino]-cyclohexanone.

On procède à partir du produit de l'exemple 4 par oxydation dans le DMF en présence de (PDC) pyridinium dichromate et on obtient ainsi le produit attendu après filtration sur silice avec pour éluant CH₂Cl₂/méthanol : 80/20.

### EXEMPLE 73 : Dichlorhydrate de O-méthyloxime de 4-[[9-cyclopentyl-6-[(phénylméthyl)amino]-9H-purin-2-yl]amino]-cyclohexanone.

On procède à partir du produit de l'exemple 72 par réaction avec la O-méthylhydroxylamine dans l'éthanol au reflux et on obtient ainsi le produit attendu.

### EXEMPLE 74 : Trichlorhydrate de trans-N2-(4-aminocyclohexyl)-9-cyclopentyl-N6-[2-[[(3,4-dichlorophényl)-méthyl]-amino]-éthyl]-9H-purine-2,6-diamine.

### Stade 1 : 2-chloro-9-cyclopentyl-N-[2-[[(3,4-dichlorophényl)-méthyl]-amino]-éthyl]-9H-purin-6-amine.

On mélange 281 mg du produit obtenu au stade 1 de l'exemple 7, 4 ml de méthanol, 0,2 ml de 3,4-dichlorobenzaldéhyde, 0,2 ml d'acide acétique, agite 2 heures et demie, ajoute 0,1 g de NaBH₃CN et agite à température ambiante pendant 1 heure. On évapore le solvant, chromatographie le résidu sur silice avec pour éluant CH₂Cl₂/méthanol/ammoniaque (95/0, 5/0, 33) et obtient 278,4 mg de produit attendu.

### Stade 2 : Trichlorhydrate de trans-N2-(4-aminocyclohexyl)-9-cyclopentyl-N6-[2-[[(3,4-dichlorophényl)-méthyl]-amino]-éthyl]-9H-purine-2,6-diamine.

On mélange 278 mg du produit obtenu au stade 1 ci-dessus, 4 ml de butanol, 360 mg de trans-1,4-diaminocyclohexane, chauffe à 140°C pendant environ 10 heures, laisse revenir à température ambiante. On évapore, chromatographie le résidu sur silice avec pour éluant CH₂Cl₂/méthanol/ammoniaque (85/15/1,5) et obtient 93 mg de produit qui est salifié par une solution de HC1 dans EtOH 1,4N. On obtient 115 mg de produit attendu.

### RMN dans DMSO

| | |
|---|---|
| 1,36 (m) -1,49 (m) | les H axiaux du cyclohexyle |
| 1,71 (m) -1,90 (m) -2,04 (m) | les CH₂ du cyclopentyle |
| 2,05 (m) | les H équatoriaux du cyclohexyle |
| 3,02 (sl) 1H | H₄ |
| 3,25 (t) 2H-3,90 (sl) 2H | les CH₂-N |
| 3,69 (supposé masqué) | H₁ axial |
| 4,24 (sl) 2H | N-CH₂- phényle |
| 4,73 (m) 2H | CH du cyclopentyle |
| 7,56 (dd) 1H | H_{c} |
| 7,67 (d) 1H | H_{b} |
| 7,87 (d) 1H | Hₐ |
| 7,93 (sl) 3H | NH |
| 9,40 >1H | |

### EXEMPLE 75 : Trichlorhydrate de trans-N2-(4-aminocyclohexyl)-9-cyclopentyl-N6-[2-[[[4-(trifluorométhoxy)-phényl]-méthyl]-amino]-éthyl]-9H-purine-2,6-diamine.

### Stade 1 : 2-chloro-9-cyclopentyl-N-[2-[[[4-(trifluorométhoxy)-phényl]-méthyl]-amino]-éthyl]-9H-purin-6-amine.

On mélange 281 mg du produit obtenu au stade 1 de l'exemple 7, 4 ml de méthanol, 0,16 ml de 4-trifluorométhoxybenzaldéhyde, 0,2 ml d'acide acétique, agite 2 heures et demie, ajoute 0,1 g de NaBH₃CN et agite à température ambiante pendant 1 heure. On évapore le solvant, chromatographie le résidu sur silice avec pour éluant CH₂Cl₂/méthanol/ammoniaque (95/0,5/0,33) et obtient 129 mg de produit attendu.

### Stade 2 : Trichlorhydrate de trans-N2-(4-aminocyclohexyl)-9-cyclopentyl-N6-[2-[[[4-(trifluorométhoxy)-phényl]-méthyl]-amino]-éthyl]-9H-purine-2,6-diamine.

On mélange 129 mg du produit obtenu au stade 1 ci-dessus, 4 ml de butanol, 170 mg de trans-1,4-diaminocyclohexane, chauffe à 140°C pendant environ 5 heures et demie, laisse revenir à température ambiante. On évapore, chromatographie le résidu sur silice avec pour éluant CH₂Cl₂/méthanol/ammoniaque (85/15/1,5) et obtient 90 mg de produit qui est salifié par une solution de HCl dans EtOH 1,4N. On obtient 65 mg de produit attendu.

### RMN dans DMSO

| | |
|---|---|
| 1,39 (m) -1,53 (m) 4H | les H axiaux du cyclohexyle |
| 1,72 (m) -1,89 (m) -2,17 (m) | les CH₂ du cyclopentyle |
| 2,06 (m) 4H | les H équatoriaux du cyclohexyle |
| 3,03 (sl) 1H | H₄ |
| 3,17 (t) 4H-4, 00 (sl) 4H | les CH₂-N |
| 3,73 (masqué) 1H | H₁ axial |
| 4,27 (s) 2H | N-CH₂- phényle |
| 4,76 (m) 1H | CH du cyclopentyle |
| 7,38-7,75 AA'BB' | F₃C-O-Phényle-CH₂ |
| 8,20 (s) 1H | N=CH |
| 8,09 ≤ 2H | NH₂ |
| 8,05-9,62 | les NH |

### EXEMPLE 76 : Trichlorhydrate de trans-N2-(4-aminocyclohexyl)-9-cyclopentyl-N6-[2-[[(3,5-dichlorophényl)-méthyl]-amino]-éthyl]-9H-purine-2,6-diamine.

### Stade 1 : 2-chloro-9-cyclopentyl-N-[2-[[(3,5-dichlorophényl)-méthyl]-amino]-éthyl]-9H-purin-6-amine.

On mélange 281 mg du produit obtenu au stade 1 de l'exemple 7, 4 ml de méthanol, 0,16 ml 3,5-dichlorobenzaldéhyde, 0,2 ml d'acide acétique, agite 2 heures et demie, ajoute 0,1 g de NaBH₃CN et agite à température ambiante pendant 1 heure. On évapore le solvant, chromatographie le résidu sur silice avec pour éluant CH₂Cl₂/méthanol/ammoniaque (95/0,5/0,33) et obtient 119 mg de produit attendu.

### Stade 2 : trichlorhydrate de trans-N2-(4-aminocyclohexyl)-9-cyclopentyl-N6-[2-[[(3,5-dichlorophényl)-méthyl]-amino]-éthyl]-9H-purine-2,6-diamine.

On mélange 119 mg du produit obtenu au stade 1 ci-dessus, 4 ml de butanol, 155 mg de trans-1,4-diaminocyclohexane, chauffe à 140°C pendant environ 5 heures, ajoute de nouveau 155 mg de trans-1,4-diaminocyclohexane, chauffe de nouveau à 140°C pendant 3 heures laisse revenir à température ambiante. On évapore, chromatographie le résidu sur silice avec pour éluant CH₂Cl₂/méthanol/ammoniaque (85/15/1,5) et obtient 17,3 mg de produit qui est salifié par une solution de HCl dans EtOH 1,4N. On obtient 23,8 mg de produit attendu.

### RMN dans DMSO

| | |
|---|---|
| 1,38 (m) -1,51 (m) 4H | les H axiaux du cyclohexyle |
| 1,71 (m) -1,91 (m) -2,16 (m) | les CH₂ du cyclopentyle |
| 2,06 (m) | les H équatoriaux du cyclohexyle |
| 3, 03 (sl) 1H | H₄ |
| 3,27 (tl) -3,97 (sl) | les CH₂-N |
| 3,72 (masqué) | H₁ |
| 4,26 (s) 2H | N-CH₂- phényle |
| 4,76 (m) 1H | CH du cyclopentyle |
| 7,59 (t) 1H | Hₐ |
| 7,70 (d) 2H | les H_{b} |
| 8,03 (sl) -8,40 (sl) -9,60 (sl) | les NH |
| 8,16 (s) | N=CH |

### EXEMPLE 77 : Trichlorhydrate de trans-N2-(4-aminocyclohexyl)-9-cyclopentyl-N6-[2-[[(4-fluorophényl)-méthyl]-amino]-éthyl]-9H-purine-2,6-diamine.

### Stade 1 : 2-chloro-9-cyclopentyl-N-[2-[[(4-fluorophényl)-méthyl]-amino]-éthyl]-9H-purin-6-amine.

On mélange 281 mg du produit obtenu au stade 1 de l'exemple 7, 4 ml de méthanol, 0,15 ml de 4-fluorométhoxybenzaldéhyde, 0,2 ml d'acide acétique, agite 2 heures et demie, ajoute 0,1 g de NaBH₃CN et agite à température ambiante pendant 1 heure. On évapore le solvant, chromatographie le résidu sur silice avec pour éluant CH₂Cl₂/méthanol/ammoniaque (95/0,5/0,33) et obtient 230 mg de produit attendu.

### Stade 2 : trichlorhydrate de trans-N2-(4-aminocyclohexyl)-9-cyclopentyl-N6-[2-[[(4-fluorophényl)-méthyl]-amino]-éthyl]-9H-purine-2,6-diamine.

On mélange 200 mg du produit obtenu au stade 1 ci-dessus, 4 ml de butanol, 400 mg de trans-1,4-diaminocyclohexane, chauffe à 140°C pendant environ 16 heures, laisse revenir à température ambiante. On évapore, chromatographie le résidu sur silice avec pour éluant CH₂Cl_{2/}méthanol/ammoniaque (85/15/1, 5) et obtient 54 mg de produit qui est salifié par une solution de HCl dans EtOH 1,4N. On obtient 57,7 mg de produit attendu.

### RMN dans DMSO

| | |
|---|---|
| 1,39 (m) -1,53 (m) 4H | les H axiaux du cyclohexyle |
| 1,71 (m) -1,90 (m) -2,17 (m) | les CH₂ du cyclopentyle |
| 2,05 (m) 4H | les H équatoriaux du cyclohexyle |
| 3,02 (sl) 1H | H₄ |
| 3,25 (tl) 2H-3,99 (sl) 2H | les CH₂-N |
| 3,74 1H | H₁ |
| 4,22 (s) 2H | N-CH₂- phényle |
| 4,77 (m) 1H | CH du cyclopentyle |
| 7,22 (tl) 2H | les H_{b} |
| 7,66 (dd) 2H | les Hₐ |
| 8,09 (sl) > 2H | NH |
| 8,22 (s) 1H | N=CH |
| 8,60 (sl) 1H-9,54 (sl) > 1H | NH |

### EXEMPLE 78 : Trichlorhydrate de trans-N2-(4-aminocyclohexyl)-9-cyclopentyl-N6-[2-[[[4-(trifluorométhyl)-phényl]-méthyl]-amino]-éthyl]-9H-purine-2,6-diamine.

### Stade 1 : 2-chloro-9-cyclopentyl-N-[2-[[[4-(trifluorométhyl)-phényl]-méthyl]-amino]-éthyl]-9H-purin-6-amine.

On mélange 281 mg du produit obtenu au stade 1 de l'exemple 7, 4 ml de méthanol, 0,19 ml de 4-trifluorométhylbenzaldéhyde, 0,2 ml d'acide acétique, agite 2 heures et demie, ajoute 0,1 g de NaBH₃CN et agite à température ambiante pendant 1 heure. On évapore le solvant, chromatographie le résidu sur silice avec pour éluant CH₂Cl₂ /méthanol /ammoniaque (95/0,5/0,33) et obtient 146 mg de produit attendu.

### Stade 2 : trichlorhydrate de trans-N2-(4-aminocyclohexyl)-9-cyclopentyl-N6-[2-[[[4-(trifluorométhyl)-phényl]-méthyl]-amino]-éthyl]-9H-purine-2,6-diamine.

On mélange 220 mg du produit obtenu comme au stade 1 ci-dessus, 4 ml de butanol, 571 mg de trans-1,4-diaminocyclohexane, chauffe à 140°C pendant environ 5 heures et demie, laisse revenir à température ambiante. On évapore, chromatographie le résidu sur silice avec pour éluant CH₂Cl₂/méthanol/ammoniaque (85/15/1,5) et salifie le produit obtenu par une solution de HCl dans EtOH 1,4N. On obtient 66 mg de produit attendu.

### RMN dans DMSO

| | |
|---|---|
| 1,39-1,53 | les H axiaux du cyclohexyle |
| 1,70-1,90-2,02-2,16 | les CH₂ du cyclopentyle |
| 2,05 | les H équatoriaux du cyclohexyle |
| 2,84-3,04 | H₄ |
| 3,29-4,00 | les CH₂-N |
| 3,55-3,73 | H₁ axial |
| 4,34 (s) | N-CH₂- phényle |
| 4,76 | CH du cyclopentyle |
| 7,76-7,85 | F₃C-Phényle |
| 8,20 | N=CH |
| 8,07-8,43-8,59 | H mobiles |

### EXEMPLE 79 : Trichlorhydrate de trans-N2-(4-aminocyclohexyl)-9-cyclopentyl-N6-[2-[[(3-chlorophényl)-méthyl]-amino]-éthyl]-9H-purine-2,6-diamine.

### Stade 1 : 2-chloro-N-[2-[[(3-chlorophényl)-méthyl]-amino]-éthyl]-9-cyclopentyl-9H-purin-6-amine.

On mélange 281 mg du produit obtenu au stade 1 de l'exemple 7, 4 ml de méthanol, 0,2 ml de 3-chlorobenzaldéhyde, 0,2 ml d'acide acétique, agite 2 heures et demie, ajoute 0,1 g de NaBH₃CN et agite à température ambiante pendant 1 heure. On évapore le solvant, chromatographie le résidu sur silice avec pour éluant CH₂Cl₂/méthanol/ammoniaque (95/0,5/0,33) et obtient 172 mg de produit attendu.

### Stade 2 : trichlorhydrate de trans-N2-(4-aminocyclohexyl)-9-cyclopentyl-N6-[2-[[(3-chlorophényl)-méthyl]-amino]-éthyl]-9H-purine-2,6-diamine.

On mélange 111 mg du produit obtenu au stade 1 ci-dessus, 4 ml de butanol, 312 mg de trans-1,4-diaminocyclohexane, chauffe à 140°C pendant environ 5 heures et demie, laisse revenir à température ambiante. On évapore, chromatographie le résidu sur silice avec pour éluant CH₂Cl₂/méthanol/ammoniaque (85/15/1,5) et obtient 92,7 mg de produit qui est salifié par une solution de HCl dans EtOH 1,4N. On obtient 68 mg de produit attendu.

### RMN dans DMSO

| | |
|---|---|
| 1,38 (m) -1,52 (m) 4H | les H axiaux du cyclohexyle |
| 1,71 (m) - 1,90 (m) - 2,15 (m) | les CH₂ du cyclopentyle |
| 2,05 (m) 4H | les H équatoriaux du cyclohexyle |
| 3,04 (sl) 1H | H₄ |
| 3,26 (tl) - 3,98 (sl) | les CH₂-N |
| 3,72 (masqué) | H₁ axial |
| 4,25 (s) 2H | CH₂- phényle |
| 4,76 (m) 1H | CH du cyclopentyle |
| 7,45 (m) 2H et 7,55 (m) 1H | H_{b}, H_{c} et H_{d} |
| 7,70 (sl) 1H | Hₐ |
| 8,04 (sl) > 2H-8,38 (sl) 1H-9,53 (sl) > 1H | NH |
| 8,15 (s) 1H | CH=N |

### EXEMPLE 80 : Dichlorhydrate de trans-N2-(4-aminocyclohexyl)-N6-[(1,1'-biphényl)-4-yl]-9-cyclopentyl-9H-purin-2,6-diamine.

### Stade 1 : 2-chloro-N-[(1,1'-biphényl)-4-yl]-9-cyclopentyl-9H-purin-6-amine.

On introduit à température ambiante 257 mg du produit obtenu au stade 1 de l'exemple 1 dans 4 ml de n-butanol et 203 mg de 4-aminobiphényle et agite pendant 5 heures et demie dans un bain à 100°C puis laisse revenir à température ambiante. On essore et rince à l'éther puis sèche sous pression réduite. On obtient ainsi 328 mg de produit attendu.

### Stade 2 : dichlorhydrate de trans-N2-(4-aminocyclohexyl)-N6-[(1,1'-biphényl)-4-yl]-9-cyclopentyl-9H-purin-2,6-diamine.

On porte 342 mg de trans-1,4-diaminocyclohexane à environ 150°C puis ajoute 234 mg du produit obtenu au stade 1 ci-dessus, maintient sous agitation à 140°C pendant 9 heures 30 minutes puis laisse revenir à température ambiante. On ajoute alors 10 ml d'eau, extrait au dichlorométhane, sèche, évapore le solvant, chromatographie le résidu sur silice (éluant : MeOH/NH₄OH 98/2), reprend dans 5 ml de solution éthanolique d'acide chlorhydrique évapore les solvants, empâte le résidu dans l'éther, sèche sous pression réduite à 60°C et récupère 93 mg de produit attendu.

### RMN dans DMSO

| | |
|---|---|
| 1,42 (m) -1,53 (m) | H axiaux du cyclohexyle |
| 1,72 (m) -1,91 (m) 4H | CH₂-CH du cyclopentyle |
| 2,09 (m) | H équatoriaux du cyclohexyle |
| 2,09 (m) -2,21 (m) 4H | CH₂-CH₂-CH du cyclopentyle |
| 3,03 (sl) | H₄ |
| 3,73 (tl) | H₁ |
| 4,84 (m) | CH du cyclopentyle |
| 7,34 (tt) 1H | H_{c} |
| 7,46 (tl) 2H | les H_{b} |
| 7,69 (dl) -8,07 (dl) 4H AA'BB' | les Hₐ + phényle |
| 8,07 3H | -NH |
| 8,93 (s) 1H | CH-C=N |
| 10,66 (sl) | NH |

### EXEMPLE 81 : Dichlorhydrate de trans-4-[[2-[(4-aminocyclohexyl)-amino]-9-cyclopentyl-9H-purin-6-yl]-amino]-benzèneacétonitrile.

### Stade 1 : 4-[(2-chloro-9-cyclopentyl-9H-purin-6-yl)-amino]-benzèneacétonitrile.

On introduit à température ambiante 257 mg du produit obtenu au stade 1 de l'exemple 1 dans 4 ml de n-butanol et 159 mg de 4-aminophénylacétonitrile et agite pendant 5 heures et demie dans un bain à 100°C puis laisse revenir à température ambiante. On essore et rince à l'éther puis sèche sous pression réduite. On obtient ainsi 296 mg de produit attendu.

### Stade 2 : dichlorhydrate de trans-4-[[2-[(4-aminocyclohexyl)-amino]-9-cyclopentyl-9H-purin-6-yl]-amino]-benzèneacétonitrile.

On porte 684 mg de trans-1,4-diaminocyclohexane à environ 140°C puis ajoute 211 mg du produit obtenu au stade 1 ci-dessus, maintient sous agitation à 140°C pendant 6 heures puis laisse revenir à température ambiante. On ajoute alors 10 ml d'eau, extrait au dichlorométhane, sèche, évapore le solvant, chromatographie le résidu sur silice (éluant : MeOH/NH₄OH 98/2), reprend dans 6 ml de solution éthanolique d'acide chlorhydrique évapore les solvants, empâte le résidu dans l'éther, sèche sous pression réduite à 60°C et récupère 199 mg de produit attendu.

### RMN dans DMSO

| | |
|---|---|
| 1,46 (m) | H axiaux du cyclohexyle |
| 1,72 (m) -1,91 (m) | |
| 2,08 (masqué) | les CH₂ du cyclopentyle |
| 2,21 (m) 8H | |
| 2,09 (m) | H équatoriaux du cyclohexyle |
| 3,02 (sl) | H₄ axial |
| 3,71 (tl) | H₁ axial |
| 3,98 (s) 2H | phényl-CH₂-CN |
| 4,84 | CH du cyclopentyle |
| 7,36-7,97 AA'BB' | phényle |
| 8,06 (sl) 3H | -NH |
| 8,81 (s) 1H | CH=N |
| 10,46 (sl) 1H | NH |

### EXEMPLE 82 : Dichlorhydrate de trans-N2-(4-aminocyclohexyl)-9-cyclopentyl-N6-[4-(4-morpholinyl)-phényl]-9H-purin-2,6-diamine.

### Stade 1 : 2-chloro-9-cyclopentyl-N-[4-(4-morpholinyl) phényl]-9H-purin-6-amine.

On introduit à température ambiante 257 mg du produit obtenu au stade 1 de l'exemple 1 dans 4 ml de n-butanol et 214 mg de 4-morpholinoaniline et agite pendant 24 heures à température ambiante. On essore et rince à l'éther puis sèche sous pression réduite à 50°C. On obtient ainsi 286 mg de produit attendu.

### Stade 2 : dichlorhydrate de trans-N2-(4-aminocyclohexyl)-9-cyclopentyl-N6-[4-(4-morpholinyl)-phényl]-9H-purin-2,6-diamine.

On porte 800 mg de trans-1,4-diaminocyclohexane à environ 140°C puis ajoute 286 mg du produit obtenu au stade 1 ci-dessus, maintient sous agitation à 140°C pendant 5 heures puis laisse revenir à température ambiante. On ajoute alors 15 ml d'eau, extrait au dichlorométhane, sèche, évapore le solvant, chromatographie le résidu sur silice (éluant : MeOH/NH₄OH 98/2), reprend dans 4 ml de solution éthanolique d'acide chlorhydrique évapore les solvants, sèche sous pression réduite à 50°C et récupère 143 mg de produit attendu.

### RMN dans DMSO

| | |
|---|---|
| 1,40 (m) -1,540 (m) | H axiaux du cyclohexyle |
| 1,72 (m) -1,91 (m) | |
| 2,07 (m) 8H | les CH₂ du cyclopentyle |
| 2,21 (m) | |
| 2,07 (m) 4H | H équatoriaux du cyclohexyle |
| 3,02 (sl) | H₄ axial |
| 3,20 (m) 4H | les CH₂-N |
| 3,71 (tt) 1H | H₁ axial |
| 3,81 (m) 4H | les CH₂-O |
| 4,83 (m) | CH du cyclopentyle |
| 7,10-7,77 AA'BB' 4H | phényle |
| 8,12 (sl) 3H-10,46 (sl) 1H | les NH et NH₂ |
| 8,78 (s) 1H | CH=N |

### EXEMPLE 83 : Dichlorhydrate de trans-4-[[2-[(4-aminocyclohexyl)-amino]-9-cyclopentyl-9H-purin-6-yl]-amino]-benzonitrile.

### Stade 1 : 4-[(2-chloro-9-cyclopentyl-9H-purin-6-yl) amino]-benzonitrile.

On introduit à température ambiante 1,03 g du produit obtenu au Stade 1 de l'exemple 1 dans 14 ml de n-butanol et 567 mg de 4-aminobenzonitrile et agite pendant 7 heures dans un bain à 100°C puis laisse 16 heures à température ambiante. On concentre partiellement, essore et rince à l'éther puis sèche sous pression réduite. On obtient ainsi 1,13 g de produit attendu.

### Stade 2 : dichlorhydrate de trans-4-[[2-[(4-aminocyclohexyl)-amino]-9-cyclopentyl-9H-purin-6-yl]amino]-benzonitrile.

On porte 2 g de trans-1,4-diaminocyclohexane à environ 150°C puis ajoute 1,18 g du produit obtenu au stade 1 ci-dessus, maintient sous agitation à 140°C pendant 6 heures puis laisse revenir à température ambiante. On ajoute alors 30 ml d'eau, extrait au dichlorométhane, sèche, évapore le solvant, chromatographie le résidu sur silice (éluant : MeOH/NH₄OH 98/2), prélève 130 mg du produit obtenu que l'on reprend dans 10 ml d'ethanol, ajoute 4 ml de solution éthanolique d'acide chlorhydrique, essore, lave à l'éther, sèche sous pression réduite à 50°C et récupère 144 mg de produit attendu.

### RMN dans DMSO

| | |
|---|---|
| 1,41 (m) -1,53 (m) | H axiaux du cyclohexyle |
| 1,72 (m) -1,91 (m) | |
| 2,03 (m) 8H | les CH₂ du cyclopentyle |
| 2,18 (m) | |
| 2,07 (m) 4H | H équatoriaux du cyclohexyle |
| 3,04 (sl) 1H | H₄ |
| 3,70 (tt) 1H | H₁ |
| 4,81 (m) 1H | CH du cyclopentyle |
| 7,76-8,23 4H AA'BB' | HN-phényle-CN |
| 8,03 (sl) 3H-10,55 (sl) cyclohexyle | xles NH |
| 8,60 (s) 1H | CH=N |

### EXEMPLE 84 : Dichlorhydrate de trans-N2-(4-aminocyclohexyl)-9-cyclopentyl-N6-(4-nitrophényl)-9H-purin-2,6-diamine.

### Stade 1 : 2-chloro-9-cyclopentyl-N-(4-nitrophényl)-9H-purin-6-amine.

On introduit à température ambiante 257 mg du produit obtenu au Stade 1 de l'exemple 1 dans 4 ml de n-butanol et 166 mg de 4-nitroaniline et agite pendant 5 heures et demi dans un bain à 100°C puis laisse revenir à température ambiante. On essore et rince à l'éther puis sèche sous pression réduite à 50°C. On obtient ainsi 275 mg de produit attendu.

### Stade 2 : dichlorhydrate de trans-N2-(4-aminocyclohexyl)-9-cyclopentyl-N6-(4-nitrophényl)-9H-purin-2,6-diamine.

On porte 740 mg de trans-1,4-diaminocyclohexane à environ 150°C puis ajoute 466 mg du produit obtenu au stade 1 ci-dessus, maintient sous agitation à 140°C pendant 6 heures puis laisse revenir à température ambiante. On ajoute alors 15 ml d'eau, extrait au dichlorométhane, sèche, évapore le solvant, chromatographie le résidu sur silice (éluant : MeOH/NH₄OH 98/2 prélève 270 mg du produit obtenu que l'on reprend dans 10 ml d'éthanol, ajoute 6 ml de solution éthanolique d'acide chlorhydrique essore, lave à l'éther, sèche sous pression réduite à 60°C et récupère 79 mg de produit attendu.

### RMN dans DMSO

| | |
|---|---|
| 1,42 (m) -1,53 (m) | H axiaux du cyclohexyle |
| 1, 73 (m) -1,92 (m) | |
| 2,10 (m) 8H | les CH₂ du cyclopentyle |
| 2,18 (m) | |
| 2,10 (m) 4H | H équatoriaux du cyclohexyle |
| 3,04 (sl) 1H | H₄ |
| 3,72 (tt) 1H | H₁ |
| 4,81 (m) 1H | CH du cyclopentyle |
| 8,04 (sl) 3H-10,64 (sl) 1H | NH et NH₂ |
| 8,20-8,30 AA'BB' | HN-phényle-NO₂ |
| 8,54 (s) 1H | CH=N |

### EXEMPLE 85 : Dichlorhydrate de trans-N2-(4-aminocyclohexyl)-9-cyclopentyl-N6-[4-(trifluorométhyl)-phényl]-9H-purin-2,6-diamine.

### Stade 1 : 2-chloro-9-cyclopentyl-N-[4-(trifluorométhyl)-phényl]-9H-purin-6-amine.

On introduit à température ambiante 257 mg du produit obtenu au Stade 1 de l'exemple 1 dans 4 ml de n-butanol et 193 mg de 4-trifluorométhylaniline et agite pendant 7 heures dans un bain à 100°C, abandonne à température ambiante, chauffe de nouveau 4 heures à 100°C puis laisse revenir à température ambiante. On évapore les solvants, empâte à l'éther puis sèche sous pression réduite à 50°C. On obtient ainsi 314 mg de produit attendu.

### Stade 2 : dichlorhydrate de trans-N2-(4-aminocyclohexyl)-9-cyclopentyl-N6-[4-(trifluorométhyl)-phényl]-9H-purin-2,6-diamine.

On porte 684 mg de trans-1,4-diaminocyclohexane à environ 150°C puis ajoute 229 mg du produit obtenu au stade 1 ci-dessus, maintient sous agitation à 140°C pendant 5 heures puis laisse revenir à température ambiante. On ajoute alors 15 ml d'eau, extrait au dichlorométhane, sèche, évapore le solvant, chromatographie le résidu sur silice (éluant : MeOH/NH₄OH 98/2), reprend dans 10 ml d'éthanol, ajoute 6 ml de solution éthanolique d'acide chlorhydrique, essore, lave à l'éther, sèche sous pression réduite à 60°C et récupère 159 mg de produit attendu.

### RMN dans DMSO

| | |
|---|---|
| 1,42 (m) -1,62 (m) | H axiaux du cyclohexyle |
| 1,72 (m) -1,91 (m) -2,19 (m) 8H | les CH₂ du cyclopentyle |
| 2,10 (m) 4H | H équatoriaux du cyclohexyle |
| 3,03 (sl) 1H | H₄ |
| 3,72 (tt) 1H | H₁ |
| 4,82 (m) 1H | CH du cyclopentyle |
| 7,66-8,23 | HN-phényle-CF₃ |
| 8,01 (sl) 3H-10,38 (sl) 1H | les NH et NH₂ |
| 8,57 (s) 1H | CH=N |

### EXEMPLE 86 : Dichlorhydrate de trans-N2-(4-aminocyclohexyl)-N6-(4-aminophényl)-9-cyclopentyl-9H-purin-2,6-diamine

On hydrogène pendant 16 heures à température ambiante, 455 mg de produit obtenu comme à l'exemple 84 dans 10 ml de tétrahydrofuranne en présence de 230 mg de palladium sur carbone actif. On filtre, rince au tétrahydrofuranne, évapore le solvant, chromatographie le résidu sur silice (éluant : MeOH/NH₄OH 98/2), reprend dans 10 ml d'éthanol, ajoute 4 ml de solution éthanolique d'acide chlorhydrique, essore, lave à l'éther, sèche sous pression réduite à 60°C et récupère 338 mg de produit attendu.

### RMN dans DMSO

| | |
|---|---|
| 1,40 (m) -1,53 (m) | H axiaux du cyclohexyle |
| 1,72 (m) -1,91 (m) | |
| 2,08 (masqué) 8H | les CH₂ du cyclopentyle |
| 2,21 (m) | |
| 2,07(1) 4H | H équatoriaux du cyclohexyle |
| 3,01 (sl) 1H | H₄ |
| 3,68 (tt) 1H | H₁ |
| 4,85 (qt) 1H | CH du cyclopentyle |
| 7,40 2H-7,99 2H AA'BB' | HN-phényle-NH₂ |
| 8,15 (sl) 3H | H mobiles + N=CH-N |
| 8,93-10,71 | H mobiles |

### EXEMPLE 87 : Chlorhydrate de trans-9-cyclopentyl-N2-(4-hydroxycyclohexyl)-N6-phényl-9H-purin-2,6-diamine.

### Stade 1 : 2-chloro-9-cyclopentyl-N-phényl-9H-purin-6-amine.

On introduit à température ambiante 2,57 g du produit obtenu au Stade 1 de l'exemple 1 dans 25 ml de n-butanol et 1,1 ml d'aniline, et chauffe à une température d'environ 90 à 100°C puis laisse revenir à température ambiante. On dilue avec 20 ml d'isopropanol, agite 15 minutes, essore et rince à l'isopropanol puis sèche sous pression réduite à 40°C. On obtient ainsi 2,43 g de produit attendu.

### Stade 2 : chlorhydrate de trans-9-cyclopentyl-N2-(4-hydroxycyclohexyl)-N6-phényl-9H-purin-2,6-diamine.

On mélange 575 mg de trans-1,4-aminocyclohexanol que l'on porte à une température de 50 à 60°C puis ajoute 313 mg du produit obtenu au stade 1 ci-dessus et agite à une température de 150 à 160°C pendant environ 20 heures. On laisse alors revenir à température ambiante, reprend avec de l'acétate d'éthyle et de l'eau et porte à une température d'environ 60°C. On laisse alors décanter, réextrait à l'acétate d'éthyle, lave avec une solution aqueuse saturée de chlorure de sodium, sèche et évapore à sec. On chromatographie le résidu sur silice (éluant : CH₂Cl₂/MeOH 95/5), reprend par une solution éthanolique d'acide chlorhydrique, laisse cristalliser, essore, sèche sous pression réduite et récupère 266 mg de produit attendu.

### RMN dans DMSO

| | |
|---|---|
| 1,29 (m) 4H-1,90 (m) 4H | les CH₂ du cyclohexyle |
| 1,70 (m) 2H-de 2 à 2,30 6H | les CH₂ du cyclopentyle |
| 3,43 (m) 1H | H₄ |
| 3,67 (m) 1H | H₁ |
| 4,84 (q) 1H | -CH du cyclopentyle |
| 7,12 (t) 1H | H_{c} |
| 7,37 (t) 2H | les H_{b} |
| 7,94 (dl) 2H | les Hₐ |
| 9,10 (s) -10,80 (s) | CH=N + H mobiles |

### EXEMPLE 88 : Dichlorhydrate de trans-9-cyclopentyl-4-[[2-[(4-hydroxycyclohexyl)amino]-9H-purin-6-yl]amino]-benzoate d'éthyle.

### Stade 1 : 4-[(2-chloro-9-cyclopentyl-9H-purin-6-yl)-amino]-benzoate d'éthyle.

On introduit à température ambiante 514 mg du produit obtenu au Stade 1 de l'exemple 1 dans 5 ml de n-butanol et 396 mg de 4-amino benzoate d'éthyle, et chauffe à une température d'environ 90 à 100°C pendant 19 heures, puis laisse revenir à température ambiante. On dilue avec 3 ml d'isopropanol, agite 30 minutes, essore et rince à l'isopropanol puis sèche sous pression réduite à 50°C. On obtient ainsi 761 mg de produit attendu.

### Stade 2 : dichlorhydrate de trans-9-cyclopentyl-4-[[2-[(4-hydroxycyclohexyl)-amino]-9H-purin-6-yl]-amino]-benzoate d'éthyle.

On mélange 575mg de trans-1,4-aminocyclohexanol que l'on porte à une température de 50 à 60°C puis ajoute 385 mg du produit obtenu au stade 1 ci-dessus et agite à une température d'environ 140°C pendant 17 heures. On laisse alors revenir à température ambiante, reprend avec de l'acétate d'éthyle et de l'eau et porte à une température d'environ 50°C. On laisse alors décanter, réextrait à l'acétate d'éthyle, lave avec une solution aqueuse saturée de chlorure de sodium, sèche et évapore à sec. On reprend par une solution éthanolique d'acide chlorhydrique, laisse cristalliser, essore, sèche sous pression réduite à 50°C et récupère 381 mg de produit brut attendu. On dissout 347 mg de ce chlorhydrate dans l'eau, ajoute une solution aqueuse d'ammoniaque (pH : 12) puis de l'acétate d'éthyle, décante, extrait à l'acétate d'éthyle, lave avec une solution aqueuse saturée de chlorure de sodium, sèche et évapore à sec. On chromatographie le résidu sur silice (éluant : CH₂Cl_{2/}MeOH 95/5), reprend par une solution éthanolique d'acide chlorhydrique, laisse cristalliser, essore, sèche sous pression réduite et récupère 300 mg de produit pur attendu.

### RMN dans DMSO

| | |
|---|---|
| 1,33 (m) 7H | CH₃ + H axiaux du cyclohexyle |
| 1,64 à 2,30 (m) | CH₂ du cyclopentyle + H |
| | équatoriaux du cyclohexyle |
| 3,46 (m) 1H-3,69 1H | les CH du cyclohexyle |
| 4,32 (t) 2H | OCH₂ |
| 4,83 (m) 1H | -CH du cyclopentyle |
| 7,93 2H-8,14 2H AA'BB' | HN-phényle-CO₂Et |
| 8,88 (sl) 1H | N-CH=N |
| 10,70 (sl) | H mobile |

### EXEMPLE 89 : Dichlorhydrate de trans-N-[2-[[2-[(4-aminocyclohexyl)-amino]-9-cyclopentyl-9H-purin-6-yl]amino]-éthyl]-4-(trifluorométhyl)-benzamide.

### Stade 1 : N-[2-[(2-chloro-9-cyclopentyl-9H-purin-6-yl)-amino]-éthyl]-4-(trifluorométhyl)-benzamide.

On mélange 280 mg du produit obtenu au stade 1 de l'exemple 7, 4 ml de chlorure de méthylène, 0,28 ml triéthylamine et 0,18 ml de chlorure de 4-trifluorométhylbenzoyle puis agite à température ambiante pendant une heure en entraînant à l'éther, essore et sèche sous pression réduite. On obtient ainsi 353 mg de produit attendu.

### Stade 2 : dichlorhydrate de trans-N-[2-[[2-[(4-aminocyclohexyl)-amino]-9-cyclopentyl-9H-purin-6-yl]-amino]-éthyl]-4-(trifluorométhyl)-benzamide.

On mélange 342 mg du produit obtenu au stade 1 ci-dessus et 428 mg de trans-1,4-diaminocyclohexane et chauffe à environ 140°C pendant 4 heures et demie. On évapore le solvant, chromatographie sur silice avec pour éluant chlorure de méthylène/méthanol/ammoniaque en proportion de 85/15/1,5 puis reprend par une solution éthanolique d'acide chlorhydrique, laisse cristalliser, essore, sèche sous pression réduite et récupère 281,6 mg de produit attendu.

### RMN dans DMSO

| | |
|---|---|
| 1,38 (m) -1,48 (m) 4H | les H axiaux du cyclohexyle |
| 1,70 (m) -1,90 (m) | |
| 2,02 (masqué) 8H | les CH₂ du cyclopentyle |
| 2,16 (m) | |
| 2,04 (m) 4H | H équatoriaux du cyclohexyle |
| 3,04 (sl) 1H | H₄ axial |
| 3,61 (m) - 3,85 (sl) | les CH₂-NH |
| 3,69 (tt) | H₁ axial |
| 4,73 (q) 1H | CH du cyclopentyle |
| 7,79-8,01 AA'BB' | -phényle- |
| 7,94 (sl) ≥ 2H-8,13(s) 1H | N=CH + H mobiles |
| 8,64 (tl) 1H | CH₂-NH |

### EXEMPLE 89 : Dichlorhydrate de trans-N-[2-[[2-[(4-aminocyclohexyl)-amino]-9-cyclopentyl-9H-purin-6-yl]-amino]-éthyl]-4-méthoxy-benzamide.

### Stade 1 : N-[2-[(2-chloro-9-cyclopentyl-9H-purin-6-yl)-amino]-éthyl]-4-méthoxy-benzamide.

On mélange 280 mg du produit obtenu au stade 1 de l'exemple 7, 4 ml de chlorure de méthylène, 0,28 ml triéthylamine et 0,18 ml de chlorure de 4-anisoyle puis agite à température ambiante pendant 30 minutes. On ajoute alors 2 ml d'eau, extrait au chlorure de méthylène, sèche et évapore les solvants. On chromatographie sur silice (éluant chlorure de méthylène/méthanol/ammoniaque 85/15/1,5 puis CH₂Cl_{2/}CH₃CN 8/2 puis chlorure de méthylène/méthanol/ammoniaque 85/15/1,5. On recueille 275 mg de produit attendu.

### Stade 2 : Dichlorhydrate de trans-N-[2-[[2-[(4-aminocyclohexyl)-amino]-9-cyclopentyl-9H-purin-6-yl]-amino]-éthyl]-4-méthoxy-benzamide.

On mélange 263 mg du produit obtenu au stade 1 ci-dessus et 360 mg de trans-1,4-diaminocyclohexane et chauffe à environ 140°C pendant 19 heures. On évapore le solvant, chromatographie sur silice avec pour éluant chlorure de méthylène/méthanol/ammoniaque en proportion de 85/15/1,5 puis reprend par une solution éthanolique d'acide chlorhydrique, laisse cristalliser, essore, sèche sous pression réduite et récupère 194 mg de produit attendu.

### RMN dans DMSO

| | |
|---|---|
| 1,37 (m) -1,49 (m) | les H axiaux du cyclohexyle |
| 1,70 (m) -1,89 (m) | |
| 2,00 (m) | les CH₂ du cyclopentyle |
| 2,18 (m) | |
| 2,07 (m) | H équatoriaux du cyclohexyle |
| 3,06 (sl) | H₄ axial |
| 3,58 (m) -3, 80 (masqué) | les CH₂-NH |
| 3,70 (tt) | H₁ axial |
| 3,82 (s) | -phényle-O-CH₃ |
| 4,74 (q) | CH du cyclopentyle |
| 6,96-7,81 AA'BB' | -phényle- |
| 7,98 (sl) -8,16 (s) -8,81 (s) | N=CH + H mobiles |
| 8,27 (tl) | CH₂-NH |

### EXEMPLE 91 : Dichlorhydrate de trans-N-[2-[[2-[(4-aminocyclohexyl)-amino]-9-cyclopentyl-9H-purin-6-yl]-amino]-éthyl]-3,5-dichloro-benzamide.

### Stade 1 : N-[2-[(2-chloro-9-cyclopentyl-9H-purin-6-yl)-amino]-éthyl]-3,5-dichloro-benzamide.

On mélange 280 mg du produit obtenu au stade 1 de l'exemple 7, 4 ml de chlorure de méthylène, 0,28 ml triéthylamine et 255 mg de chlorure de 3,5-dichlorobenzoyle puis agite à température ambiante pendant 30 minutes. On ajoute alors 2 ml d'eau, extrait au chlorure de méthylène, sèche et évapore les solvants. On obtient ainsi 454 mg de produit attendu.

### Stade 2 : dichlorhydrate de trans-N-[2-[[2-[(4-aminocyclohexyl)-amino]-9-cyclopentyl-9H-purin-6-yl]-amino]-éthyl]-3,5-dichloro-benzamide.

On mélange 360 mg du produit obtenu au stade 1 ci-dessus et 450 mg de trans-1,4-diaminocyclohexane et chauffe à environ 140°C pendant 6 heures. On évapore le solvant, chromatographie sur silice (éluant chlorure de méthylène/méthanol/ammoniaque 85/15/1,5) puis reprend par une solution éthanolique d'acide chlorhydrique, laisse cristalliser, essore, sèche sous pression réduite et récupère 280 mg de produit attendu.

### RMN dans DMSO

| | |
|---|---|
| 1,37 (m) -1, 52 (m) 4H | les H axiaux du cyclohexyle |
| 1,71 (m) -1,89 (m) | |
| 2,01 (masqué) 8H | les CH₂ du cyclopentyle |
| 2,17 (m) | |
| 2,06 (m) 4H | H équatoriaux du cyclohexyle |
| 3,05 (sl) 1H | H₄ axial |
| 3,58 (m) -3,87 (sl) | les CH₂-NH |
| 3,70 (tt) | H₁ axial |
| 4,75 (q) 1H | CH du cyclopentyle |
| 7,71 (tl) 1H | Hₐ |
| 7,82 (dl) 2H | les H_{b} |
| 8,03 (sl) 3H | |
| 8,21 (sl) | HN=CH + H mobiles |
| 8,96 (sl) ≤ H1 | |
| 8,70 (tl) 1H | CH₂-NH |

### EXEMPLE 92 : Dichlorhydrate de trans-N-[2-[[2-[(4-aminocyclohexyl)-amino]-9-cyclopentyl-9H-purin-6-yl]-amino]-éthyl]-4-chloro-benzamide.

### Stade 1 : N-[2-[(2-chloro-9-cyclopentyl-9H-purin-6-yl)-amino]-éthyl]-4-chloro-benzamide.

On mélange 280 mg du produit obtenu au stade 1 de l'exemple 7, 4 ml de chlorure de méthylène, 0,28 ml triéthylamine et 0,16 ml de chlorure de 4-chlorobenzoyle puis agite à température ambiante pendant environ 1 heure. On ajoute alors 4 ml d'eau, extrait au chlorure de méthylène, sèche et évapore les solvants. On obtient ainsi 230 mg de produit attendu.

### Stade 2 : dichlorhydrate de trans-N-[2-[[2-[(4-aminocyclohexyl)-amino]-9-cyclopentyl-9H-purin-6-yl]-amino]-éthyl]-4-chloro-benzamide.

On mélange 222 mg du produit obtenu au stade 1 ci-dessus et 302 mg de trans-1,4-diaminocyclohexane et chauffe à 140°C pendant 6 heures. On évapore le solvant, chromatographie sur silice (éluant chlorure de méthylène/méthanol/ammoniaque 85/15/1,5) puis reprend par une solution éthanolique d'acide chlorhydrique, laisse cristalliser, essore, sèche sous pression réduite et récupère 208 mg de produit attendu.

### RMN dans DMSO

| | |
|---|---|
| 1,35 (m) -1,49 (m) 4H | les H axiaux du cyclohexyle |
| 1,70 (m) -1,88 (m) | |
| 1,97 (masqué) 8H | les CH₂ du cyclopentyle |
| 2,15 (m) | |
| 2,04 (m) 4H | H équatoriaux du cyclohexyle |
| 3,05 (sl) 1H | H₄ axial |
| 3,57 (m) -3,82 (sl) | les CH₂-NH |
| 3,68 (tl) | H₁ axial |
| 4,74 (q) 1H | CH du cyclopentyle |
| 7,50 (tl)-7,85 AA'BB' | -phényle- |
| 8,01 (sl) 3H | |
| 8,20 (sl) ≤ | HN=CH + H mobiles |
| 8,97 (sl) ≤ 1H | |
| 8,58 (tl) 1H | CH₂-NH |

### EXEMPLE 93 : Dichlorhydrate de trans-N-[2-[[2-[(4-aminocyclohexyl)-amino]-9-cyclopeatyl-9H-purin-6-yl]-amino]-éthyl] -3,4-dichloro-benzamide.

### Stade 1 : N-[2-[(2-chloro-9-cyclopentyl-9H-purin-6-yl)-amino]-éthyl]-3,4-dichloro-benzamide.

On mélange 280 mg du produit obtenu au stade 1 de l'exemple 7, 4 ml de chlorure de méthylène, 0,28 ml triéthylamine et 251 mg de chlorure de 3,4-dichloro-benzoyle puis agite à température ambiante pendant 1 heure. On ajoute alors 2 ml d'eau, extrait au chlorure de méthylène, sèche et évapore les solvants. On obtient ainsi 237 mg de produit attendu.

### Stade 2 : Dichlorhydrate de trans-N-[2-[[2-[(4-aminocyclohexyl)-amino]-9-cyclopentyl-9H-purin-6-yl]-amino]-éthyl]-3,4-dichloro-benzamide.

On mélange 225 mg du produit obtenu au stade 1 ci-dessus et 285 mg de trans-1,4-diaminocyclohexane et chauffe à environ 140°C pendant 6 heures. On évapore le solvant, chromatographie sur silice (éluant chlorure de méthylène/méthanol/ammoniaque 85/15/1,5) puis reprend par une solution éthanolique d'acide chlorhydrique, laisse cristalliser, essore, sèche sous pression réduite et récupère 168 mg de produit attendu.

### RMN dans DMSO

| | |
|---|---|
| 1,35 (m) -1,50 (m) 4H | les H axiaux du cyclohexyle |
| 1,69 (m) -1,88 (m) | |
| 2,04 (masqué) 8H | les CH₂ du cyclopentyle |
| 2,16 (m) | |
| 2,04 (m) 4H | H équatoriaux du cyclohexyle |
| 3,04 (sl) 1H | H₄ axial |
| 3,58 (m) -3,82 (sl) | les CH₂-NH |
| 3,68 (tl) 1H | H₁ axial |
| 4,74 (q) 1H | CH du cyclopentyle |
| 7,71 (d) 1H | H_{c} |
| 7,80 (dd) 1H | H_{b} |
| 8,03 (d) 1H | Hₐ |
| 8,06 (sl) 3H | |
| 8,24 (sl) | N=CH + H mobiles |
| 9,15 (sl) ≤ 1H | |
| 8,72 (tl) 1H | CH₂-NH |

### EXEMPLE 94 : Dichlorhydrate de trans-N-[2-[[2-[(4-aminocyclohexyl)-amino]-9-cyclopentyl-9H-purin-6-yl]-amino]-éthyl]-3,4-diméthoxy-benzamide.

### Stade 1 : N-[2-[(2-chloro-9-cyclopentyl-9H-purin-6-yl)-amino]-éthyl]-3,4-diméthoxy-benzamide.

On mélange 280 mg du produit obtenu au stade 1 de l'exemple 7, 4 ml de chlorure de méthylène, 0,28 ml triéthylamine et 241 mg de chlorure de 3,4-diméthoxy-benzoyle puis agite à température ambiante pendant environ 5 heures. On ajoute alors 2 ml d'eau, extrait au chlorure de méthylène, sèche et évapore les solvants. On obtient ainsi 230 mg de produit attendu.

### Stade 2 : Dichlorhydrate de trans-N-[2-[[2-[(4-aminocyclohexyl)-amino]-9-cyclopentyl-9H-purin-6-yl]-amino]-éthyl]-3,4-diméthoxy-benzamide.

On mélange 230 mg du produit obtenu au stade 1 ci-dessus et 293 mg de trans-1,4-diaminocyclohexane et chauffe à 140°C pendant 6 heures. On évapore le solvant, chromatographie sur silice (éluant chlorure de méthylène/méthanol/ammoniaque 85/15/1,5) puis reprend par une solution éthanolique d'acide chlorhydrique, laisse cristalliser, essore, sèche sous pression réduite et récupère 99 mg de produit attendu.

### RMN dans DMSO

| | |
|---|---|
| 1,35 (m) -1,48 (m) 4H | les H axiaux du cyclohexyle |
| 1, 69 (m) -1,88 (m) | |
| 2,03 (masqué) | les CH₂ du cyclopentyle |
| 2,15 (m) 8H | |
| 2,02 (m) 4H | H équatoriaux du cyclohexyle |
| 3,05 (sl) 1H | H₄ axial |
| 3,46 (m) -3,77 (sl) | les CH₂-NH |
| 3,67 (tl) | H₁ supposé axial |
| 3,80 (s) 3Hx2 | les OCH₃ |
| 4,74 (qt) 1H | CH du cyclopentyle |
| 6,99 (d) 1H | H₃' |
| 7,40 à 7,48 (m) 2H | H₄'- H₆' |
| 7,99 (dl) >2H | NH₂ + N=CH-N |
| 8,22 (sl) -8,32 (tl) -9,04 (sl) | les H mobiles |

### EXEMPLE 95 : Dichlorhydrate de trans-N-[2-[[2-[(4-aminocyclohexyl)-amino]-9-cyclopentyl-9H-purin-6-yl]-amino]-éthyl]-2-chloro-4-nitro-benzamide.

### Stade 1 : 2-chloro-N-[2-[(2-chloro-9-cyclopentyl-9H-purin-6-yl)-amino]-éthyl]-4-nitro-benzamide.

On mélange 280 mg du produit obtenu au stade 1 de l'exemple 7, 4 ml de chlorure de méthylène, 0,28 ml triéthylamine et 264 mg de chlorure de 2-chloronitrobenzoyle puis agite à température ambiante pendant 3 heures. On ajoute alors 2 ml d'eau, extrait au chlorure de méthylène, sèche et évapore les solvants. On obtient ainsi 295 mg de produit attendu.

### Stade 2 : Dichlorhydrate de trans-N-[2-[[2-[(4-aminocyclohexyl)-amino]-9-cyclopentyl-9H-purin-6-yl]-amino]-éthyl]-2-chloro-4-nitro-benzamide.

On mélange 295 mg du produit obtenu au stade 1 ci-dessus et 362 mg de trans-1,4-diaminocyclohexane et chauffe à environ 140°C pendant 6 heures. On évapore le solvant, chromatographie sur silice (éluant chlorure de méthylène/méthanol/ammoniaque 85/15/1,5) puis reprend par une solution éthanolique d'acide chlorhydrique, laisse cristalliser, essore, sèche sous pression réduite et récupère 105 mg de produit attendu.

### RMN dans DMSO

| | |
|---|---|
| 1,35 (m) -1,50 (m) 4H | les H axiaux du cyclohexyle |
| 1, 70 (m) -1,90 (m) | |
| 1,97 (masqué) 8H | les CH₂ du cyclopentyle |
| 2,17 (m) | |
| 2,05 (m) 4H | H équatoriaux du cyclohexyle |
| 3,04 (sl) 1H | H₄ axial |
| 3,59 (m)-3,83 (masqué) | les CH₂-NH |
| 3,71 (m) 1H | H₁ axial |
| 4,76 (q) 1H | CH du cyclopentyle |
| 7,77 (d) 1H | H_{c} |
| 8,19 (dd) 1H | H_{b} |
| 8,28 (d) 1H | Hₐ |
| 8,02 (sl) 3H-9,02 (sl) ≤ 1H | N=CH + H mobiles |
| 8,71 (tl) 1H | CH₂-NH |

### EXEMPLE 96 : Dichlorhydrate de trans-N-[2-[[2-[(4-aminocyclohexyl)-amino]-9-cyclopentyl-9H-purin-6-yl]-amino]-éthyl]-3,5-bis(trifluorométhyl)-benzamide.

### Stade 1 : N-[2-[(2-chloro-9-cyclopentyl-9H-purin-6-yl]-amino]-éthyl]-3,5-bis(trifluorométhyl)-benzamide.

On mélange 280 mg du produit obtenu au stade 1 de l'exemple 7, 4 ml de chlorure de méthylène, 0,28 ml triéthylamine et 241 mg de chlorure de 3,5-trifluorométhylbenzoyle puis agite à température ambiante pendant environ 5 heures. On ajoute alors 2 ml d'eau, extrait au chlorure de méthylène, sèche et évapore les solvants. On obtient ainsi 390 mg de produit attendu.

### Stade 2 : Dichlorhydrate de trans-N-[2-[[2-[(4-aminocyclohexyl)-amino]-9-cyclopentyl-9H-purin-6-yl]-amino]-éthyl]-3,5-bis(trifluorométhyl)-benzamide.

On mélange 368 mg du produit obtenu au stade 1 ci-dessus et 402 mg de trans-1,4-diaminocyclohexane et chauffe à 140°C pendant 6 heures. On évapore le solvant, chromatographie sur silice (éluant chlorure de méthylène/méthanol/ammoniaque 85/15/1,5) puis reprend par une solution éthanolique d'acide chlorhydrique, laisse cristalliser, essore, sèche sous pression réduite et récupère 210 mg de produit attendu.

### RMN dans DMSO

| | |
|---|---|
| 1,33 (m) -1,48 (m) 4H | les H axiaux du cyclohexyle |
| 1,69 (m) -1,90 (m) -2,15 (m) 8H | les CH₂ du cyclopentyle |
| 2,00 (m) 4H | H équatoriaux du cyclohexyle |
| 3,02 (sl)1H | H₄ axial |
| 3,63 (m) | les CH₂-NH |
| 3,67 (masqué) | H₁ supposé axial |
| 4,74 (qt) 1H | CH du cyclopentyle |
| 8,25 (s) 1H | Hₐ |
| 8,45 (s) 2H | les H_{b} |
| 8,04 (sl) 3H | CH=N + H mobiles |
| 9,07 (tl) 1H | CH₂-NH |

### EXEMPLE 97 : Dichlorhydrate de trans-N-[2-[[2-[(4-aminocyclohexyl)-amino]-9-cyclopentyl-9H-purin-6-yl]-amino]-éthyl]-4-(méthylthio)-benzamide.

### Stade 1 : N-[2-[(2-chloro-9-cyclopentyl-9H-purin-6-yl)-amino]-éthyl]-4-(méthylthio)-benzamide.

On mélange 200 mg d'acide 4-(méthylthio) benzoique, 205 mg de 1-hydroxybenzotriazole hydrate, 290 mg de chlorhydrate de 1-(3-diméthylaminopropyl)-3-éthylcarbodiimide dans 4 ml de dichlorométhane, ajoute 280 mg du produit obtenu au stade 1 de l'exemple 7 et agite 4 heures et demie à température ambiante. On ajoute de l'eau, essore, lave à l'éther, sèche et recueille 336 mg de produit attendu.

### Stade 2 : dichlorhydrate de trans-N-[2-[[2-[(4-aminocyclohexyl)-amino]-9-cyclopentyl-9H-purin-6-yl]-amino]-éthyl]-4-(méthylthio)-benzamide.

On mélange 296 mg du produit obtenu au stade 1 ci-dessus et 393 mg de trans-1,4-diaminocyclohexane et chauffe à environ 140°C pendant 6 heures. On évapore le solvant, chromatographie sur silice (éluant chlorure de méthylène/méthanol/ammoniaque 85/15/1,5) puis reprend par une solution éthanolique d'acide chlorhydrique, laisse cristalliser, essore, sèche sous pression réduite et récupère 51 mg de produit attendu.

### RMN dans DMSO

| | |
|---|---|
| 1,35 (m) -1,50 (m) 4H | les H axiaux du cyclohexyle |
| 1,69 (m) -1,90 (m) | |
| 1,95 (m) 8H | les CH₂ du cyclopentyle |
| 2,15 (m) | |
| 2,03 (m) 4H | H équatoriaux du cyclohexyle |
| 2,50 (masqué) | CH₃-S-phényle |
| 3,05 (sl) 1H | H₄ axial |
| 3,57 (m) -3,80 (masqué) | les CH₂-N |
| 3,68 (tl) | H₁ axial |
| 4,73 (q) 1H | CH du cyclopentyle |
| 7,29-7,77 AA'BB' 4H | -phényle- |
| 8,02 (sl) ≤3H | |
| 8,21 (sl) 1H | N=CH + H mobiles |
| 9,06 (sl) ≤1H | |
| 8,45 (tl) 1H | CH₂-NH |

### EXEMPLE 98 : Dichlorhydrate de trans-N-[2-[[2-[(4-aminocyclohexyl)-amino]-9-cyclopentyl-9H-purin-6-yl]-amino]-éthyl]-4-fluoro-benzamide.

### Stade 1 : N-[2-[(2-chloro-9-cyclopentyl-9H-purin-6-yl)-amino]-éthyl]-4-fluoro-benzamide.

On mélange 280 mg du produit obtenu au stade 1 de l'exemple 7, 4 ml de chlorure de méthylène, 0,28 ml triéthylamine et 0,14 ml de chlorure de 4-fluorobenzoyle puis agite à température ambiante pendant 2 heures. On ajoute alors de l'éther, essore, sèche, reprend à l'eau, extrait au chlorure de méthylène, sèche et évapore les solvants. On obtient ainsi 283 mg de produit attendu.

### Stade 2 : dichlorhydrate de trans-N-[2-[[2-[(4-aminocyclohexyl)-amino]-9-cyclopentyl-9H-purin-6-yl]-amino]-éthyl]-4-fluoro-benzamide.

On mélange 247 mg du produit obtenu au stade 1 ci-dessus et 350 mg de trans-1,4-diaminocyclohexane et chauffe à 140°C pendant 6 heures. On évapore le solvant, chromatographie sur silice (éluant chlorure de méthylène/méthanol/ammoniaque 85/15/1,5) puis reprend par une solution éthanolique d'acide chlorhydrique, laisse cristalliser, essore, sèche sous pression réduite et récupère 130 mg de produit attendu.

### RMN dans DMSO

| | |
|---|---|
| 1,37 (m)- 1,48 (m) 4H | les H axiaux du cyclohexyle |
| 1,69 (m) -1,89 (m) | |
| 2,03 (masqué) 8H | les CH₂ du cyclopentyle |
| 2,15 (m) | |
| 2,03 (m) 4H | H équatoriaux du cyclohexyle |
| 3,06 (sl) 1H | H₄ axial |
| 3,47 à 3,96(m) | les CH₂-NH + H1 supposé axial |
| 4,74 (qt) 1H | CH du cyclopentyle |
| 7,25 (t) 2H-7,90 (t) 2H | F-phényle-CO |
| 8,04 (sl) >2H | NH₂ + N=CH |
| 8,23 (sl) < 1H | |
| 8,53 (tl) < 1H | les H mobiles |
| 9,07 (sl) < 1H | |

### EXEMPLE 99 : Dichlorhydrate de trans-N-[2-[[2-[(4-aminocyclohexyl)-amino]-9-cyclopentyl-9H-purin-6-yl]-amino]-éthyl]-3-(trifluorométhyl)-benzamide.

### Stade 1 : N-[2-[(2-chloro-9-cyclopentyl-9H-purin-6-yl)-amino]-éthyl]-3-(trifluorométhyl)-benzamide.

On mélange 280 mg du produit obtenu au stade 1 de l'exemple 7, 203 mg de 1-hydroxybenzotriazole hydrate, 287 mg de chlorhydrate de 1-(3-diméthylaminopropyl)-3-éthylcarbodiimide dans 4 ml de dichlorométhane, ajoute 230 mg d'acide 3-trifluorométhyl benzoïque et agite 6 heures et demie à température ambiante. On ajoute de l'eau, essore, lave à l'éther, sèche et recueille 307 mg de produit attendu.

### Stade 2 : dichlorhydrate de trans-N-[2-[[2-[(4-aminocyclohexyl)-amino]-9-cyclopentyl-9H-purin-6-yl]-amino]-éthyl]-3-(trifluorométhyl)-benzamide.

On mélange 290 mg du produit obtenu au stade 1 ci-dessus et 365 mg de trans-1,4-diaminocyclohexane et chauffe à environ 140°C pendant 6 heures. On évapore le solvant, chromatographie sur silice (éluant chlorure de méthylène/méthanol/ammoniaque 85/15/1,5) puis reprend par une solution éthanolique d'acide chlorhydrique, laisse cristalliser, essore, sèche sous pression réduite et récupère 206 mg de produit attendu.

### RMN dans DMSO

| | |
|---|---|
| 1,36 (m) -1,51 (m) 4H | les H axiaux du cyclohexyle |
| 1, 70 (m) -1, 90 (m) | |
| 1,96 (m) 8H | les CH₂ du cyclopentyle |
| 2,16 (m) | |
| 2,04 (m) 4H | H équatoriaux du cyclohexyle |
| 3,05 (sl) 1H | H₄ axial |
| 3,61 (m) -3, 88 (sl) | les CH₂-NH |
| 3,70 (tl) 1H | H₁ axial |
| 4,74 (q) 1H | CH du cyclopentyle |
| 7,70 (t) 1H | H_{c} |
| 7,86 (dl) 1H | H_{b} |
| 8,13 (sl) 2H | Hₐ et H_{d} |
| 7,99 (sl) ≥2H | |
| 8,17 (s) 1H | N=CH + H mobiles |
| 8,88 (sl) ≤1H | |
| 8,71 (tl) 1H | CH₂-NH |

### EXEMPLE 100 : Dichlorhydrate de trans-N-[2-[[2-[(4-aminocyclohexyl)-amino]-9-cyclopentyl-9H-purin-6-yl]-amino]-éthyl]-3-(trifluorométhoxy)-benzamide.

### Stade 1 : N-[2-[(2-chloro-9-cyclopentyl-9H-purin-6-yl)-amino]-éthyl]-3-(trifluorométhoxy)-benzamide.

On mélange 280 mg du produit obtenu au stade 1 de l'exemple 7, 203 mg de 1-hydroxybenzotriazole hydrate, 287 mg de chlorhydrate de 1-(3-diméthylaminopropyl)-3-éthyl-carbodiimide dans 4 ml de dichlorométhane, ajoute 230 mg d'acide 3-trifluorométhoxy benzoïque et agite 4 heures et demie à température ambiante. On ajoute de l'eau, extrait au chlorure de méthylène, sèche et évapore les solvants. On recristallise dans l'éther, essore, sèche et recueille 326 mg de produit attendu.

### Stade 2 : dichlorhydrate de trans-N-[2-[[2-[(4-aminocyclohexyl)-amino]-9-cyclopentyl-9H-purin-6-yl]-amino]-éthyl]-3-(trifluorométhoxy)-benzamide.

On mélange 300 mg du produit obtenu au stade 1 ci-dessus et 365 mg de trans-1,4-diaminocyclohexane et chauffe à environ 140°C pendant 6 heures. On évapore le solvant, chromatographie sur silice (éluant chlorure de méthylène/méthanol/ammoniaque 85/15/1,5) puis reprend par une solution éthanolique d'acide chlorhydrique, laisse cristalliser, essore, sèche sous pression réduite et récupère 200 mg de produit attendu.

### RMN dans DMSO

| | |
|---|---|
| 1,36 (m) -1,50 (m) 4H | les H axiaux du cyclohexyle |
| 1,70 (m) -1,90 (m) | |
| 1,96 (m) 8H | les CH₂ du cyclopentyle |
| 2,15 (m) | |
| 2,04 (m) 4H | H équatoriaux du cyclohexyle |
| 3,05 (sl) 1H | H₄ axial |
| 3,60 (m) -3,84 (sl) | les CH₂-NH |
| 3,70 (tt) 1H | H₁ axial |
| 4,74 (q) 1H | CH du cyclopentyle |
| 7,49 (dl) 1H | H_{b} |
| 7,59 (t) 1H | H_{c} |
| 7,75 (tl) 1H | Hₐ |
| 7,87 (dl) 1H | H_{d} |
| 7,94 (sl) ≤3H-8,10 (sl) 1H | N=CH + H mobiles |
| 8,60(tl) 1H | CH₂-NH |

### EXEMPLE 101 : Dichlorhydrate de trans-N-[2-[[2-[(4-aminocyclohexyl)-amino]-3-chloro-9-cyclopentyl-9H-purin-6-yl]-amino]-éthyl]-benzamide.

### Stade 1 : 3-chloro-N-[2-[(2-chloro-9-cyclopentyl-9H-purin-6-yl)-amino]-éthyl]-benzamide.

On mélange 190 mg d'acide 3-chlorobenzoique, 205 mg de 1-hydroxybenzotriazole hydrate, 287 mg de chlorhydrate de 1-(3-diméthylaminopropyl)-3-éthylcarbodiimide dans 4 ml de dichlorométhane, ajoute 280 mg du produit obtenu au stade 1 de l'exemple 7 et agite 5 heures et demie à température ambiante. On ajoute de l'eau, extrait au chlorure de méthylène, sèche et évapore les solvants. On recristallise dans l'éther, essore, sèche et recueille 334 mg de produit attendu.

### Stade 2 : Dichlorhydrate de trans-N-[2-[[2-[(4-aminocyclohexyl)-amino]-3-chloro-9-cyclopentyl-9H-purin-6-yl]-amino]-éthyl]-benzamide.

On mélange 292 mg du produit obtenu au stade 1 ci-dessus et 396 mg de trans-1,4-diaminocyclohexane et chauffe à environ 140°C pendant 6 heures. On évapore le solvant, chromatographie sur silice (éluant chlorure de méthylène/méthanol/ammoniaque 85/15/1,5) puis reprend par une solution éthanolique d'acide chlorhydrique, laisse cristalliser, essore, sèche sous pression réduite et récupère 127,5 mg de produit attendu.

### RMN dans DMSO

| | |
|---|---|
| 1,35 (m) -1,50 (m) 4H | les H axiaux du cyclohexyle |
| 1,69 (m) -1,90 (m) | |
| 1,97 (m) 8H | les CH₂ du cyclopentyle |
| 2,15 (m) | |
| 2,03 (m) 4H | H équatoriaux du cyclohexyle |
| 3,05 (sl) 1H | H₄ axial |
| 3,58 (m) -3,81 (masqué) | les CH₂-NH |
| 3,68 (tl) 1H | H₁ axial |
| 4,74 (q) 1H | CH du cyclopentyle |
| 7,47 (t) 1H | H_{c} |
| 7,57 (ddd) 1H | H_{d} |
| 7,78 (dl) 1H | H_{b} |
| 7,84 (sl) 1H | Hₐ |
| 8,03 (sl) 3H | |
| 8,23 (sl) 1H | N=CH + H mobiles |
| 9,06 (sl) 1H | |
| 8,63 (tl) 1H | CH₂-NH |

### EXEMPLE 102 : Dichlorhydrate de trans-4-[[2-[(4-aminocyclohexyl)-amino]-9-cyclopentyl-9H-purin-6-yl]-amino]-benzèneacétate d'éthyle.

### Stade 1 : 4-[(2-chloro-9-cyclopentyl-9H-purin-6-yl)-amino]-benzèneacétate d'éthyle.

On mélange à température ambiante 257 mg du produit obtenu au Stade 1 de l'exemple 1 dans 4 ml d'éthanol, et 215 mg de 4-amino-benzèneacétate d'éthyle et 165 mg de carbonate de potassium puis chauffe à une température d'environ 90°C pendant 20 heures puis laisse revenir à température ambiante. On dilue avec 15 ml d'acétate d'éthyle et 10 ml d'eau, extrait à l'acétate d'éthyle, lave à l'eau, évapore les solvants, reprend à l'éther isopropylique, essore et sèche sous pression réduite à 50°C. On obtient ainsi 222 mg de produit attendu.

### Stade 2 : dichlorhydrate de trans-4-[[2-[(4-aminocyclohexyl)-amino]-9-cyclopentyl-9H-purin-6-yl]-amino]-benzèneacétate d'éthyle.

On mélange 180 mg du produit obtenu au stade 1 ci-dessus et 360 mg de trans-1,4-diaminocyclohexane et chauffe à environ 140°C pendant 5 heures. On évapore le solvant, chromatographie sur silice (éluant méthanol /ammoniaque 98/2) puis reprend par une solution éthanolique d'acide chlorhydrique, évapore les solvants, empâte à l'éther, essore, sèche sous pression réduite à 50°C et récupère 142 mg de produit attendu.

### RMN dans DMSO

| | |
|---|---|
| 1,20 (t) | CH₃-CH₂-O |
| 1,40 (m)- 1,49 (m) 4H | les H axiaux du cyclohexyle |
| 1,72 (m) -1,91 (m) | |
| 2,07 (masqué) 8H | les CH₂ du cyclopentyle |
| 2,20 (m) | |
| 2,07 (m) 4H | H équatoriaux du cyclohexyle |
| 3,02 (sl) 1H | H₄ axial |
| 3,63 (s) 2H | henyl-CH₂-CO |
| 3,70 (tl) 1H | H₁ axial |
| 4,10 (q) | CH₃-CH₂-O |
| 4,84 (q) 1H | CH du cyclopentyle |
| 7,27-7,90 AA'BB' | -phényle- |
| 8,07 (sl) ≥3H | |
| 8,93 (sl) 1H | N=CH + H mobiles |
| 10,56 (sl) 1H | |

### EXEMPLE 103 : Dichlorhydrate de trans-4-[[2-[(4-aminocyclohexyl)-amino]-9-cyclopentyl-9H-purin-6-yl]-amino]-N-(2-thiazolyl)-benzènesulfonamide.

### Stade 1 : 4-[(2-chloro-9-cyclopentyl-9H-purin-6-yl)-amino]-N-(2-thiazolyl)-benzènesulfonamide.

On introduit à température ambiante 257 mg du produit obtenu au Stade 1 de l'exemple 1 dans 4 ml de n-butanol et 306 mg de 4-amino-N-(2-thiazolyl)-benzènesulfonamide et plonge dans un bain à la température d'environ 100°C pendant 14 heures sous agitation puis laisse revenir à température ambiante. On essore, rince à l'éther puis sèche sous pression réduite à 50°C. On obtient ainsi 74 mg de produit attendu.

### Stade 2 : dichlorhydrate de trans-4-[[2-[(4-aminocyclohexyl)-amino]-9-cyclopentyl-9H-purin-6-yl]-amino]-N-(2-thiazolyl)-benzènesulfonamide.

On porte 400 mg de trans-1,4-diaminocyclohexane à environ 150°C puis ajoute 333 mg du produit obtenu au stade 1 ci-dessus. On laisse sous agitation pendant 3 heures puis laisse revenir à température ambiante. On dilue avec 30 ml de dichlorométhane, ajoute 10 ml de méthanol, lave avec 10 ml d'eau, sèche, évapore les solvants et chromatographie sur silice (éluant : MeOH/NH₄OH 98/2). On reprend le résidu par une solution éthanolique d'acide chlorhydrique, évapore les solvants, empâte à l'éther, essore, sèche sous pression réduite à température ambiante et récupère 66 mg de produit attendu.

### RMN dans DMSO

| | |
|---|---|
| 1,38 (m) -1,51 (m) 4H | les H axiaux du cyclohexyle |
| 1,71 (m) -1,91 (m) | |
| 2,05 (m) 8H | les CH₂ du cyclopentyle |
| 2,18 (m) | |
| 2,09 (m) 4H | H équatoriaux du cyclohexyle |
| 3,02 (sl) 1H | H₄ axial |
| 3,69 (masqué) 1H | H₁ axial |
| 4,82 (q) 1H | CH du cyclopentyle |
| 6,81 (d) -7,20 (d) 2H | Hₐ et H_{b} |
| 7,77-8,15 AA'BB' 4H | -phényle- |
| 8,06 (sl) ≤3H | |
| 8,81 (s) 1H | N=CH + H mobiles |
| 10,64 (sl) ≤1H | |

### EXEMPLE 104 : Dichlorhydrate de trans-4-[[2-[(4-aminocyclohexyl)-amino]-9-cyclopentyl-9H-purin-6-yl]-amino]-benzènesulfonamide.

### Stade 1 : 4-[(2-chloro-9-cyclopentyl-9H-purin-6-yl)-amino]-benzènesulfonamide.

On introduit à température ambiante 257 mg du produit obtenu au Stade 1 de l'exemple 1 dans 4 ml de n-butanol et 207 mg de sulfanilamide et plonge dans un bain à la température d'environ 100°C pendant 16 heures sous agitation puis laisse revenir à température ambiante. On essore, rince à l'éther puis sèche sous pression réduite à 50°C. On obtient ainsi 339 mg de produit attendu.

### Stade 2 : Dichlorhydrate de trans-4-[[2-[(4-aminocyclohexyl)-amino]-9-cyclopentyl-9H-purin-6-yl]-amino]-benzènesulfonamide.

On porte 400 mg de trans-1,4-diaminocyclohexane à environ 150°C puis ajoute 275 mg du produit obtenu au stade 1 ci-dessus. On laisse sous agitation pendant 3 heures puis laisse revenir à température ambiante, évapore les solvants et chromatographie sur silice (éluant : MeOH/NH₄OH 98/2). On reprend le résidu par une solution éthanolique d'acide chlorhydrique, évapore les solvants, empâte à l'éther, essore, sèche sous pression réduite à température ambiante et récupère 205 mg de produit attendu.

### RMN dans DMSO

| | |
|---|---|
| 1,40 (m) -1,55 (m) 4H | les H axiaux du cyclohexyle |
| 1,72 (m) -1,91 (m) | |
| 2,08 (masqué) 8H | les CH₂ du cyclopentyle |
| 2,19 (m) | |
| 2,08 (d) 4H | H équatoriaux du cyclohexyle |
| 3,03 (sl) 1H | H₄ axial |
| 3,71 (tt) 1H | H₁ axial |
| 4,84 (qt) 1H | CH du cyclopentyle |
| 7,83-8,16 AA'BB' 4H | -phényle- |
| 8,08 (sl) >2H | NH₂-cyclohexyle + N=CH-N |
| 7,17 (sl) | |
| 2H-8,88 (sl) 1H | H mobiles |
| 10,75 (sl) 1H | |

### EXEMPLE 105 : Dichlorhydrate de trans-N2-(4-aminocyclohexyl)-9-cyclopentyl-N6-(4-méthoxyphényl)-9H-purine-2,6-diamine.

### Stade 1 : 2-chloro-9-cyclopentyl-N-(4-méthoxyphényl)-9H-purin-6-amine.

On introduit à température ambiante 257 mg du produit obtenu au Stade 1 de l'exemple 1 dans 4 ml de n-butanol et 148 mg de p-anisidine et plonge dans un bain à la température d'environ 100°C pendant 17 heures sous agitation puis laisse revenir à température ambiante. On essore, rince à l'éther puis sèche sous pression réduite à 50°C. On obtient ainsi 239 mg de produit attendu.

### Stade 2 : dichlorhydrate de trans-N2-(4-aminocyclohexyl)-9-cyclopentyl-N6-(4-méthoxyphényl)-9H-purine-2,6-diamine.

On porte 400 mg de trans-1,4-diaminocyclohexane à environ 150°C puis ajoute 172 mg du produit obtenu au stade 1 ci-dessus. On laisse sous agitation pendant 2 heures et demie puis laisse revenir à température ambiante. On évapore les solvants et chromatographie le résidu sur silice (éluant : MeOH/NH₄OH 98/2). On reprend le résidu par une solution éthanolique d'acide chlorhydrique, évapore les solvants, empâte à l'éther, essore, sèche sous pression réduite à température ambiante et récupère 188 mg de produit attendu.

### RMN dans DMSO

| | |
|---|---|
| 1,36 (m) -1,51 (m) 4H | les H axiaux du cyclohexyle |
| 1,71 (m) -1,91 (m) | |
| 2,05 (masqué) 8H | les CH₂ du cyclopentyle |
| 2,20 (m) | |
| 2,06 (d) 4H | H équatoriaux du cyclohexyle |
| 3,02 (sl) 1H | H₄ axial |
| 3,68 (tt) 1H | H₁ axial |
| 3,78 (s) 3H | OCH₃ |
| 4,82 (qt) 1H | CH du cyclopentyle |
| 6,98 2H-7,78 2H AA'BB' | -phényle-O |
| 8,06 (sl) <3H | NH₂-cyclohexyle + N=CH-N |
| 8,83 (sl) <2H-10,53 (sl) <1H | H mobiles |

### EXEMPLE 106 : Dichlorhydrate de trans-4-[[2-[(4-aminocyclohexyl)-amino]-9-cyclopentyl-9H-purin-6-yl]-amino]-benzèneacétate de butyle.

### Stade 1 : 4-[(2-chloro-9-cyclopentyl-9H-purin-6-yl)-amino]-benzèneacétate de butyle.

On mélange à température ambiante 257 mg du produit obtenu au Stade 1 de l'exemple 1 dans 4 ml d'éthanol, 215 mg de 4-amino phényl méthyl carboxylate d'éthyle et 165 mg de carbonate de potassium puis chauffe à une température d'environ 100°C pendant 24 heures puis laisse revenir à température ambiante. On évapore les solvants, dilue avec 10 ml d'eau, extrait à l'acétate d'éthyle, évapore les solvants et chromatographie sur silice (éluant : CH₂Cl₂/AcOEt 8/2).

### Stade 2 : dichlorhydrate de trans-4-[[2-[(4-aminocyclohexyl)-amino]-9-cyclopentyl-9H-purin-6-yl]-amino]-benzène-acétate de butyle.

On porte 400 mg de trans-1,4-diaminocyclohexane à environ 150°C et 200 mg du produit obtenu au stade 1 ci-dessus. On laisse sous agitation pendant 5 heures et demie puis laisse revenir à température ambiante, évapore les solvants et chromatographie le résidu sur silice (éluant : MeOH/NH₄OH 98/2). On reprend le résidu par une solution éthanolique d'acide chlorhydrique, évapore les solvants, empâte à l'éther, essore, sèche sous pression réduite à température ambiante et récupère 165 mg de produit attendu.

### RMN dans DMSO

| | |
|---|---|
| 0,88 (t) | CH₃-CH₂-CH₂-CH₂O |
| 1,32 (m) | CH₃-CH₂-CH₂-CH₂O |
| 1,56 (m) | CH₃-CH₂-CH₂-CH₂O |
| 4,06 (t) | CH₃-CH₂-CH₂-CH₂O |
| 1,37 (m) -1,52 (m) 4H | les H axiaux du cyclohexyle |
| 1,72 (m) -1,92 (m) | |
| 2,08 (m) 8H | les CH₂ du cyclopentyle |
| 2,21 (m) | |
| 2,08 (d) 4H | H équatoriaux du cyclohexyle |
| 3,02 (sl) 1H | H₄ axial |
| 3,64 (s) 2H | -phényle-CH₂-CO |
| 3,70 (tt) 1H | H₁ axial |
| 4,85 (qt) 1H | CH du cyclopentyle |
| 7,28 2H-7,90 2H AA'BB' | -phényle-O |
| 8,10 (sl) <3H | NH₂-cyclohexyle + N=CH-N |
| 9,02 (sl) <1H-10,67 (sl) <1H | H mobiles |

### EXEMPLE 107 : Dichlorhydrate de trans-N2-(4-aminocyclohexyl)-9-cyclopentyl-N6-[4-(1H-tétrazol-5-yl)phényl]-9H-purin-2,6-diamine.

On mélange à température ambiante 296 mg du produit obtenu à l'exemple 83 dans 3 ml de toluène et 0,26 ml d'azido tributyl étain puis chauffe à une température d'environ 120°C pendant 22 heures puis laisse revenir à température ambiante. On ajoute 7 ml de tetrahydrofuranne à la suspension obtenue, fait barboter de l'acide chlorhydrique gazeux pendant 1 minute puis de l'azote pendant 10 minutes, essore le produit obtenu, le rince à l'éther et le sèche sous pression réduite à 50°C. On obtient ainsi 374 mg de produit attendu.

### RMN dans DMSO

| | |
|---|---|
| 1,43 (m) -1,55 (m) 4H | les H axiaux du cyclohexyle |
| 1,72 (m) -1,91 (m) | |
| 2,09 (m) 8H | les CH₂ du cyclopentyle |
| 2,20 (m) | |
| 2,09 (m) 4H | H équatoriaux du cyclohexyle |
| 3,06 (sl) 1H | H₄ axial |
| 3,75 (tt) 1H | H₁ axial |
| 4,83 (m) 1H | CH du cyclopentyle |
| 8,12-8,23 AA'BB' | -phényle-O |
| 8,03 (sl) ≥3H | |
| 8,84 (sl) 1H | N=CH-N + H mobiles |
| 10,69 (sl) 1H | |

### EXEMPLE 108 : Dichlorhydrate de trans-3-[[2-[(4-aminocyclohexyl)-amino]-9-cyclopentyl-9H-purin-6-yl]-amino]-benzamide.

### Stade 1 : 3-[(2-chloro-9-cyclopentyl-9H-purin-6-yl)-amino]-benzamide.

On introduit à température ambiante 257 mg du produit obtenu au Stade 1 de l'exemple 1 dans 4 ml de n-butanol et 163 mg de 3-amino benzamide et plonge dans un bain à la température d'environ 100°C pendant 16 heures sous agitation puis laisse revenir à température ambiante. On essore, rince à l'éther puis sèche sous pression réduite à température ambiante. On obtient ainsi 334 mg de produit attendu.

### Stade 2 : dichlorhydrate de trans-3-[[2-[(4-aminocyclohexyl)-amino]-9-cyclopentyl-9H-purin-6-yl]-amino]-benzamide.

On porte 400 mg de trans-1,4-diaminocyclohexane à environ 150°C et 250 mg du produit obtenu au stade 1 ci-dessus. On laisse sous agitation pendant 5 heures puis laisse revenir à température ambiante pendant 16 heures. On ajoute 8 ml de méthanol, chromatographie sur silice (éluant : MeOH/NH₄OH 98/2). On reprend le résidu par une solution éthanolique d'acide chlorhydrique, évapore les solvants, empâte à l'éther, essore, sèche sous pression réduite à température ambiante et récupère 101 mg de produit attendu.

### RMN dans DMSO

| | |
|---|---|
| 1,38 (m) -1,52 (m) 4H | les H axiaux du cyclohexyle |
| 1,71 (m) -1,92 (m) | |
| 2,03 (masqué) | les CH₂ du cyclopentyle |
| 2,21 (m) 8H | |
| 2,05 (d) 4H | H équatoriaux du cyclohexyle |
| 2,99 (sl) 1H | H₄ axial |
| 3,76 (t) 1H | H₁ axial |
| 4,85 (qt) 1H | CH du cyclopentyle |
| 7,46 (t) 1H | H₅' |
| 7,60 (d) 1H | H₆' |
| 8,09 (sl) 2H | H₄' |
| 8,11 (sl) 3H | NH₂-cyclohexyle + N=CH-N |
| 8,29 (sl) 1H | H₂' |
| 7,29 (sl) | |
| 9,04 (s) 1H | H mobiles |
| 10,79 (sl) <1H | |

### EXEMPLE 109 : Dichlorhydrate de trans-5-[[2-[(4-aminocyclohexyl)-amino]-9-cyclopentyl-9H-purin-6-yl]-amino]-1,3-benzènedicarboxylate de diéthyle.

### Stade 1 : 5-[(2-chloro-9-cyclopentyl-9H-purin-6-yl)-amino]-1,3-benzènedicarboxylate de diéthyle.

On mélange à température ambiante 514 mg du produit obtenu au Stade 1 de l'exemple 1 dans 8 ml de butanol, 570 mg de 5-amino-1,3-benzènediacétate de diéthyle et 331 mg de carbonate de potassium puis chauffe à une température d'environ 100°C pendant 5 heures et demie puis laisse revenir à température ambiante. On dilue avec 15 ml d'eau, extrait à l'acétate d'éthyle, lave à l'eau, sèche, évapore les solvants, empâte à l'éther, sèche sous pression réduite et obtient ainsi 723 mg de produit attendu.

### Stade 2 : Dichlorhydrate de trans-5-[[2-[(4-aminocyclohexyl)-amino]-9-cyclopentyl-9H-purin-6-yl]-amino]-1,3-benzène-dicarboxylate de diéthyle.

On porte 1,12 g de trans-1,4-diaminocyclohexane à environ 150°C et 641 mg du produit obtenu au stade 1 ci-dessus. On laisse sous agitation pendant 6 heures et demie puis laisse revenir à température ambiante pendant 16 heures. On ajoute 8 ml de méthanol, chromatographie sur silice (éluant : MeOH/NH₄OH 98/2). On reprend 116 mg de produit par une solution éthanolique d'acide chlorhydrique, évapore les solvants, empâte à l'éther, essore, sèche sous pression réduite à température ambiante et récupère 144 mg de produit attendu.

### RMN dans DMSO

| | |
|---|---|
| 1,37 (m) 10H | les H axiaux du cyclohexyle |
| 1,72 (m) -1,92 (m) | |
| 2,05 (masqué) 8H | les CH₂ du cyclopentyle |
| 2,21 (m) | |
| 2,03 (m) 4H | les CH₃ + H équatoriaux du cyclohexyle |
| 2,97 (sl) 1H | H₄ axial |
| 3,73 (t) 1H | H₁ axial |
| 4,40 (q) 4H | les CH₂O |
| 4,84 (qt) 1H | CH du cyclopentyle |
| 8,02 (sl) <3H | NH₂-cyclohexyle + N=CH-N |
| 8,20 (t) 1H | H₄' |
| 8, 66 (d) 2H | H₂'-H₆' |
| 8,85 (sl) -10,90 (sl) <1H | H mobiles |

### EXEMPLE 110 : Acide trans-5-[[2-[(4-aminocyclohexyl)-amino]-9-cyclopentyl-9H-purin-6-yl]-amino]-1,3-benzènedicarboxylique (sel de disodium).

On introduit 370 mg de produit préparé comme à l'exemple 109 dans 5 ml d'éthanol, ajoute 1,5 ml de soude 1N et agite 21 heures à température ambiante. On ajoute 2 ml d'acide chlorhydrique concentré au milieu réactionnel puis 10 ml d'eau, essore le précipité, sèche sous pression réduite à 50°C. On reprend le résidu dans 50 ml de méthanol, ajoute 3 ml de soude 1N, évapore à sec, empâte dans l'éther et recueille 475 mg de produit attendu.

### RMN dans DMSO

| | |
|---|---|
| 1,12 (m) -1,27 (m) 4H | H axiaux du cyclohexyle |
| 1,67 (m) -1,95 (m) 4H | H équatoriaux du cyclohexyle |
| 1,68 (m) -1,90 (m) | |
| 2,05 (m) 8H | les CH₂ du cyclopentyle |
| 2,51 (masqué) | |
| 2,51 (masqué) | H₄ axial |
| 3,75 (tt) 1H | H₁ axial |
| 4,61 (m) 1H | CH du cyclopentyle |
| 7,40 (s) 1H | N=CH |
| 7,87 (s) 1H | Hₐ |
| 7,99 (s) 2H | les H_{b} |

### EXEMPLE 111 : Dichlorhydrate de trans-3-[[[2-[(4-aminocyclohexyl)-amino]-9-cyclopentyl-9H-purin-6-yl]-amino]-méthyl]-benzoate d'éthyle.

### Stade 1 : 3-[[(2-chloro-9-cyclopentyl-9H-purin-6-yl)-amino]-méthyl]-benzoate d'éthyle.

On introduit à température ambiante 1,03 g du produit obtenu au stade 1 de l'exemple 1 dans 18 ml de n-butanol et 872 mg de 3-(aminométhyl)-benzèneacétate d'éthyle et agite pendant 5 heures et demie dans un bain à 100°C puis laisse revenir à température ambiante. On évapore les solvants, reprend au chlorure de méthylène, lave à l'eau, sèche et évapore les solvants. On chromatographie le résidu sur silice (éluant : CH₂Cl₂/AcOEt 9/1) et recueille 482 mg de produit attendu.

### Stade 2 : dichlorhydrate de trans-3-[[[2-[(4-aminocyclohexyl)-amino]-9-cyclopentyl-9H-purin-6-yl]-amino]-méthyl]-benzoate d'éthyle.

On porte 750 mg de trans-1,4-diaminocyclohexane à environ 150°C puis ajoute 375 mg du produit obtenu au stade 1 ci-dessus, maintient sous agitation à 140°C pendant 5 heures 30 minutes puis laisse revenir à température ambiante. On ajoute alors 5 ml de méthanol, chromatographie sur silice (éluant : MeOH/NH₄OH 98/2, recueille 314 mg de produit, en reprend 67 mg de dans 4 ml de solution éthanolique d'acide chlorhydrique évapore les solvants et récupère 84 mg de produit attendu.

### RMN dans DMSO

| | |
|---|---|
| 1,32 (t) 3H | CH₃-CH₂O |
| 4, 32 (q) 2H | CH₃-CH₂O |
| 1,35 (m) -1,48 (m) | H axiaux du cyclohexyle |
| 1169 (m) -1,88 (m) | |
| 2,03 (masqué) 8H | les CH₂ du cyclopentyle |
| 2,17 (m) | |
| 3,02 (sl) 1H | H₄ axial |
| 3,69 (tl) 1H | H₁ axial |
| 4,76 (qt) 1H | CH du cyclopentyle |
| 4,91 (sl) | HN-CH₂-phényle |
| 7,50 (t) 1H | H₅' |
| 7,70 (d) 1H-7,87 (d) 1H | H₄'-H₆' |
| 8,01 (s) 1H | H₂' |
| 8,08 (sl) <3H | NH₂-cyclohexyle + N=CH-N |
| 8,42 (sl) <1H-9, 53 (sl) <1H | H mobiles |

### EXEMPLE 112 : Acide trans-3-[[[2-[(4-aminocyclohexyl)-amino]-9-cyclopentyl-9H-purin-6-yl]amino]-méthyl]-benzoïque.

On introduit à température ambiante 250 mg du produit obtenu à l'exemple 111 dans 5 ml d'éthanol et ajoute 0,6 ml de soude N. On agite 12 heures à température ambiante, évapore les solvants, empâte à l'éther sèche sous pression réduite à température ambiante et recueille 227 mg de produit attendu.

### RMN dans DMSO

| | |
|---|---|
| de 1,00 à 1,30 (m) 5H | les H axiaux du cyclohexyle |
| 1,66 (m) -1, 90 (m) -2, 03 (m) 8H | les CH2 du cyclopentyle |
| 1,75 (m) -1,90 (m) 5H | les H équatoriaux du cyclohexyle |
| 3,60 (m) 1H | H₁ axial |
| 4,65 (m) 1H | CH du cyclopentyle |
| 4,68 (masqué) 2H | HN-CH₂-phényle |
| 5,73 (dl) 1H | N=C(NH)-N= |
| 7,15 (t) 1H | H_{c} |
| 7,25 (dl) -7,69 (masqué) 2H | H_{b}-H_{d} |
| 7,40 (tl) 1H | HN-CH₂-phényle |
| 7,68 (s) 1H | N=CH |
| 7,86 (sl) 1H | Hₐ |

### EXEMPLE 113 : COMPOSITION PHARMACEUTIQUES

On a préparé des comprimés répondant à la formule suivante :
Produit de l'exemple 6 0,2 g
Excipient pour un comprimé terminé (détail de l'excipient : lactose, talc, amidon, stéarate de magnésium). à 1 g

### EXEMPLE 114 : COMPOSITION PHARMACEUTIQUE

On a préparé des comprimés répondant à la formule suivante :
Produit de l'exemple 59 0,2 g
Excipient pour un comprimé terminé à (détail de l'excipient : lactose, talc, amidon, stéarate de magnésium). 1 g

## Revendications

1. Produits de formule (I) : 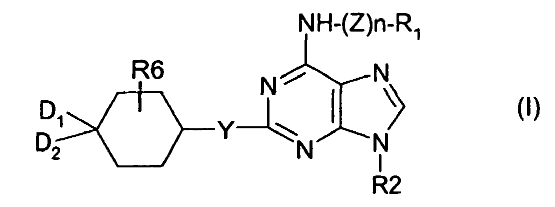 dans laquelle :
Z représente le radical divalent -CH₂-, -SO₂- -CO-, -(CH₂)₂-NH-, -(CH₂)₂-Nalkyle, -(CH₂)₂-N-CH₂-phényle dans lesquels les radicaux phényle sont éventuellement substitués par un atome d'halogène, un radical hydroxyle, trifluoro-méthyle, alcoxy renfermant au plus 4 atomes de carbone ou carboxy libre, salifié ou estérifié,
n représente l'entier 0 ou 1,
R₁ est choisi parmi l'atome d'hydrogène et les radicaux phényle, -CH₂-phényle, -SO₂-phényle, -CO-phényle, pyridyle, alkyle et -SO₂-alkyle, dans lesquels les radicaux alkyle renferment au plus 4 atomes de carbone et sont éventuellement substitués par un radical carboxy libre, salifié ou estérifié, et tous les radicaux phényle sont éventuellement substitués par un ou plusieurs radicaux choisis parmi les atomes d'halogène et les radicaux hydroxyle, cyano, nitro, trifluorométhyle, trifluorométhoxy, thioalkyle et alcoxy renfermant au plus 4 atomes de carbone, alkyle renfermant au plus 4 atomes de carbone éventuellement substitué par un radical cyano, -COOH ou COOalk, les radicaux phényle, tétrazolyle, cycloalkyle interrompu par un ou plusieurs atomes d'oxygène ou d'azote, les radicaux -SO₂NH₂ et SO₂-NH-thiazolyle, les radicaux dioxol, carboxy libre, estérifié ou salifié et les radicaux -NHR₄ et -CONHR₄ dans lesquels R₄ représente un atome d'hydrogène, un radical alkyle renfermant au plus 4 atomes de carbone ou un radical cyclohexyle éventuellement substitué par un radical NH₂,
R₂ représente les radicaux cyclopentyle, tétrahydrofuryle ou le radical tétrahydro-thiényle,
Y représente l'atome d'oxygène ou le radical -NH ou -Nalkyle dans lequel le radical alkyle linéaire ou ramifié renferme au plus 4 atomes de carbone,
D₁ et D₂ soit, identiques ou différents, sont choisis parmi l'atome d'hydrogène, le radical hydroxyle, les radicaux alkyle et alcoxy linéaires ou ramifiés renfermant au plus 4 atomes de carbone et les radicaux -NH₂, -NH-COOtBu ou
-NHalkyle dans lequel le radical alkyle linéaire ou ramifié renferme au plus 4 atomes de carbone, soit forment ensemble le radical =O ou =N-Oalkyle, dans lequel le radical alkyle linéaire ou ramifié renferme au plus 4 atomes de carbone,
R₆ représente l'atome d'hydrogène, un atome d'halogène ou le radical hydroxyle,
lesdits produits de formule (I) étant sous toutes les formes isomères possibles racémiques, énantiomères et diastéréo-isomères, ainsi que les sels d'addition avec les acides minéraux et organiques ou avec les bases minérales et organiques desdits produits de formule (I).

2. Produits de formule (I) telle que définie à la revendication 1, répondant à la formule (Id): 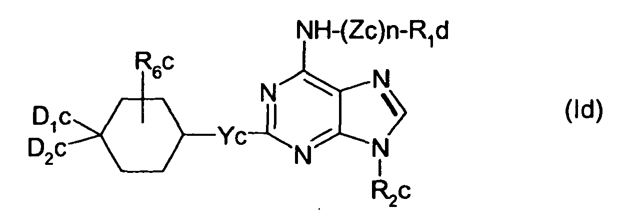 dans laquelle:
Zc représente le radical divalent -CH₂-, -SO₂-, -CO-, -(CH₂)₂-NH-, -(CH₂)₂-Nalkyle, -(CH₂)₂-N-CH₂-phényle dans lesquels les radicaux phényle sont éventuellement substitués par un atome d'halogène, un radical hydroxyle, trifluoro-méthyle, alcoxy renfermant au plus 4 atomes de carbone ou carboxy libre, salifié ou estérifié,
n représente l'entier 0 ou 1,
R₁d est choisi parmi l'atome d'hydrogène et les radicaux phényle, -CH₂-phényle, -SO₂-phényle, -CO-phényle, alkyle et
-SO₂-alkyle, dans lesquels les radicaux alkyle renferment au plus 4 atomes de carbone et sont éventuellement substitués par un radical carboxy libre, salifié ou estérifié, et tous les radicaux phényle sont éventuellement substitués par un ou plusieurs radicaux choisis parmi les atomes d'halogène et les radicaux hydroxyle, cyano, nitro, trifluorométhyle, trifluorométhoxy, thioalkyle et alcoxy renfermant au plus 4 atomes de carbone, alkyle renfermant au plus 4 atomes de carbone éventuellement substitué par un radical cyano ou carboxy libre ou estérifié, les radicaux morpholinyle, phényle, tétrazolyle, -SO₂NH₂, SO₂-NH-thiazolyle, dioxol, carboxy libre, estérifié ou salifié, -NHR₄c et -CONHR₄c dans lesquels R₄c représente un atome d'hydrogène, un radical alkyle renfermant au plus 4 atomes de carbone ou un radical cyclohexyle éventuellement substitué par un radical NH₂,
R₂c représente les radicaux cyclopentyle, tétrahydrofuryle ou le radical tétrahydro-thiényle,
Yc représente l'atome d'oxygène ou le radical -NH ou -N-alkyle dans lequel le radical alkyle linéaire ou ramifié renferme au plus 4 atomes de carbone,
D₁c et D₂c soit, identiques ou différents, sont choisis parmi l'atome d'hydrogène, le radical hydroxyle, les radicaux alkyle et alcoxy linéaires ou ramifiés renfermant au plus 4 atomes de carbone et les radicaux -NH₂, -NH-COOtBu ou
-NH-alkyle dans lequel le radical alkyle linéaire ou ramifié renferme au plus 4 atomes de carbone, soit forment ensemble le radical =O ou =N-Oalkyle, dans lequel le radical alkyle linéaire ou ramifié renferme au plus 4 atomes de carbone,
R₆c représente l'atome d'hydrogène, un atome d'halogène ou le radical hydroxyle,
lesdits produits de formule (Id) étant sous toutes les formes isomères possibles racémiques, énantiomères et diastéréo-isomères, ainsi que les sels d'addition avec les acides minéraux et organiques ou avec les bases minérales et organiques desdits produits de formule (Id).

3. Produits de formule (I) telle que définie aux revendications 1 et 2, répondant aux formules suivantes :
- Dichlorhydrate de trans-4-[[[2-[(4-aminocyclohexyl)-amino]-9-cyclopentyl-9H-purin-6-y]-amino]-méthyl]-benzoate de butyle,
- Dichlorhydrate de trans-4-[[2-[(4-aminocyclohexyl)-amino]-9-cyclopentyl-9H-purin-6-yl]-amino]-benzoate d'éthyle,
- Trichlorhydrate de trans-N2-(4-aminocyclohexyl)-9-cyclo-pentyl-N6-[2-[(phénylméthyl)-amino]-éthyl]-9H-purin-2,6-diamine,
- Trichlorhydrate de trans-N2-(4-aminocyclohexyl)-N6-(2-aminoéthyl)-9-cyclopentyl-9H-purin-2,6-diamine,
- Trichlorhydrate de trans-N2-(4-aminocyclohexyl)-9-cyclo-pentyl-N6-[2-[[(4-méthoxyphényl)-méthyl]-amino]-éthyl]-9H-purin-2,6-diamine,
- Trichlorhydrate de trans-N2-(4-aminocyclohexyl)-N6-[2-[[[4-chloro-3-(trifluorométhyl)-phenyl]-méthyl]-amino]-éthyl]-9-cyclopentyl-9H-purin-2,6-diamine,
- Trichlorhydrate de trans-N2-(4-aminocyclohexyl)-9-cyclo-pentyl-N6-[(diphénylméthyl)-amino]-éthyl]-9H-purin-2,6-diamine,
- Trichlorhydrate de trans-N2-(4-aminocyclohexyl)-N6-[2-[[(4-chlorophênyl)-méthyl]-amino]-éthyl]-9-cyclopentyl-9H-purin-2,6-diamine,
- Dichlorhydrate de trans(+-)-4-[[2-[(4-aminocyclohexyl)-amino]-9-(tétrahydro-3-thiényl)-9H- purin-6-yl]-amino]-benzoate d'éthyle,
- Dichlorhydrate de trans(+-)-N2-(4-aminocyclohexyl)-9-(tétrahydro-3-thiényl)-N6-[4-(trifluorométhoxy)-phényl]-9H-purin-2,6-diamine,
- Dichlorhydrate de trans(+-)-N2-(4-aminocyclohexyl)-9-(tétrahydro-3-furanyl)-N6-[(4-trifluorométhoxy)-phényl]-9H-purin-2,6-diamine,

4. Produits de formule (I) telle que définie à l'une quelconque des revendications 1 et 2, répondant aux formules suivantes :
- Dichlorhydrate de trans -3-[[2-[(4-aminocyclohexyl)-amino]-9-cyclopentyl-9H-purin-6-yl]-amino]-benzoate d'éthyle,
- Trichlorhydrate de trans-N2-(4-aminocyclohexyl)-9-cyclopentyl-N6-[2-[[(3,4-dichlorophény)-méthyl]-amino]-éthyl]-9H-purine-2,6-diamine,
- Trichlorhydrate de trans-N2-(4-aminocyclohexyl)-9-cyclopentyl-N6-[2-[[(3,5-dichlorophényl)-méthyl]-amino]-éthyl]-9H-purine-2,6-diamine,
- Dichlorhydrate de trans-4-[[2-[(4-aminocyclohexyl)-amino]-9-cyclopentyl-9H-purin-6-yl]amino]benzèneacétonitrile,
- Dichlorhydrate de trans-N2-(4-aminocyclohexyl)-9-cyclopentyl-N6-[4-(4-morpholinyl)-phényl]-9H-purine-2,6-diamine,
- Dichlorhydrate de trans-4- [[2-[(4-aminocyclohexyl)-amino]-9-cyclopentyl-9H-purin-6-yl]amino]-benzonitrile,
- Dichlorhydrate de trans-N2-(4-aminocyclohexyl)-9-cyclopentyl-N6-(4-nitrophényl)-9H-purine-2,6-diamine,
- Dichlorhydrate de trans-N2-(4-aminocyclohexyl)-N6-(4-aminophényl)-9-cyclopentyl-9H-purine-2,6-diamine,
- Dichlorhydrate de trans-N2-(4-aminocyclohexyl)-9-cyclopentyl-N6-(4-méthoxyphényl)-9H-purine-2,6-diamine,
- Dichlorhydrate de trans-5-[[2-[(4-amino-cyclohexyl)-amino]-9-cyclopentyl-9H-purin-6-yl]-amino]-1,3-benzènedicarboxylate de diéthyle.

5. Procédé de préparation des produits de formule (I), telle que définie à la revendication 1, **caractérisé en ce que** l'on soumet le composé de formule (II) : 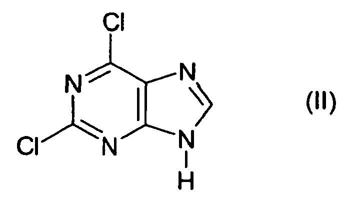 à une réaction avec un composé de formule (III) :
R₂' -OH (III)
dans laquelle R₂' a la signification indiquée à la revendication 1 pour R₂, dans laquelle les éventuelles fonctions réactives sont éventuellement protégées par des groupements protecteurs, pour obtenir le produit de formule (IV) : 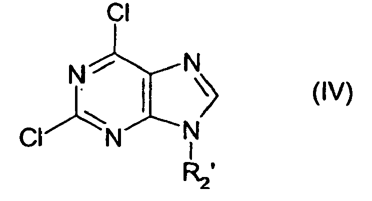 dans laquelle R₂' a la signification indiquée ci-dessus, produit de formule (IV) que l'on soumet aux réactions de l'une quelconque des voies 1 à 6 suivantes :
soit, selon la voie 1, l'on soumet le produit de formule (IV) à une réaction avec un composé de formule (V) :
NH₂-(Z₁')n-R₁' (V)
dans laquelle R₁' a la signification indiquée à la revendication 1 pour R₁, dans laquelle les éventuelles fonctions réactives sont éventuellement protégées par des groupements protecteurs, et n représente l'entier 0 ou 1 et lorsque n représente 1, alors Z₁' représente -CH₂ pour obtenir un produit de formule (VIII) : 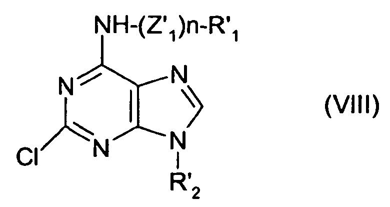 dans laquelle R₁', R₂' et Z₁' ont les significations indiquées ci-dessus,
soit, selon la voie 2, l'on soumet le produit de formule (IV) à une réaction avec un composé de formule (VI):
NH₂-SO₂-R₁' (VI)
dans laquelle R₁' a la signification indiquée ci-dessus, dans laquelle les éventuelles fonctions réactives sont éventuellement protégées par des groupements protecteurs, pour obtenir un produit de formule (IX) : 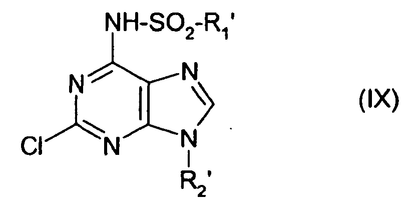 dans laquelle R₁' et R₂' ont les significations indiquées ci-dessus,
soit, selon la voie 3, l'on soumet le produit de formule (IV) à une réaction avec le composé de formule (VII):
NH₂-(CH₂)₂-NH₂ (VII)
pour obtenir un produit de formule (X) : 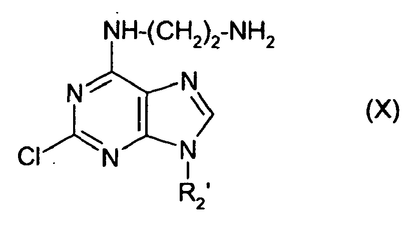 dans laquelle R₂' a la signification indiquée ci-dessus, produit de formule (X) que l'on soumet :
soit à une réaction avec un composé de formule (XI) :
Cl-SO₂-R₁" (XI)
dans laquelle -SO₂-R₁" représente les valeurs correspondantes de R₁' et R₁' a la signification indiquée ci-dessus, pour obtenir un composé de formule (XII) : 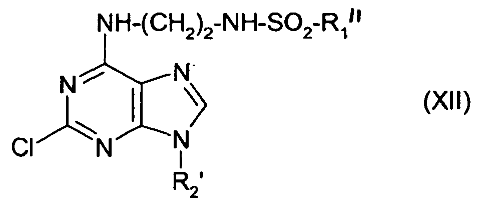 dans laquelle R₁" et R₂' ont les significations indiquées ci-dessus,
soit à une réaction avec un produit de formule (XI)_{A} :
Cl-CO-R₁"' (XI)_{A}
dans laquelle -CO-R₁"' représente les valeurs correspondantes de R₁' et R₁' a la signification indiquée ci-dessus, pour obtenir un produit de formule (XII)_{A} : 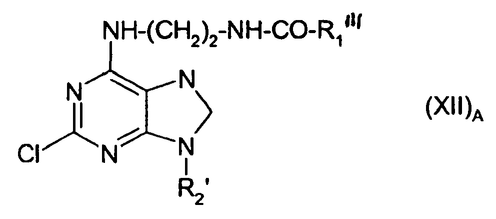 dans laquelle R₁"' et R₂' ont les significations indiquées ci-dessus,
soit à une réaction en présence d'un réducteur avec un produit de formule (XVII) :
R₇-CHO (XVII)
dans laquelle R₇ représente un radical aryle ou alkyle, ces radicaux étant tels que définis ci-dessus pour le radical R₁ dans lesquels les éventuelles fonctions réactives sont éventuellement protégées,
pour obtenir un produit de formule (XIII) : 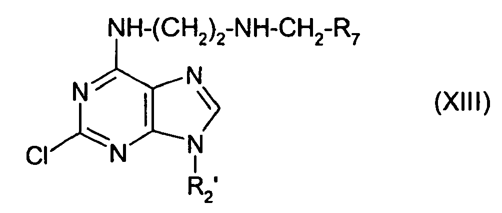 dans laquelle R₂' et R₇ ont les significations indiquées ci-dessus,
soit, selon la voie 4, l'on soumet le produit de formule (IV) à une réaction avec un composé de formule (XVIII) :
R₁'-CO-NH₂ (XVIII)
dans laquelle R₁' a la signification indiquée ci-dessus, pour obtenir un produit de formule (M₁) : 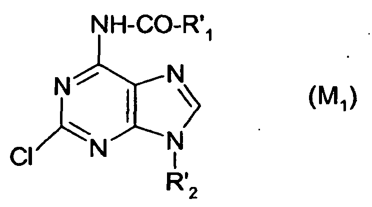 dans laquelle R₁' et R₂' ont les significations indiquées ci-dessus,
produits de formules (VIII), (IX), (XII), (XIII) et (M₁), que l'on peut soumettre aux réactions de l'une quelconque des voies a) ou b) suivantes :
a) soit à une réaction avec un composé de formule (XIV) : 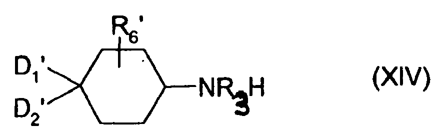 dans laquelle D₁', D₂' et R₆' ont les significations indiquées précédemment respectivement pour D₁, D₂ et R₆ dans lesquelles les éventuelles fonctions réactives sont éventuellement protégées par des groupements protecteurs, et R₃ représente l'atome d'hydrogène ou un radical alkyle renfermant au plus 4 atomes de carbone, pour obtenir un produit de formule (Ix) : 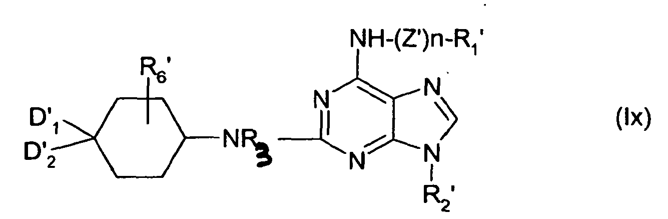 dans laquelle R₁', R₂', R₃, R₆', D₁' et D₂' ont les significations indiquées ci-dessus et Z' a la signification indiquée ci-dessus pour Z dans laquelle les éventuelles fonctions réactives sont éventuellement protégées par des groupements protecteurs,
produit de formule (Ix) qui correspond donc à un produit de formule (I') dans laquelle Y représente -NR₃-
les produits de formule (I') ayant la signification indiquée ci-dessus pour les produits de formule (I) dans laquelle les éventuelles fonctions réactives sont éventuellement protégées par des groupements protecteurs,
b) soit à une réaction avec un composé de formule (XV) : 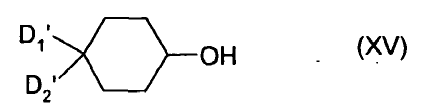 dans laquelle D₁' et D₂' ont les significations indiquées ci-dessus,
pour obtenir un produit de formule (Iy) : 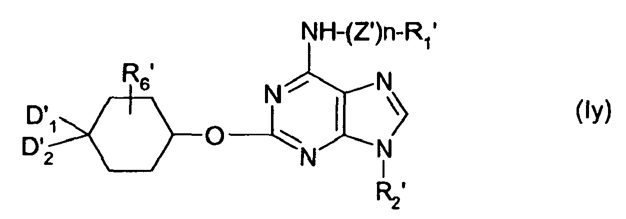 dans laquelle R₁', R₂', R₆', D₁', D₂' et Z' ont les significations indiquées ci-dessus,
produit de formule (Iy) qui correspond donc à un produit de formule (I') tel que défini ci-dessus dans laquelle Y représente -O-,
produits de formules (Ix) et (Iy) qui peuvent être des produits de formule (I) et que, pour obtenir des ou d'autres produits de formule (I), l'on peut soumettre, si désiré et si nécessaire, à l'une ou plusieurs des réactions de transfor-mations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction de réduction des composés nitrés en composés aminés,
j) une réaction d'élimination des groupements protecteurs que peuvent porter les fonctions réactives protégées,
k) une réaction de salification par un acide minéral ou organique ou par une base pour obtenir le sel correspondant,
l) une réaction de dédoublement des formes racémiques en produits dédoublés,
lesdits produits de formule (I) ainsi obtenus étant sous toutes les formes isomères possibles racémiques, énantiomères et diastéréoisomères.

6. A titre de médicaments, les produits de formule (I) telle que définie aux revendications 1 à 4, ainsi que les sels d'addition avec les acides minéraux et organiques ou avec les bases minérales et organiques pharmaceutiquement acceptables desdits produits de formule (I).

7. Les compositions pharmaceutiques contenant à titre de principe actif, l'un au moins des médicaments tels que définis à la revendication 6.

8. A titre de produits industriels, les composés de formules (IX), (X), (XII), (XII)_{A}, (XIII) et (M₁) telles que définies à la revendication 5.

9. Compositions pharmaceutiques selon la revendication 7, **caractérisées en ce qu'**elles sont utilisées comme médicaments antimitotiques, en particulier pour la chimiothérapie de cancers ou encore pour le traitement de psoriasis, de parasitoses telles que celles dues à des champignons ou à des protistes ou de la maladie d'Alzheimer.

10. Compositions pharmaceutiques selon la revendication 7, **caractérisées en ce qu'**elles sont utilisées comme médicaments antineurodégénératifs notamment anti-apoptose neuronale.

11. Utilisation des produits de formule (I) telle que définie aux revendications 1 à 4, pour la préparation de médicaments destinés à la chimiothérapie de cancers, au traitement de psoriasis, de parasitoses telles que celles dues à des champignons ou à des protistes, au traitement de la maladie d'Alzheimer ou au traitement d'affections neurodégénératives notamment l'apoptose neuronale.

## Claims

1. Products of formula (I): 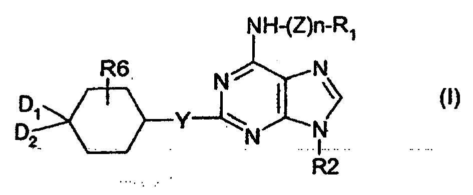 in which:
Z represents the divalent radical -CH₂-, -SO₂-, -CO-, -(CH₂)₂-NH-, -(CH₂)₂-Nalkyl or -(CH₂)₂-N-CH₂-phenyl in which the phenyl radicals are optionally substituted by a halogen atom, a hydroxyl radical, a trifluoromethyl radical, an alkoxy radical including at most 4 carbon atoms or a free, salified or esterified carboxyl radical,
n represents the integer 0 or 1,
R₁ is chosen from the hydrogen atom and radicals of the type phenyl, -CH₂-phenyl, -SO₂-phenyl, -CO-phenyl, pyridyl, alkyl and -SO₂-alkyl in which the alkyl radicals include at most 4 carbon atoms and are optionally substituted by a free, salified or esterified carboxyl radical and all the phenyl radicals are optionally substituted by one or more radicals chosen from halogen atoms and radicals of the type hydroxyl, cyano, nitro, trifluoromethyl, trifluoromethoxy, thioalkyl and alkoxy including at most 4 carbon atoms, alkyl including at most 4 carbon atoms optionally substituted by a cyano, -COOH or COOalk radical, radicals of the type phenyl, tetrazolyl, cycloalkyl interrupted by one or more oxygen or nitrogen atoms, radicals of the type -SO₂NH₂ and SO₂-NH- thiazolyl, radicals of the type dioxolyl, free, esterified or salified carboxyl and radicals of the type -NHR₄ and -CONHR₄ in which R₄ represents a hydrogen atom, an alkyl radical including at most 4 carbon atoms or a cyclohexyl radical optionally substituted by an NH₂ radical,
R₂ represents the cyclopentyl radical, the tetrahydrofuryl radical or the tetrahydrothienyl radical,
Y represents the oxygen atom or the -NH or -Nalkyl radical in which the linear or branched alkyl radical includes at most 4 carbon atoms,
D₁ and D₂ are either identical or different and are chosen from the hydrogen atom, the hydroxyl radical, linear or branched alkyl and alkoxy radicals including at most 4 carbon atoms and -NH₂, -NH-COOtBu or -NHalkyl radicals in which the linear or branched alkyl radical includes at most 4 carbon atoms or together form the =0 or =N-Oalkyl radical in which the linear or branched alkyl radical includes at most 4 carbon atoms,
R₆ represents the hydrogen atom, a halogen atom or the hydroxyl radical,
the said products of formula (I) being in all the possible isomeric forms, racemic, enantiomeric and diastereoisomeric, and the addition salts with inorganic and organic acids or with inorganic and organic bases of the said products of formula (I).

2. Products of formula (I) as defined in Claim 1, corresponding to the formula (Id): 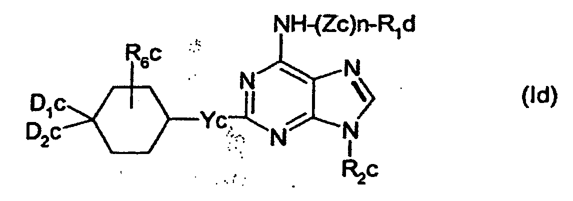 in which:
Zc represents the divalent radical -CH₂-, -SO₂-, -CO-, -(CH₂)₂-NH-, -(CH₂)₂-Nalkyl or -(CH₂)₂-N-CH₂-phenyl in which the phenyl radicals are optionally substituted by a halogen atom, a hydroxyl radical, a trifluoromethyl radical, an alkoxy radical including at most 4 carbon atoms or a free, salified or esterified carboxyl radical,
n represents the integer 0 or 1,
R₁d is chosen from the hydrogen atom and radicals of the type phenyl, -CH₂-phenyl, -SO₂-phenyl, -CO-phenyl, alkyl and -SO₂-alkyl, in which the alkyl radicals include at most 4 carbon atoms and are optionally substituted by a free, salified or esterified carboxyl radical and all the phenyl radicals are optionally substituted by one or more radicals chosen from halogen atoms and radicals of the type hydroxyl, cyano, nitro, trifluoromethyl, trifluoromethoxy, thioalkyl and alkoxy including at most 4 carbon atoms, alkyl including at most 4 carbon atoms optionally substituted by a cyano or free or esterified carboxyl radical, radicals of the type morpholinyl, phenyl, tetrazolyl, -SO₂NH₂, SO₂-NH thiazolyl, dioxolyl, free, esterified or salified carboxyl, -NHR₄c and -CONHR₄c in which R₄c represents a hydrogen atom, an alkyl radical including at most 4 carbon atoms or a cyclohexyl radical optionally substituted by an NH₂ radical,
R₂c represents the cyclopentyl radical, the tetrahydrofuryl radical or the tetrahydrothienyl radical,
Yc represents the oxygen atom or the -NH or -Nalkyl radical in which the linear or branched alkyl radical includes at most 4 carbon atoms,
D₁c and D₂c are either identical or different and are chosen from the hydrogen atom, the hydroxyl radical, linear or branched alkyl and alkoxy radicals including at most 4 carbon atoms and -NH₂, -NH-COOtBu or -NH-alkyl radicals in which the linear or branched alkyl radical includes at most 4 carbon atoms or together form the =O or =N-Oalkyl radical in which the linear or branched alkyl radical includes at most 4 carbon atoms,
R₆c represents the hydrogen atom, a halogen atom or the hydroxyl radical,
the said products of formula (Id) being in all the possible isomeric forms, racemic, enantiomeric and diastereoisomeric, and the addition salts with inorganic and organic acids or with inorganic and organic bases of the said products of formula (Id).

3. Products of formula (I) as defined in Claims 1 and 2, corresponding to the following formulae:
- Butyl trans-4-[[[2-[(4-aminocyclohexyl)amino]-9-cyclopentyl-9H-purin-6-yl]amino]methyl]benzoate dihydrochloride,
- Ethyl trans-4-[[2-[(4-aminocyclohexyl)amino]-9-cyclopentyl-9H-purin-6-yl]amino]benzoate dihydrochloride,
- trans-N2-(4-Aminocyclohexyl)-9-cyclopentyl-N6-[2-[(phenylmethyl)amino]ethyl]-9H-purine-2,6-diamine trihydrochloride,
- trans-N2-(4-Aminocyclohexyl)-N6-(2-aminoethyl)-9-cyclopentyl-9H-purine-2,6-diamine trihydrochloride,
- trans-N2-(4-Aminocyclohexyl)-9-cyclopentyl-N6-[2-[[(4-methoxyphenyl)methyl]amino]ethyl]-9H-purine-2,6-diamine trihydrochloride,
- trans-N2-(4-Aminocyclohexyl)-N6-[2-[[[4-chloro-3-(trifluoromethyl)phenyl]methyl]amino]ethyl]-9-cyclopentyl-9H-purine-2,6-diamine trihydrochloride,
- trans-N2-(4-Aminocyclohexyl)-9-cyclopentyl-N6-[(diphenylmethyl)amino]ethyl]-9H-purine-2,6-diamine trihydrochloride,
- trans-N2-(4-Aminocyclohexyl)-N6-[2-[[(4-chlorophenyl)methyl]amino]ethyl]-9-cyclopentyl-9H-purine-2,6-diamine trihydrochloride,
- Ethyl trans-(+-)-4-[[2-[(4-aminocyclohexyl)amino]-9-(tetrahydro-3-thienyl)-9H-purin-6-yl]amino]benzoate dihydrochloride,
- trans-(+-)-N2-(4-Aminocyclohexyl)-9-(tetrahydro-3-thienyl)-N6-[4-(trifluoromethoxy)phenyl]-9H-purine-2,6-diamine dihydrochloride,
- trans-(+-)-N2-(4-Aminocyclohexyl)-9-(tetrahydro-3-furanyl)-N6-[4-(trifluoromethoxy)phenyl]-9H-purine-2,6-diamine dihydrochloride.

4. Products of formula (I) as defined in either one of Claims 1 and 2, corresponding to the following formulae:
- Ethyl trans-3-[[2-[(4-aminocyclohexyl)amino]-9-cyclopentyl-9H-purin-6-yl]amino]benzoate dihydrochloride,
- trans-N2-(4-Aminocyclohexyl)-9-cyclopentyl-N6-[2-[[(3,4-dichlorophenyl)methyl]amino]ethyl]-9H-purine-2,6-diamine trihydrochloride,
- trans-N2-(4-Aminocyclohexyl)-9-cyclopentyl-N6-[2-[[(3,5-dichlorophenyl)methyl]amino]ethyl]-9H-purine-2,6-diamine trihydrochloride,
- trans-4-[[2-[(4-Aminocyclohexyl)amino]-9-cyclopentyl-9H-purin-6-yl]amino]benzeneacetonitrile dihydrochloride,
- trans-N2-(4-Aminocyclohexyl)-9-cyclopentyl-N6-[4-(4-morpholinyl)phenyl]-9H-purine-2,6-diamine dihydrochloride,
- trans-4-[[2-[(4-aminocyclohexyl)amino]-9-cyclopentyl-9H-purin-6-yl]amino]benzonitrile dihydrochloride,
- trans-N2-(4-Aminocyclohexyl)-9-cyclopentyl-N6-(4-nitrophenyl)-9H-purine-2,6-diamine dihydrochloride,
- trans-N2-(4-Aminocyclohexyl)-N6-(4-aminophenyl)-9-cyclopentyl-9H-purine-2,6-diamine dihydrochloride,
- trans-N2-(4-Aminocyclohexyl)-9-cyclopentyl-N6-(4-methoxyphenyl)-9H-purine-2,6-diamine dihydrochloride,
- Diethyl trans-5-[[2-[(4-aminocyclohexyl)amino]-9-cyclopentyl-9H-purin-6-yl]amino]-1,3-benzenedicarboxylate dihydrochloride.

5. Process for the preparation of products of formula (I) as defined in Claim 1, **characterized in that** the compound of formula (II): 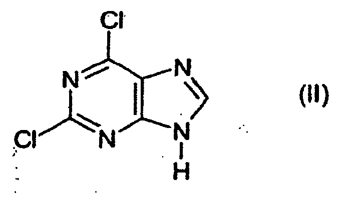 is reacted with a compound of formula (III):
R₂'-OH (III)
in which R₂' has the meaning indicated in Claim 1 for R₂, in which the possible reactive functional groups are optionally protected by protective groups, to produce the product of formula (IV): 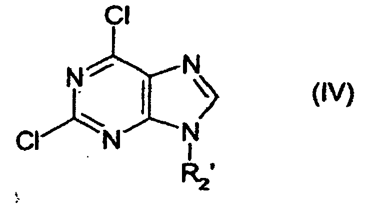 in which R₂' has the meaning indicated above, which product of formula (IV) is subjected to the reactions of any one of the following routes 1 to 6:
either, according to route 1, the product of formula (IV) is reacted with a compound of formula (V):
NH₂-(Z₁')n-R₁' (V)
in which R₁' has the meaning indicated in Claim 1 for R₁, in which the possible reactive functional groups are optionally protected by protective groups, and n represents the integer 0 or 1 and, when n represents 1, then Z₁' represents -CH₂, to produce a product of formula (VIII): 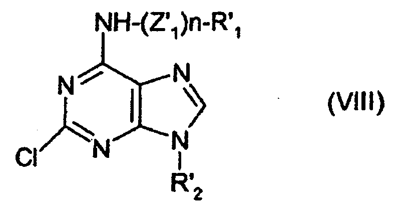 in which R₁', R₂' and Z₁' have the meanings indicated above,
or, according to route 2, the product of formula (IV) is reacted with a compound of formula (VI):
NH₂-SO₂-R₁' (VI)
in which R₁' has the meaning indicated above, in which the possible reactive functional groups are optionally protected by protective groups, to produce a product of formula (IX): 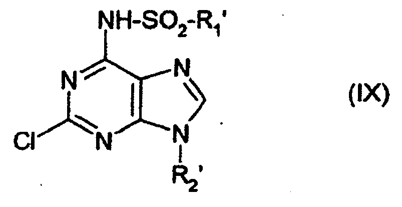 in which R₁' and R₂' have the meanings indicated above,
or, according to route 3, the product of formula (IV) is reacted with the compound of formula (VII):
NH₂-(CH₂)₂-NH₂ (VII)
to produce a product of formula (X) : 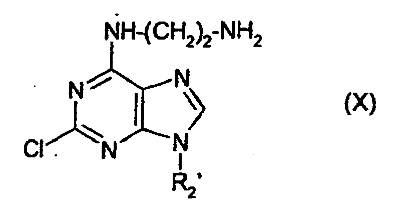 in which R₂' has the meaning indicated above, which product of formula (X) is reacted:
either with a compound of formula (XI):
Cl-SO₂-R₁" (XI)
in which -SO₂-R₁" represents the corresponding values of R₁' and R₁' has the meaning indicated above, to produce a compound of formula (XII): 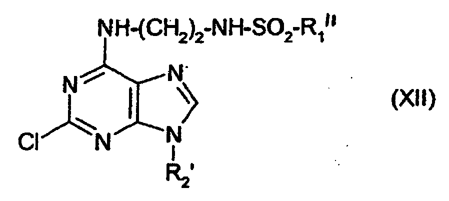 in which R₁" and R₂' have the meanings indicated above,
or with a product of formula (XI)_{A}:
Cl-CO-R₁'" (XI)_{A}
in which -CO-R₁"' represents the corresponding values of R₁' and R₁' has the meaning indicated above, to produce a product of formula (XII)_{A}: 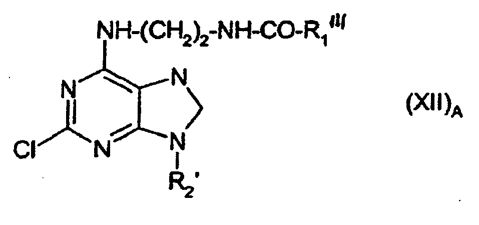 in which R₁"' and R₂' have the meanings indicated above,
or, in the presence of a reducing agent, with a product of formula (XVII):
R₇-CHO (XVII)
in which R₇ represents an aryl or alkyl radical, these radicals being as defined above for the R₁ radical in which the possible reaction functional groups are optionally protected,
to produce a product of formula (XIII): 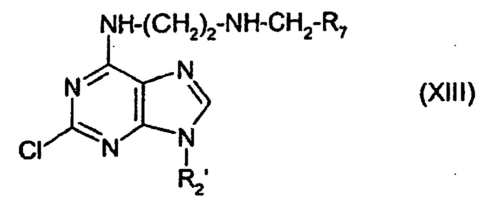 in which R₂' and R₇ have the meanings indicated above,
or, according to route 4, the product of formula (IV) is reacted with a compound of formula (XVIII) :
R₁'-(CO)-NH₂ (XVIII)
in which R₁' has the meaning indicated above, to produce a product of formula (M₁) : 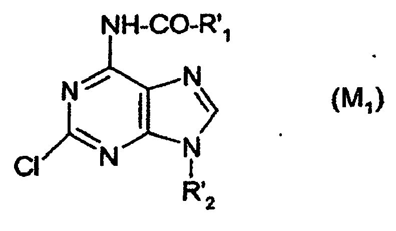 in which R₁' and R₂' have the meanings indicated above, which products of formulae (VIII), (IX), (XII), (XIII) and (M₁), can be reacted according to either one of the following routes a) or b):
a) either with a compound of formula (XIV): 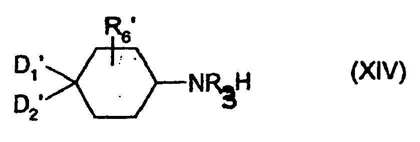 in which D₁', D₂' and R₆' have the meanings indicated above for D₁, D₂ and R₆ respectively, in which the possible reactive functional groups are optionally protected by protective groups, and R₃ represents the hydrogen atom or an alkyl radical including at most 4 carbon atoms, to produce a product of formula (Ix): 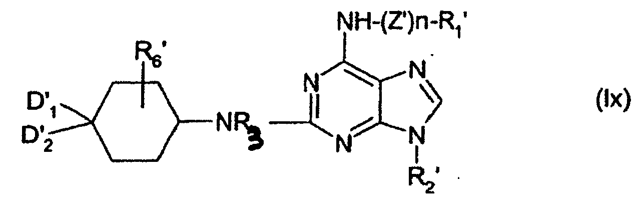 in which R₁', R₂', R₃, R₆', D₁' and D₂' have the meanings indicated above and Z' has the meaning indicated above for Z, in which the possible reactive functional groups are optionally protected by protective groups,
which product of formula (Ix) thus corresponds to a product of formula (I') in which Y represents -NR₃-,
the products of formula (I') having the meaning indicated above for the products of formula (I) in which the possible reactive functional groups are optionally protected by protective groups,
b) or with a compound of formula (XV) : 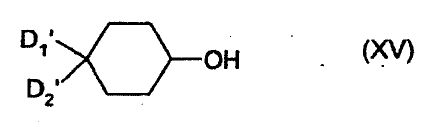 in which D₁' and D₂' have the meanings indicated above, to produce a product of formula (Iy): 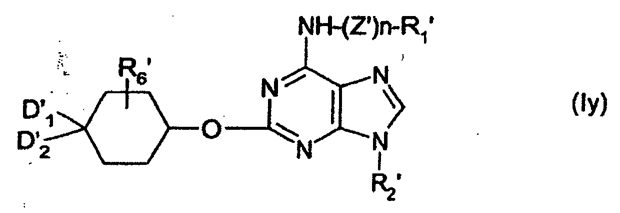 in which R₁', R₂', R₆', D₁', D₂' and Z' have the meanings indicated above,
which product of formula (Iy) thus corresponds to a product of formula (I') as defined above in which Y represents -O-,
which products of formulae (Ix) and (Iy) can be products of formula (I) and, to produce products or other products of formula (I), can be subjected, if desired and if necessary, to one or more of the following conversion reactions, in any order:
a) an esterification reaction on an acid functional group,
b) a saponification reaction on an ester functional group to give an acid functional group,
c) an oxidation reaction on an alkylthio group to give the corresponding sulphoxide or sulphone,
d) a conversion reaction on a ketone functional group to give an oxime functional group,
e) a reduction reaction on the free or esterified carboxyl functional group to give an alcohol functional group,
f) a conversion reaction on an alkoxy functional group to give a hydroxyl functional group or alternatively on a hydroxyl functional group to give an alkoxy functional group,
g) an oxidation reaction on an alcohol functional group to give an aldehyde, acid or ketone functional group,
h) a conversion reaction on a nitrile radical to give a tetrazolyl,
i) a reduction reaction on nitro compounds to give amine compounds,
j) an elimination reaction on the protective groups which the protected reactive functional groups may carry,
k) a salification reaction by an inorganic or organic acid or by a base to produce the corresponding salt,
l) a resolution reaction on the racemic forms to give resolved products,
the said products of formula (I) thus obtained being in all the possible isomeric forms, racemic, enantiomeric and diasteroisomeric.

6. As medicaments, the products of formula (I) as defined in Claims 1 to 4 and the addition salts with inorganic and organic acids or with the pharmaceutically acceptable inorganic and organic acids or with pharmaceutically acceptable inorganic and organic bases of the said products of formula (I).

7. The pharmaceutical compositions comprising, as active principle, at least one of the medicaments as defined in Claim 6.

8. As industrial products, the compounds of formulae (IX), (X), (XII), (XII)_{A}, (XIII) and (M₁) as defined in Claim 5.

9. Pharmaceutical compositions according to Claim 7, **characterized in that** they are used as antimitotic medicaments, in particular for the chemotherapy of cancers, or alternatively for the treatment of psoriasis, parasitoses, such as those due to fungi or to protists, or Alzheimer's disease.

10. Pharmaceutical compositions according to Claim 7, **characterized in that** they are used as antineurodegenerative medicaments, in particular as medicaments for combating neuronal apoptosis.

11. Use of the products of formula (I) as defined in Claims 1 to 4, for the preparation of medicaments intended for the chemotherapy of cancers, for the treatment of psoriasis or of parasitoses, such as those due to fungi or to protists, for the treatment of Alzheimer's disease or for the treatment of neurodegenerative conditions, in particular neuronal apoptosis.

## Patentansprüche

1. Produkte der Formel (I) 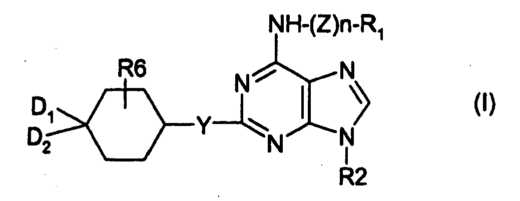 in der
Z den divalenten Rest -CH₂-, -SO₂-, -CO-, -(CH₂)₂-NH-, -(CH₂)₂-Nalkyl, -(CH₂)₂-N-CH₂-phenyl darstellt, in denen die Reste Phenyl gegebenenfalls substituiert sind durch ein Halogenatom, einen Rest Hydroxyl, Trifluormethyl, Alkoxy, umfassend höchstens 4 Kohlenstoffatome oder Carboxy, frei, in Salz überführt oder verestert,
n ein Ganzes von 0 oder 1 darstellt,
R₁ ausgewählt wird unter dem Wasserstoffatom und den Resten Phenyl, -CH₂-phenyl, -SO₂-phenyl, -CO-phenyl, Pyridyl, Alkyl und -SO₂-alkyl, in denen die Reste Alkyl höchstens 4 Kohlenstoffatome umfassen und gegebenenfalls substituiert sind durch einen Rest Carboxy, frei, in Salz überführt oder verestert, und alle Reste Phenyl gegebenenfalls substituiert sind durch einen oder mehrere Reste, ausgewählt unter den Halogenatomen und den Resten Hydroxyl, Cyano, Nitro, Trifluormethyl, Trifluormethoxy, Thioalkyl und Alkoxy, umfassend höchstens 4 Kohlenstoffatome, Alkyl, umfassend höchstens 4 Kohlenstoffatome, gegebenenfalls substituiert durch einen Rest Cyano, -COOH oder COOalk, den Resten Phenyl, Tetrazolyl, Cycloalkyl, unterbrochen durch ein oder mehrere Atome von Sauerstoff oder Stickstoff, den Resten -SO₂H₂ und SO₂-NH-thiazolyl, den Resten Dioxol, Carboxy, frei, in Salz überführt oder verestert und den Resten -NHR₄ und CONHR₄, in denen R₄ ein Wasserstoffatom, einen Rest Alkyl, umfassend höchstens 4 Kohlenstoffatome, oder einen Rest Cyclohexyl darstellt, gegebenenfalls substituiert durch einen Rest NH₂,
R₂ die Reste Cyclopentyl, Tetrahydrofuryl oder den Rest Tetrahydro-thienyl darstellt,
Y das Sauerstoffatom oder den Rest -NH oder -Nalkyl darstellt, worin der lineare oder verzweigte Rest Alkyl höchstens 4 Kohlenstoffatome umfaßt,
D₁ und D₂ entweder, gleich oder verschieden, ausgewählt werden unter dem Wasserstoffatom, dem Rest Hydroxyl, den Resten Alkyl und Alkoxy, linear oder verzweigt, umfassend höchstens 4 Kohlenstoffatome und den Resten -NH₂, -NH-COOtBu oder -NH-alkyl, worin der lineare oder verzweigte Rest Alkyl höchstens 4 Kohlenstoffatome umfaßt,
oder zusammen den Rest =O oder =N-Oalkyl bilden, worin der lineare oder verzweigte Rest Alkyl höchstens 4 Kohlenstoffatome umfaßt,
R₆ das Wasserstoffatom, ein Halogenatom oder den Rest Hydroxyl darstellt,
wobei die genannten Produkte der Formel (I) in allen möglichen racemischen isomeren Formen, Enantiomeren und Diastereoisomeren vorliegen können, sowie Additionssalze der genannten Produkte der Formel (I) mit Mineralsäuren und organischen Säuren oder mit Mineralbasen und organischen Basen.

2. Produkte der Formel (I) wie in Anspruch 1 definiert, entsprechend der Formel (Id) 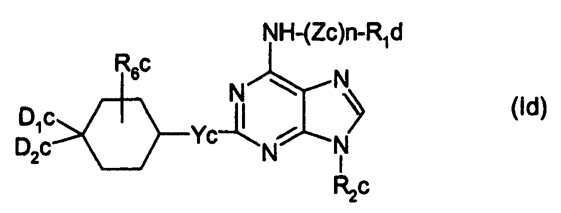 in der
Zc den divalenten Rest -CH₂-, -SO₂-, -CO-, - (CH₂) ₂-NH-, -(CH₂)₂-Nalkyl, -(CH₂)₂-N-CH₂-phenyl darstellt, in denen die Reste Phenyl gegebenenfalls substituiert sind durch ein Halogenatom, einen Rest Hydroxyl, Trifluormethyl, Alkoxy, umfassend höchstens 4 Kohlenstoffatome oder Carboxy, frei, in Salz überführt oder verestert,
n ein Ganzes von 0 oder 1 darstellt,
R₁d ausgewählt wird unter dem Wasserstoffatom und den Resten Phenyl, -CH₂-phenyl, -SO₂-phenyl, -CO-phenyl, Alkyl und -SO₂-alkyl, in denen die Reste Alkyl höchstens 4 Kohlenstoffatome umfassen und gegebenenfalls substituiert sind durch einen Rest Carboxy, frei, in Salz überführt oder verestert, und alle Reste Phenyl gegebenenfalls substituiert sind durch einen oder mehrere Reste, ausgewählt unter den Halogenatomen und den Resten Hydroxyl, Cyano, Nitro, Trifluormethyl, Trifluormethoxy, Thioalkyl und Alkoxy, umfassend höchstens 4 Kohlenstoffatome, Alkyl, umfassend höchstens 4 Kohlenstoffatome, gegebenenfalls substituiert durch einen Rest Cyano oder Carboxy, frei, in Salz überführt oder verestert, den Resten Morpholinyl, Phenyl, Tetrazolyl, -SO₂NH₂, SO₂-NH-thiazolyl, Dioxol, Carboxy, frei, in Salz überführt oder verestert, 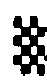 NHR₄c und CONHR₄c, in denen R₄c ein Wasserstoffatom, einen Rest Alkyl, umfassend höchstens 4 Kohlenstoffatome, oder einen Rest Cyclohexyl darstellt, gegebenenfalls substituiert durch einen Rest NH₂,
R₂c die Reste Cyclopentyl, Tetrahydrofuryl oder den Rest Tetrahydro-thienyl darstellt,
Yc das Sauerstoffatom oder den Rest 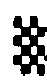 NH oder 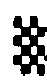 N-alkyl darstellt, worin der lineare oder verzweigte Rest Alkyl höchstens 4 Kohlenstoffatome umfaßt,
D₁c und D₂c entweder, gleich oder verschieden, ausgewählt werden unter dem Wasserstoffatom, dem Rest Hydroxyl, den Resten Alkyl und Alkoxy, linear oder verzweigt, umfassend höchstens 4 Kohlenstoffatome und den Resten 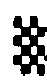 NH₂, -NH-COOtBu oder 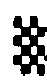 NH-alkyl, worin der lineare oder verzweigte Rest Alkyl höchstens 4 Kohlenstoffatome umfaßt,
oder zusammen den Rest =O oder =N-Oalkyl bilden, worin der lineare oder verzweigte Rest Alkyl höchstens 4 Kohlenstoffatome umfaßt,
R₆c das Wasserstoffatom, ein Halogenatom oder den Rest Hydroxyl darstellt,
wobei die genannten Produkte der Formel (Id) in allen möglichen racemischen isomeren Formen, Enantiomeren und Diastereoisomeren vorliegen können, sowie Additionssalze der genannten Produkte der Formel (Id) mit Mineralsäuren und organischen Säuren oder mit Mineralbasen und organischen Basen.

3. Produkte der Formel (I) wie in Anspruch 1 und 2 definiert, entsprechend den folgenden Formeln:
- Dihydrochlorid von trans-4-{{[2-[(4-Aminocyclohexyl)-amino]-9-cyclopentyl-9H-purin-6-yl]-amino}-methyl}-benzoesäure-butylester,
- Dihydrochlorid von trans-4-{[2-[(4-Aminocyclohexyl)-amino]-9-cyclopentyl-9H-purin-6-yl]-amino}-benzoesäure-ethylester,
- Trihydrochlorid von trans-N2-(4-Aminocyclohexyl)-9-cyclopentyl-N6-{2-[(phenylmethyl)-amino]-ethyl}-9H-purin-2,6-diamin,
- Trihydrochlorid von trans-N2-(4-Aminocyclohexyl)-N6-(2-aminoethyl)-9-cyclopentyl-9H-purin-2,6-diamin,
- Trihydrochlorid von trans-N2-(4-Aminocyclohexyl)-9-cyclopentyl-N6-{2-[[(4-methoxyphenyl)-methyl]-amino]-ethyl}-9H-purin-2,6-diamin,
- Trihydrochlorid von trans-N2-(4-Aminocyclohexyl)-N6-{2-{[[4-chlor-3-(trifluormethyl)-phenyl]-methyl]-amino}-ethyl}-9-cyclopentyl-9H-purin-2,6-diamin,
- Trihydrochlorid von trans-N2-(4-Aminocyclohexyl)-9-cyclopentyl-N6-{[(diphenylmethyl)-amino]-ethyl}-9H-purin-2,6-diamin,
- Trihydrochlorid von trans-N2-(4-Aminocyclohexyl)-N6-{2-[[(4-chlorphenyl)-methyl]-amino]-ethyl}-9-cyclopentyl-9H-purin-2,6-diamin,
- Dihydrochlorid von trans(+-)-4-{[2-[(4-Aminocyclohexyl)-amino]-9-(tetrahydro-3-thienyl)-9H-purin-6-yl]-amino}-benzoesäureethylester,
- Dihydrochlorid von trans(+-)-N2-(4-Aminocyclohexyl)-9-(tetrahydro-3-thienyl)-N6-[4-(trifluormethoxy)-phenyl]-9H-purin-2,6-diamin,
- Dihydrochlorid von trans(+-)-N2-(4-Aminocyclohexyl)-9-(tetrahydro-3-furanyl)-N6-[4-(trifluormethoxy)-phenyl]-9H-purin-2,6-diamin.

4. Produkte der Formel (I) wie in irgendeinem der Ansprüche 1 und 2 definiert, entsprechend den folgenden Formeln:
- Dihydrochlorid von trans-3-{[2-[(4-Aminocyclohexyl)-amino]-9-cyclopentyl-9H-purin-6-yl]-amino}-benzoesäure-ethylester,
- Trihydrochlorid von trans-N2-(4-Aminocyclohexyl)-9-cyclopentyl-N6-{2-[[(3,4-dichlorphenyl)-methyl]-amino]-ethyl}-9H-purin-2,6-diamin,
- Trihydrochlorid von trans-N2-(4-Aminocyclohexyl)-9-cyclopentyl-N6-{2-[[(3,5-dichlorphenyl)-methyl]-amino]-ethyl}-9H-purin-2,6-diamin,
- Dihydrochlorid von trans-4-{[2-[(4-Aminocyclohexyl)-amino]-9-cyclopentyl-9H-purin-6-yl]-amino}-benzol-acetonitril,
- Dihydrochlorid von trans-N2-(4-Aminocyclohexyl)-9-cyclopentyl-N6-[4-(4-morpholinyl)-phenyl]-9H-purin-2,6-diamin,
- Dihydrochlorid von trans-4-{[2-[(4-Aminocyclohexyl)-amino]-9-cyclopentyl-9H-purin-6-yl]-amino}-benzonitril,
- Dihydrochlorid von trans-N2-(4-Aminocyclohexyl)-9-cyclopentyl-N6-(4-nitrophenyl)-9H-purin-2,6-diamin,
- Dihydrochlorid von trans-N2-(4-Aminocyclohexyl)-N6-(4-aminophenyl)-9-cyclopentyl-9H-purin-2,6-diamin,
- Dihydrochlorid von trans-N2-(4-Aminocyclohexyl)-9-cyclopentyl-N6-(4-methoxyphenyl)-9H-purin-2,6-diamin,
- Dihydrochlorid von trans-5-{[2-[(4-Aminocyclohexyl)-amino]-9-cyclopentyl-9H-purin-6-yl]-amino}-1,3-benzoldicarbonsäurediethylester.

5. Verfahren zur Herstellung der Produkte der Formel (I) wie in Anspruch 1 definiert, **dadurch gekennzeichnet, daß** man die Verbindung der Formel (II) 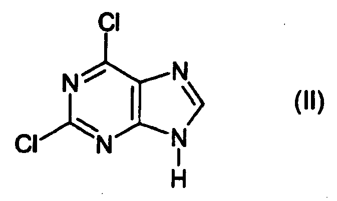 einer Reaktion mit einer Verbindung der Formel (III)
R₂'-OH (III)
unterzieht, in der R₂' die in Anspruch 1 bei R₂ angegebene Bedeutung besitzt, worin die eventuellen reaktiven Funktionen gegebenenfalls durch Schutzgruppen geschützt sind, um das Produkt der Formel (IV) 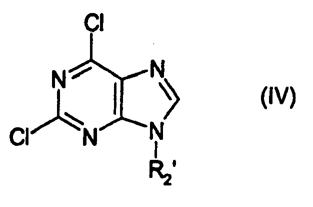 zu erhalten, in der R₂' die vorstehend angegebene Bedeutung besitzt, wonach man das Produkt der Formel (IV) den Reaktionen nach irgendeinem der folgenden Wege 1 bis 6 unterzieht:
entweder, gemäß Weg 1, unterzieht man das Produkt der Formel (IV) einer Reaktion mit einer Verbindung der Formel (V)
NH₂-(Z₁')-R₁' (V)
in der R₁' die vorstehend in Anspruch 1 bei R₁ angegebene Bedeutung besitzt, worin die eventuellen reaktiven Funktionen gegebenenfalls durch Schutzgruppen geschützt sind, und n ein Ganzes von 0 oder 1 darstellt, und wenn n 1 ist, dann Z₁' -CH₂ darstellt, um ein Produkt der Formel (VIII) 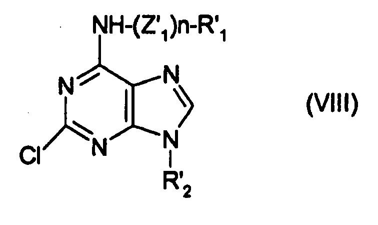 zu erhalten,
in der R₁', R₂' und Z₁' die vorstehend angegebenen Bedeutungen besitzen,
oder, gemäß Weg 2, unterzieht man das Produkt der Formel (IV) einer Reaktion mit einer Verbindung der Formel (VI)
NH₂-SO₂-R₁' (VI)
in der R₁' die vorstehend angegebene Bedeutung besitzt, worin die eventuellen reaktiven Funktionen gegebenenfalls durch Schutzgruppen geschützt sind, um ein Produkt der Formel (IX) 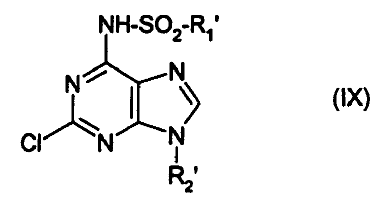 zu erhalten, in der R₁' und R₂' die vorstehend angegebenen Bedeutungen besitzen,
oder, gemäß Weg 3, unterzieht man das Produkt der Formel (IV) einer Reaktion mit einer Verbindung der Formel (VII)
NH₂-(CH₂)₂-NH₂ (VII)
um ein Produkt der Formel (X) 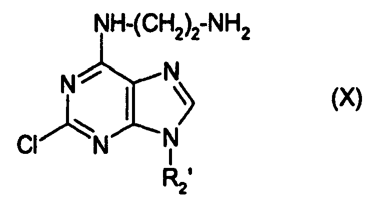 zu erhalten, in der R₂' die vorstehend angegebene Bedeutung besitzt, das man unterzieht:
entweder einer Reaktion mit einer Verbindung der Formel (XI)
Cl-SO₂-R₁" (XI)
in der -SO₂-R₁" die entsprechenden Werte von R₁' aufweist und R₁' die vorstehend angegebene Bedeutung besitzt, um eine Verbindung der Formel (XII) 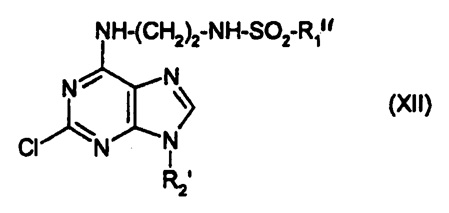 zu erhalten, in benen Bedeutungen besitzen,
oder einer Reaktion mit einem Produkt der Formel (XI)_{A}
Cl-CO-R₁"' (XI)_{A}
in der -CO-R₁"' die entsprechenden Werte von R₁' aufweist und R₁' die vorstehend angegebene Bedeutung besitzt, um eine Verbindung der Formel (XII)_{A} 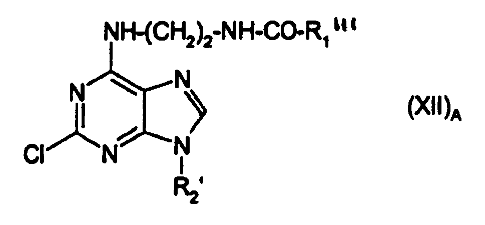 zu erhalten, in der R₁"' und R₂' die vorstehend angegebenen Bedeutungen besitzen,
oder einer Reaktion in Anwesenheit eines Reduktionsmittels mit einem Produkt der Formel (XVII)
R₇-CHO (XVII)
in der R₇ einen Rest Aryl oder Alkyl darstellt, wobei diese Reste wie vorstehend beim Rest R₁ definiert sind, worin die eventuellen reaktiven Funktionen gegebenenfalls durch Schutzgruppen geschützt sind, um ein Produkt der Formel (XIII) 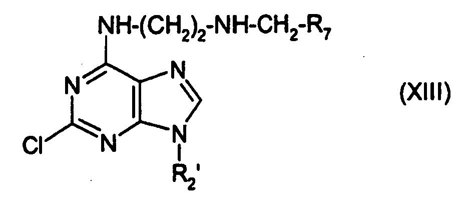 zu erhalten, in der R₂' und R₇ die vorstehend angegebenen Bedeutungen besitzen,
oder, gemäß Weg 4, unterzieht man das Produkt der Formel (IV) einer Reaktion mit einer Verbindung der Formel (XVIII)
R₁'-CO-NH₂ (XVIII)
in der R₁' die vorstehend angegebene Bedeutung besitzt, um ein Produkt der Formel (M₁) 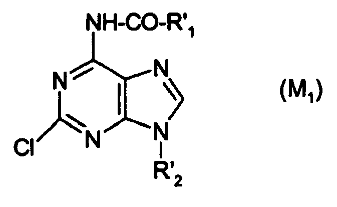 zu erhalten,
in der R₁' und R₂' die vorstehend angegebenen Bedeutungen besitzen, wobei man die Produkte der Formeln (VIII), (IX), (XII), (XIII) und (M₁) den Reaktionen nach irgendeinem der folgenden Wege a) oder b) unterziehen kann:
a) entweder einer Reaktion mit einer Verbindung der Formel (XIV) 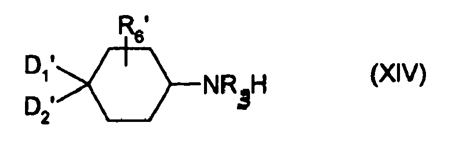 in der D₁', D₂' und R₆' die vorstehend jeweils für D₁, D₂ und R₆ angegebenen Bedeutungen besitzen, worin die eventuellen reaktiven Funktionen gegebenenfalls durch Schutzgruppen geschützt sind, und R₃ das Wasserstoffatom oder einen Rest Alkyl, umfassend höchstens 4 Kohlenstoffatome, darstellt, um ein Produkt der Formel (Ix) 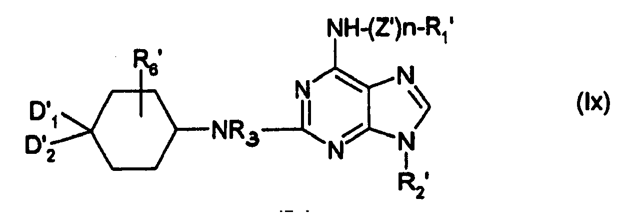 zu erhalten, in der R₁', R₂', R₃, R₆', D₁' und D₂' die vorstehend angegebenen Bedeutungen besitzen und Z' die vorstehend bei Z angegebene Bedeutung besitzt, worin die eventuellen reaktiven Funktionen gegebenenfalls durch Schutzgruppen geschützt sind, wobei das Produkt der Formel (Ix) somit einem Produkt der Formel (I') entspricht, in der Y -NR₃- darstellt, und die Produkte der Formel (I') die oben bei den Produkten der Formel (I) angegebenen Bedeutungen besitzen, worin die eventuellen reaktiven Funktionen gegebenenfalls durch Schutzgruppen geschützt sind,
b) oder einer Reaktion mit einer Verbindung der Formel (XV) 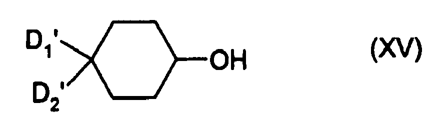 in der
D₁' und D₂' die vorstehend angegebenen Bedeutungen besitzen, um ein Produkt der Formel (Iy) 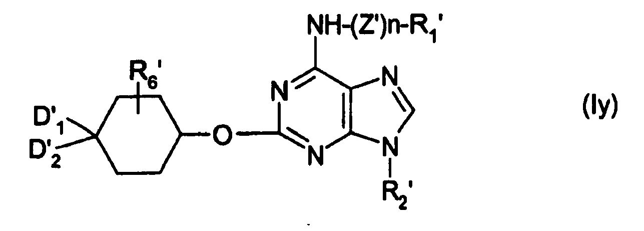 zu erhalten, in der R₁', R₂', R₆', D₁', D₂' und Z' die vorstehend angegebenen Bedeutungen besitzen, wobei das Produkt der Formel (Iy) somit einem wie oben definierten Produkt der Formel (I') entspricht, worin Y 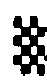 O- darstellt,
und man die Produkte der Formeln (Ix) und (Iy), die Produkte der Formel (I) sein können, um diese oder andere Produkte der Formel (I) zu erhalten, wenn erwünscht und wenn erforderlich, einer oder mehreren der nachfolgend Reaktionen zur Umwandlung in irgendeiner Reihenfolge unterziehen kann:
a) einer Reaktion zur Veresterung der Säurefunktion,
b) einer Reaktion zur Verseifung der Esterfunktion zur Säurefunktion,
c) einer Reaktion zur Oxidation der Alkylthiogruppe zum entsprechenden Sulfoxid oder Sulfon,
d) einer Reaktion zur Umwandlung der Ketonfunktion zur Oximfunktion,
e) einer Reaktion zur Reduktion der freien oder veresterten Carboxyfunktion zur Alkoholfunktion,
f) einer Reaktion zur Umwandlung der Alkoxyfunktion zur Hydroxylfunktion oder auch der Hydroxylfunktion zur Alkoxyfunktion,
g) einer Reaktion zur Oxidation der Alkoholfunktion zur Aldehydfunktion, Säurefunktion oder Ketonfunktion,
h) einer Reaktion zur Umwandlung des Restes Nitril zum Tetrazolyl,
i) einer Reaktion zur Reduktion der nitrierten Verbindungen zu den Aminverbindungen,
j) einer Reaktion zur Entfernung der Schutzgruppen, die von den geschützten reaktiven Funktionen getragen werden können,
k) einer Reaktion zur Salzbildung durch eine Mineralsäure oder organische Säure oder durch eine Base, um das entsprechende Salz zu erhalten,
l) einer Reaktion zur Aufspaltung der racemischen Formen in die Spaltprodukte,
wobei die auf diese Weise erhaltenen Produkte der Formel (I) in allen möglichen racemischen isomeren Formen, Enantiomeren und Diastereoisomeren vorliegen können.

6. Als Arzneimittel die Produkte der Formel (I) wie in den Ansprüchen 1 bis 4 definiert sowie die Additionssalze der genannten Produkte der Formel (I) mit pharmazeutisch akzeptablen Mineralsäuren und organischen Säuren oder mit Mineralbasen und organischen Basen.

7. Pharmazeutische Zusammensetzungen, enthaltend als Wirkstoff mindestens eines der wie in Anspruch 6 definierten Arzneimittel.

8. Als industrielle Produkte die Verbindungen der Formeln (IX), (X), (XII), (XII)_{A}, (XIII) und (M₁) wie in Anspruch 5 definiert.

9. Pharmazeutische Zusammensetzungen nach Anspruch 7, **dadurch gekennzeichnet, daß** sie als antimitotische Arzneimittel, insbesondere für die Chemotherapie von Krebs oder auch zur Behandlung von Psoriasis, von Parasitosen wie denen, die mit Pilzen einhergehen oder bei Protisten oder der Alzheimer-Erkrankung verwendet werden.

10. Pharmazeutische Zusammensetzungen nach Anspruch 7, **dadurch gekennzeichnet, daß** sie als antineurodegenerative Arzneimittel, insbesondere neuronale anti-apoptose Arzneimittel verwendet werden.

11. Verwendung der Produkte der Formel (I) wie in den Ansprüchen 1 bis 4 definiert, zur Herstellung von Arzneimitteln, die vorgesehen sind für die Chemotherapie von Krebs, zur Behandlung von Psoriasis, von Parasitosen wie denen, die mit Pilzen einhergehen oder bei Protisten, zur Behandlung der Alzheimer-Erkrankung oder zur Behandlung von neurodegenerativen Erkrankungen, insbesondere der neuronalen Apoptose.
